# EUROPÄISCHE PATENTSCHRIFT

(11) **EP 2 523 960 B1**
(45) Veröffentlichungstag und Bekanntmachung des Hinweises auf die Patenterteilung: **16.10.2013**
(21) Anmeldenummer: 11700339.2
(22) Anmeldetag: 12.01.2011
(51) Int. Cl.: C07D 498/04, A61K 31/519, A61P 9/00

(54) **HETEROCYCLISCHE CARBONSÄUREDERIVATE MIT EINEM 2,5-SUBSTITUIERTEN OXAZOLOPYRIMIDINRING**
HETEROCYCLIC CARBOXYLIC ACID DERIVATIVES HAVING A 2,5-SUBSTITUTED OXAZOLOPYRIMIDINE RING
Dérivés de l'oxazolopyrimidine à substitution 2,5 en tant qu'agonistes du récepteur Edg-1

(30) Priorität: 14.01.2010 EP 10305043
(43) Veröffentlichungstag der Anmeldung: 21.11.2012
(73) Patentinhaber: SANOFI, 75008 Paris (FR)
(72) Erfinder: KADEREIT, Dieter, 65926 Frankfurt am Main (DE); SCHAEFER, Matthias, 65926 Frankfurt am Main (DE); HACHTEL, Stephanie, 65926 Frankfurt am Main (DE); DIETRICH, Axel, 65926 Frankfurt am Main (DE); HUEBSCHLE, Thomas, 65926 Frankfurt am Main (DE); HISS, Katrin, 65926 Frankfurt am Main (DE)
(74) Vertreter: Then, Johann
(86) Internationale Anmeldenummer: PCT/EP2011/050302
(87) Internationale Veröffentlichungsnummer: WO 2011/086080

(56) Entgegenhaltungen:
- WO-A1-2005/000833
- WO-A1-2009/154775
- WO-A2-2007/061458
- WO-A2-2007/109334

## Beschreibung

Die vorliegende Erfindung betrifft Heterocyclische Carbonsäurederivate mit einem 2,5-substituierten Oxazolopyrimidinring, sowie deren physiologisch akzeptable Salze.

Es sind bereits strukturähnliche Verbindungen im Stand der Technik beschrieben (siehe WO2009/154775), die zur Behandlung multipler Sklerose geeignet sind. Die Wirkungsweise dieser Verbindungen besteht darin, durch Aktivierung des EDG-1 Rezeptors eine Desensitisierung des EDG-1 Signalweges zu verursachen (sog. Superagonismus), der dann einem funktionellen Antagonismus des EDG-1-Signalweges gleichkommt. Systemisch bedeutet das, daß vor allem auf Lymphozyten der EDG-1 Signalweg dauerhaft unterdrückt wird, wodurch diese Zellen nicht mehr chemotaktisch dem S1P Gradienten zwischen Blut und Lymphflüssigkeit folgen können. Dies bedingt, dass die betroffenen Lymphozyten nicht mehr das sekundäre lymphatische Gewebe verlassen können (verstärktes Homeing) und die Zahl der frei zirkulierenden Lymphozyten im Plasma stark gesenkt wird. Dieser Mangel an Lymphozyten im Plasma (Lymphopenie) bewirkt eine Immunsuppression, die zwingend für den Wirkmechanismus der in WO 2009/154775 beschriebenen EDG-1-Rezeptor-Modulatoren erforderlich ist.

WO 2007/061458 und WO 2007/109334 offenbaren S1P- Rezeptor-Modulatoren sowie deren Verwendung in der Behandlung von Krankheilen, die in Verbindung mit der S1P-Rezeptor-Aktivität stehen.

Der vorliegenden Erfindung lag die Aufgabe zugrunde, Verbindungen zur Verfügung zustellen, die spezifisch zur Wundheilung und insbesondere zur Behandlung von Wundheilungsstörungen von Diabetes Patienten geeignet sind. Weiter war es wünschenswert Verbindungen zur Verfügung zustellen, die zur Behandlung des Diabetische Fußsyndroms (DFS) geeignet sind.

Weiter war es wünschenswert eine wiederholbare Aktivierung des EDG-1-Rezeptor Signalweges zu erreichen, was damit pharmakologisch eine persistente Aktivierung des EDG-1 Signalweges ermöglicht.

Die vorliegende Erfindung betrifft Oxazolopyrimidinverbindungen der Formel I. 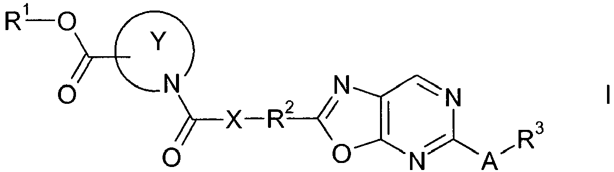 worin A, R¹, R², R³, X und Y wie nachstehend definiert sind. Der Wirkmechanismus der Verbindungen der Formel I beruht also nicht auf Desensitisierung des EDG-1-Signalweges und steht somit dem in WO 2009/154775 beschriebenen Wirkmechanismus diametral gegenüber. Die Erfindung betrifft ferner Verfahren zur Herstellung von Verbindungen der Formel I, ihre Verwendung, insbesondere als Wirkstoff in Pharmazeutika, und pharmazeutische Zusammensetzungen, die sie enthalten.

Patienten mit Diabetes haben gegenüber gesunden Menschen eine verzögerte Wundheilung und eine erhöhte Infektionsrate, vor allem bei länger währender Hyperglykämie, zum Beispiel hervorgerufen durch eine schlechte Blutzuckereinstellung. Zu den Ursachen gehören Durchblutungsstörungen, vor allem im Bereich der kleinen Gefäße, die zu einer verschlechterten Sauerstoff- und Nährstoffversorgung des Gewebes führen. Außerdem besteht eine verminderte Zellteilungs- und Zellmigrationsrate von Keratinozyten, Fibroblasten und dermalen Endothelzellen. Zusätzlich ist die Aktivität verschiedener Abwehrzellen (Granulozyten) mit reduzierter Phagozytose (Aufnahme und Zerstörung von Bakterien) eingeschränkt. Auch die Funktion der Antikörper (Immunglobuline) gegen Bakterien ist bei hohen Blutzuckerwerten eingeschränkt. Dementsprechend müssen Wunden und Infektionen bei Diabetes-Patienten besonders versorgt werden.

Der Edg-1-Rezeptor gehört zur Familie der Edg-Rezeptoren (Edg = Endothelial Differentiation Gene) von gegenwärtig acht identifizierten GPCRs (G-Proteingekoppelten Rezeptoren) der Klasse A. Diese Familie kann in Unterfamilien von durch Sphingosin-1-Phosphat (S1P) aktivierten Rezeptoren (fünf Mitglieder) und durch Lysophosphatidsäure (LPA) aktivierte Rezeptoren (drei Mitglieder) unterteilt werden. Der endogene Ligand S1 P ist ein pluripotentes Lysophospholipid, das durch Aktivierung von GPCRs aus der Edg-Rezeptorfamilie, nämlich Edg-1 (= S1P1), Edg-3 (= S1P3), Edg-5 (= S1P2), Edg-6 (= S1P4) und Edg-8 (S1P5), auf verschiedene Zelltypen wirkt. Wenngleich S1 P auch als intrazellulärer Botenstoff beschrieben wird, werden zahlreiche zelluläre Antworten von S1P über die Aktivierung von Edg-Rezeptoren - vermittelt werden. S1 P wird durch die Enzymfamilie der Sphingosinkinasen (SPHK) erzeugt und durch verschiedene Phosphatasen oder Lyasen abgebaut.

Gegenstand der vorliegenden Erfindung ist eine Oxazolopyrimidinverbindung der Formel I in einer beliebigen ihrer stereoisomeren Formen oder einer Mischung von stereoisomeren Formen in beliebigem Verhältnis oder ein physiologisch akzeptables Salz davon oder ein physiologisch akzeptables Solvat einer derartigen Verbindung oder eines derartigen Salzes, 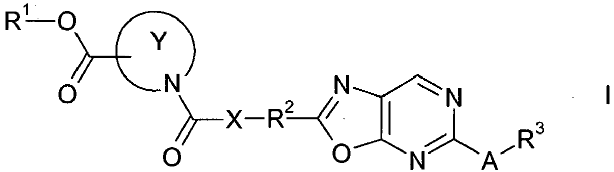 worin
A ausgewählt ist aus NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl und (C₁-C₆)-Alkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxygruppe an die Gruppe R² gebunden ist;
Y für einen 4-gliedrigen bis 7-gliedrigen, gesättigten oder teilweise ungesättigten, monocyclischen oder bicyclischen Heterocyclus steht, der neben dem in Formel I dargestellten Ringstickstoffatom 0, 1, 2 oder 3 gleiche oder verschiedene Ringheteroatome, die unter N, O und S ausgewählt sind, enthält, wobei ein oder zwei der zusätzlichen Ringstickstoffatome ein Wasserstoffatom oder einen (C₁-C₄)-Alkylsubstituenten tragen können und eines der Ringschwefelatome eine oder zwei Oxogruppen tragen kann und wobei der Heterocyclus gegebenenfalls an einem oder mehreren Ringkohlenstoffatomen durch gleiche oder verschiedene (C₁-C₄-)-Alkylsubstituenten substituiert ist;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R²¹ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₂-C₆)-Alkenyl, (C₂-C₆)-Alkinyl, (C₃-C₇)-Cyclo-alkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R²¹ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-al kyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind;
wobei alle Cycloalkyl- und Cycloalkandiylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, Alkandiyl-, CᵤH₂ᵤ-, CᵥH₂ᵥ-, C_{w}H_{2w}-, C_{z}H_{2z}-, Alkenyl-, Alkendiyl-, Alkinyl- and Alkindiylgruppen unabhängig voneinander und unabhängig von anderen Substituenten gegebenenfalls durch einen oder mehrere Fluorsubstituenten substituiert sind.

Strukturelemente wie Gruppen, Substituenten, Heteroringglieder, Zahlen oder andere Merkmale, beispielsweise Alkylgruppen, Gruppen wie R²² oder R³¹, Zahlen wie m, u und v, die in den Verbindungen der Formel I mehrmals vorkommen können, können alle unabhängig voneinander eine beliebige der angegebenen Bedeutungen besitzen und in jedem Fall gleich oder voneinander verschieden sein. Beispielsweise können die Alkylgruppen in einer Dialkylaminogruppe gleich oder verschieden sein.

Alkyl-, Alkenyl- und Alkinylgruppen können linear, d.h. geradkettig, oder verzweigt sein. Dies gilt auch, wenn sie Teil anderer Gruppen, beispielsweise Alkyloxygruppen (= Alkoxygruppen, Alkyl-O-Gruppen), Alkyloxycarbonylgruppen oder alkylsubstituierter Aminogruppen, sind oder wenn sie substituiert sind. Je nach der jeweiligen Definition kann die Zahl der Kohlenstoffatome in einer Alkylgruppe 1, 2, 3, 4, 5 oder 6 oder 1, 2, 3 oder 4 oder 1, 2 oder 3 betragen. Beispiele für Alkyl sind Methyl, Ethyl, Propyl einschließlich n-Propyl und Isopropyl, Butyl, einschließlich n-Butyl, sek.-Butyl, Isobutyl und tert.-Butyl, Pentyl einschließlich n-Pentyl, 1-Methylbutyl, Isopentyl, Neopentyl und tert.-Pentyl und Hexyl einschließlich n-Hexyl, 3,3-Dimethylbutyl und Isohexyl. Doppelbindungen und Dreifachbindungen in Alkenylgruppen und Alkinylgruppen können in beliebigen Positionen vorliegen. In einer Ausführungsform der Erfindung enthalten Alkenylgruppen eine Doppelbindung und Alkinylgruppen eine Dreifachbindung. In einer Ausführungsform der Erfindung enthält eine Alkenylgruppe oder Alkinylgruppe mindestens drei Kohlenstoffatome und ist über ein Kohlenstoffatom, das nicht Teil einer Doppelbindung oder Dreifachbindung ist, an den Rest des Moleküls gebunden. Beispiele für Alkenyl und Alkinyl sind Ethenyl, Prop-1-enyl, Prop-2-enyl (= Allyl), But-2-enyl, 2-Methylprop-2-enyl, 3-Methylbut-2-enyl, Hex-3-enyl, Hex-4-enyl, Prop-2-inyl (= Propargyl), But-2-inyl, But-3-inyl, Hex-4-inyl oder Hex-5-inyl. Substituierte Alkylgruppen, Alkenylgruppen und Alkinylgruppen können in beliebigen Positionen substituiert sein, mit der Maßgabe, daß die jeweilige Verbindung ausreichend stabil und für den gewünschten Zweck wie die Verwendung als Arzneistoff geeignet ist. Die Voraussetzung, daß eine spezifische Gruppe und eine Verbindung der Formel I ausreichend stabil und für den gewünschten Zweck wie die Verwendung als Arzneistoff geeignet sind, gilt allgemein in bezug auf die Definitionen aller Gruppen in den Verbindungen der Formel I.

Soweit anwendbar, gelten die vorstehenden Erklärungen bezüglich Alkyl-, Alkenyl- und Alkinylgruppen entsprechend für zweiwertige Alkylgruppen wie die Gruppen Alkandiyl, CᵤH₂ᵤ, CᵥH₂ᵥ, C_{w}H_{2w} und C_{z}H_{2z} und zweiwertige Alkenylgruppen und Alkinylgruppen wie die Gruppen Alkendiyl und Alkindiyl, die somit ebenfalls linear und verzweigt sein können. Die Doppelbindungen and Dreifachbindungen in Alkendiyl- und Alkindylgruppen können in beliebigen Positionen vorliegen. In einer Ausführungsform der Erfindung enthalten die Alkendiylgruppen eine Doppelbindung und Alkindiylgruppen enthalten eine Dreifachbindung. Beispiele für zweiwertige Alkylgruppen sind -CH₂- (= Methylen), -CH₂-CH₂-, -CH₂-CH₂-CH₂-, -CH₂-CH₂-CH₂-CH₂-, -CH(CH₃)-, -C(CH₃)₂-, -CH(CH₃)-CH₂-, -CH₂-CH(CH₃)-, -C(CH₃)₂-CH₂-, -CH₂-C(CH₃)₂-, Beispiele für zweiwertige Alkenylgruppen sind -CH=CH-, -CH₂-CH=CH-, -CH=CH-CH₂-, -CH₂-CH=CH-CH₂-, -CH₂-CH₂-CH=CH-, -C(CH₃)=C(CH₃)-, und Beispiele für zweiwertige Alkinylgruppen sind -C≡C-, -CH₂-C≡C-, -C≡C-CH₂-, -C(CH₃)₂-C≡C-, -C≡C-C(CH₃)₂-, -CH₂-C≡C-CH₂-, -CH₂-CH₂-C≡C-. Wenn eine Zahl in einer zweiwertigen Gruppe, wie beispielsweise die Zahl z in der Gruppe C_{z}H_{2z}, für 0 (= null) steht, sind die beiden Gruppen, die an die in Rede stehende Gruppe, wie C_{z}H_{2z}, gebunden sind, über eine Einfachbindung direkt miteinander verbunden.

Die Zahl der Ringkohlenstoffatome in einer Cycloalkylgruppe kann 3, 4, 5, 6 oder 7 betragen. In einer Ausführungsform der Erfindung beträgt die Zahl der Ringkohlenstoffatome in einer Cycloalkylgruppe unabhängig von der Zahl der Ringkohlenstoffatome in einer anderen Cycloalkylgruppe 3, 4, 5 oder 6, in einer anderen Ausführungsform 3, 4 oder 5, in einer anderen Ausführungsform 3 oder 4, in einer anderen Ausführungsform 3, in einer anderen Ausführungsform 5, 6 oder 7, in einer anderen Ausführungsform 5 oder 6, in einer anderen Ausführungsform 6 oder 7, in einer anderen Ausführungsform 6. Dies gilt für zweiwertige Cycloalkylgruppen, d.h. Cycloalkandiylgruppen, die über ein oder zwei Ringkohlenstoffatome an benachbarte Gruppen gebunden werden können. Beispiele für Cycloalkylgruppen sind Cyclopropyl, Cyclobutyl, Cyclopentyl, Cyclohexyl und Cycloheptyl. Beispiele für zweiwertige Cycloalkylgruppen sind Cyclopropan-1,1-diyl, Cyclopropan-1,2-diyl, Cyclobutan-1,3-diyl, Cyclopentan-1,1-diyl, Cyclopentan-1,2-diyl, Cyclopentan-1,3-diyl, Cyclohexan-1,1-diyl, Cyclohexan-1,2-diyl, Cyclohexan-1,3-diyl, Cyclohexan-1,4-diyl, Cycloheptan-1,4-diyl. Unabhängig voneinander und unabhängig von anderen Substituenten sind Cycloalkylgruppen und Cycloalkandiylgruppen gegebenenfalls durch einen oder mehrere gleiche oder verschiedene (C₁-C₄)-Alkylsubstituenten substituiert, die in beliebigen Positionen stehen können, d.h. Cycloalkylgruppen können durch Alkylsubstituenten unsubstituiert oder durch Alkylsubstituenten, beispielsweise 1, 2, 3 oder 4 oder 1 oder 2 (C₁-C₄)-Alkylsubstituenten, beispielsweise Methylgruppen, substituiert sein. Beispiele für alkylsubstituierte Cycloalkylgruppen und Cycloalkandiylgruppen sind 4-Methylcyclohexyl, 4-tert.-Butylcyclohexyl oder 2,3-Dimethylcyclopentyl, 2,2-Dimethylcyclopropan-1,1-diyl, 2,2-Dimethylcyclopropan-1,2-diyl, 2,2-Dimethylcyclopentan-1,3-diyl, 6,6-Dimethylcycloheptan-1,4-diyl. Beispiele für Cycloalkylalkylgruppen, die beispielsweise Gruppen wie (C₃-C₇)-Cycloalkyl-C_{z}H_{2z}- repräsentieren können, sind Cyclopropylmethyl, Cyclobutylmethyl, Cyclopentylmethyl, Cyclohexylmethyl, Cycloheptylmethyl, 1-Cyclopropylethyl, 2-Cyclopropylethyl, 1-Cyclobutylethyl, 2-Cyclobutylethyl, 2-Cyclopentylethyl, 2-Cyclohexylethyl, 2-Cycloheptylethyl.

Unabhängig voneinander und unabhängig von anderen Substituenten sind Alkylgruppen, zweiwertige Alkylgruppen, Alkenylgruppen, zweiwertige Alkenylgruppen, Alkinylgruppen, zweiwertige Alkinylgruppen, Cycloalkylgruppen und zweiwertige Cycloalkylgruppen gegebenenfalls durch einen oder mehrere Fluorsubstituenten substituiert, die in beliebigen Positionen stehen können, d.h. diese Gruppen können durch Fluorsubstituenten unsubstituiert oder durch Fluorsubstituenten, beispielsweise 1, 2, 3, 4, 5, 6, 7, 8, 9, 10, 11, 12 oder 13 oder 1, 2, 3, 4, 5, 6, 7, 8 oder 9 oder 1, 2, 3, 4, 5, 6 oder 7 oder 1, 2, 3, 4 oder 5 oder 1, 2 oder 3 oder 1 oder 2 Fluorsubstituenten, substituiert sein. Beispiele für fluorsubstituierte derartige Gruppen sind Trifluormethyl, 2-Fluorethyl, 2,2,2-Trifluorethyl, Pentafluorethyl, 3,3,3-Trifluorpropyl, 2,2,3,3,3-Pentafluorpropyl, 4,4,4-Trifluorbutyl, Heptafluorisopropyl, -CHF-, -CF₂-, -CF₂-CH₂-, -CH₂-CF₂-, -CF₂-CF₂-, -CF(CH₃)-, -C(CF₃)₂-, 1-Fluorcyclopropyl, 2,2-Difluorcyclopropyl, 3,3-Difluorcyclobutyl, 1-Fluorcyclohexyl, 4,4-Difluorcyclohexyl, 3,3,4,4,5,5-Hexafluorcyclohexyl, 2,2-Difluorcyclopropan-1,2-diyl. Beispiele für Alkyloxygruppen, in denen die Alkylgruppierung fluorsubstituiert ist, sind Trifluormethoxy, 2,2,2-Trifluorethoxy, Pentafluorethoxy und 3,3,3-Trifluorpropoxy. In einer Ausführungsform der Erfindung beträgt die Gesamtzahl der Fluorsubstituenten und (C₁-C₄)-Alkylsubstituenten, die unabhängig von anderen Substituenten gegebenenfalls an Cycloalkylgruppen und Cycloalkandiylgruppen in den Verbindungen der Formel I vorliegen, 1, 2, 3, 4, 5, 6, 7, 8, 9, 10 oder 11, in einer anderen Ausführungsform 1, 2, 3, 4, 5, 6, 7, 8 oder 9, in einer anderen Ausführungsform 1, 2, 3, 4 oder 5, in einer anderen Ausführungsform 1, 2, 3 oder 4.

Gruppen wie Phenyl, Naphthyl (= Naphthalinyl) und Reste von aromatischen Heterocyclen, die gegebenenfalls durch einen oder mehrere Substituenten substituiert sind, können unsubstituiert oder substituiert sein, beispielsweise durch 1, 2, 3, 4 oder 5 oder 1, 2, 3 oder 4 oder 1, 2 oder 3 oder 1 oder 2 oder 1 gleiche oder verschiedene Substituenten, die in beliebigen Positionen stehen können. In einer Ausführungsform der Erfindung ist die Gesamtzahl der Nitrosubstituenten in einer Verbindung der Formel I nicht größer als zwei. Aromatische Stickstoffheterocyclen, die in dem zugrundeliegenden Ringsystem ein Wasserstoffatom an einem Ringstickstoffatom in einem 5-gliedrigen Ring, wie beispielsweise einem Pyrrol-, Imidazol-, Indol- oder Benzoimidazolring, tragen, können an den Kohlenstoffatomen und/oder an derartigen Ringstickstoffatomen substituiert sein. In einer Ausführungsform der Erfindung sind Substituenten an derartigen Ringstickstoffatomen aus (C₁-C₄)-Alkylgruppen ausgewählt, d.h. derartige Ringstickstoffatome in aromatischen Heterocyclen tragen ein Wasserstoffatom oder einen (C₁-C₄)-Alkylsubstituenten. Wenn bezüglich Ringstickstoffatomen in aromatischen Heterocyclen und anderen Heterocyclen angegeben ist, daß sie ein Wasserstoffatom oder einen Substituenten tragen können, so tragen derartige Ringstickstoffatome entweder ein Wasserstoffatom oder einen Substituenten oder nicht. Ringstickstoffatome, die ein Wasserstoffatom oder einen Substituenten tragen, kommen in einem stickstoffhaltigen aromatischen 5-gliedrigen Ring, wie er beispielsweise in Pyrrol, Imidazol, Indol oder Benzoimidazol vorliegt, und in einem nichtaromatischen Ring einschließlich eines gesättigten Rings vor. Ringstickstoffatome, die kein Wasserstoffatom oder keinen Substituenten tragen, sofern sie nicht in positiv geladener Form vorliegen, einschließlich weiterer Ringstickstoffatome neben den Ringstickstoffatomen, die ein Wasserstoffatom oder einen Substituenten tragen, kommen in einem aromatischen Ring, wie er beispielsweise in Thiazol, Imidazol, Pyridin oder Benzoimidazol vorliegt, und in einem nichtaromatischen Ring, in dem sie Brückenkopfatome oder Teil einer Doppelbindung sind, und als Ringstickstoffatome, über die ein Ring gebunden ist, vor. Geeignete Ringstickstoffatome in aromatischen Heterocyclen in den Verbindungen der Formel I, wie das Ringstickstoffatom in einem Pyridinring, spezifisch ein Ringstickstoffatom in einem aromatischen Heterocyclus, der R² repräsentiert, können auch einen Oxysubstituenten -O⁻ tragen und als N-Oxid vorliegen, und derartige Ringstickstoffatome können auch als quartäres Salz, beispielsweise als N-(C₁-C₄)-Alkylsalz wie N-Methylsalz, vorliegen, wobei in einer Ausführungsform der Erfindung das Gegenanion in einem derartigen quartären Salz ein physiologisch akzeptables Anion ist, das sich von einer Säure, die ein physiologisch akzeptables Salz bildet, ableitet. In monosubstituierten Phenylgruppen kann der Substituent in der 2-Position, der 3-Position oder der 4-Position stehen. In disubstituierten Phenylgruppen können die Substituenten in 2,3-Position, 2,4-Position, 2,5-Position, 2,6-Position, 3,4-Position oder 3,5-Position stehen. In trisubstituierten Phenylgruppen können die Substituenten in 2,3,4-Position, 2,3,5-Position, 2,3,6-Position, 2,4,5-Position, 2,4,6-Position oder 3,4,5-Position stehen. Naphthyl kann 1-Naphthyl (= Naphthalin-1-yl) oder 2-Naphthyl (= Naphthalin-2-yl) sein. In monosubstituierten 1-Naphthylgruppen kann der Substituent in der 2-, 3-, 4-, 5-, 6-, 7- oder 8-Position stehen. In monosubstituierten 2-Naphthylgruppen kann der Substituent in der 1-, 3-, 4-, 5-, 6-, 7- oder 8-Position stehen. In disubstituierten Naphthylgruppen können die Substituenten ebenfalls in beliebigen Positionen sowohl in dem Ring, über den die Naphthylgruppe gebunden ist, und/oder in dem anderen Ring stehen. Diese Aussage bezüglich der einwertigen Reste gilt entsprechend für die jeweiligen zweiwertigen Reste, wie beispielsweise Phenylengruppen, die R² repräsentieren, die somit ebenfalls unsubstituiert oder substituiert sein können, beispielsweise durch 1, 2, 3 oder 4 oder durch 1, 2 oder 3 oder durch 1 oder 2 oder durch 1 gleiche oder verschiedene Substituenten, die in beliebigen Positionen stehen können.

In R² oder R³ repräsentierenden Resten von aromatischen Heterocyclen, die als Heteroaryl- und Heteroarylengruppen bezeichnet sein können, sowie in allen anderen heterocyclischen Ringen in den Verbindungen der Formel I einschließlich der Gruppe Het und der nichtaromatischen heterocyclischen Gruppen, die R³ repräsentieren, sind die Ringheteroatome im allgemeinen aus N, O und S ausgewählt, wobei N Ringstickstoffatome, die ein Wasserstoffatom oder einen Substituenten tragen, sowie Ringstickstoffatome, die kein Wasserstoffatom und keinen Substituenten tragen, einschließt. Ringheteroatome können in beliebigen Positionen stehen, vorausgesetzt, daß das heterocyclische System in der Technik bekannt und stabil und als Untergruppe für den gewünschten Zweck der Verbindung der Formel I wie die Verwendung als Arzneistoff geeignet ist. In einer Ausführungsform der Erfindung können zwei Ringsauerstoffatome nicht in benachbarten Ringpositionen eines Heterocyclus stehen, in einer anderen Ausführungsform können zwei Ringheteroatome, die aus Sauerstoff und Schwefel ausgewählt sind, nicht in benachbarten Ringpositionen eines beliebigen Heterocyclus stehen. Gesättigte Ringe enthalten keine Doppelbindung im Ring. Ungesättigte Ringsysteme können aromatisch oder teilweise ungesättigt einschließlich teilweise aromatisch sein, wobei in letzterem Fall ein Ring in einem bicyclischen Ringsystem aromatisch ist und das Ringsystem über ein Atom im nichtaromatischen Ring gebunden ist. Je nach der jeweiligen Gruppe können ungesättigte Ringe eine, zwei, drei, vier oder fünf Doppelbindungen im Ring enthalten. Aromatische Gruppen enthalten ein cyclisches System von sechs oder zehn delokalisierten pi-Elektronen im Ring. Je nach der jeweiligen Gruppe können gesättigte und nichtaromatisch ungesättigte heterocyclische Ringe einschließlich Het und nichtaromatischen Gruppen, die R³ repräsentieren, 3-gliedrig, 4-gliedrig, 5-gliedrig, 6-gliedrig 7-gliedrig, 8-gliedrig, 9-gliedrig oder 10-gliedrig sein. In einer Ausführungsform der Erfindung sind aromatische heterocyclische Ringe 5-gliedrige oder 6-gliedrige monocyclische Ringe oder 8-gliedrige, 9-gliedrige oder 10-gliedrige bicyclische Ringe, in einer anderen Ausführungsform 5-gliedrige oder 6-gliedrige monocyclische Ringe oder 9-gliedrige oder 10-gliedrige bicyclische Ringe, in einer anderen Ausführungsform 5-gliedrige oder 6-gliedrige monocyclische Ringe, wobei die 8-gliedrigen, 9-gliedrigen oder 10-gliedrigen bicyclischen Ringe aus zwei anellierten 5-gliedrigen Ringen, einem 5-gliedrigen Ring und einem 6-gliedrigen Ring, die miteinander anelliert sind, bzw. zwei anellierten 6-gliedrigen Ringen zusammengesetzt sind. In bicyclischen aromatischen heterocyclischen Gruppen können ein oder beide Ringe Heteroringglieder enthalten, und ein oder beide Ringe können aromatisch sein. Im allgemeinen werden bicyclische Ringsysteme mit einem aromatischen Ring und einem nichtaromatischen Ring als aromatisch erachtet, wenn sie über ein Kohlenstoffatom im aromatischen Ring gebunden sind, und als nichtaromatisch, wenn sie über ein Kohlenstoffatom im nichtaromatischen Ring gebunden sind. Sofern nicht anders angegeben, können heterocyclische Gruppen einschließlich aromatischer heterocyclischer Gruppen über ein beliebiges geeignetes Ringkohlenstoffatom und im Fall von Stickstoffheterocyclen über ein beliebiges geeignetes Ringstickstoffatom gebunden sein. In einer Ausführungsform der Erfindung ist eine aromatische heterocyclische Gruppe in einer Verbindung der Formel I unabhängig von jeder anderen aromatischen heterocyclischen Gruppe über ein Ringkohlenstoffatom gebunden, in einer anderen Ausführungsformen über ein Ringstickstoffatom. Je nach der Definition der jeweiligen heterocyclischen Gruppe beträgt in einer Ausführungsform der Erfindung die Zahl der Ringheteroatome, die in einer heterocyclischen Gruppe unabhängig von der Zahl von Ringheteroatomen in einer anderen heterocyclischen Gruppe vorliegen kann, 1, 2, 3 oder 4, in einer anderen Ausführungsform 1,2 oder 3, in einer anderen Ausführungsform 1 oder 2, in einer anderen Ausführungsform 1, wobei die Ringheteroatome gleich oder verschieden sein können. Heterocyclische Gruppen, die gegebenenfalls substituiert sind, können unabhängig von jeder anderen heterocyclischen Gruppe unsubstituiert oder durch einen oder mehrere gleiche oder verschiedene Substituenten, beispielsweise 1, 2, 3, 4 oder 5 oder 1, 2, 3 oder 4 oder 1, 2 oder 3 oder 1 oder 2 oder 1 Substituenten, die bei der Definition der jeweiligen Gruppe angegeben sind, substituiert sein. Substituenten an heterocyclischen Gruppen können in beliebigen Positionen stehen. So können Substituenten in einer Pyridin-2-ylgruppe beispielsweise in der 3-Position und/oder 4-Position und/oder 5-Position und/oder 6-Position stehen, in einer Pyridin-3-ylgruppe in der 2-Position und/oder 4-Position und/oder 5-Postion und/oder 6-Position stehen und in einer Pyridin-4-ylgruppe in der 2-Position und/oder 3-Position und/oder 5-Position und/oder 6-Position stehen.

Beispiele für Grundkörper von Heterocyclen, von denen sich heterocyclische Gruppen einschließlich aromatischer heterocyclischer Gruppen, gesättigter heterocyclischer Gruppen und nichtaromatischer ungesättigter heterocyclischer Gruppen ableiten können, sind Azet, Oxet, Pyrrol, Furan, Thiophen, Imidazol, Pyrazol, [1,3]Dioxol, Oxazol (= [1,3]Oxazol), Isoxazol (= [1,2]Oxazol), Thiazol (= [1,3]Thiazol), Isothiazol (= [1,2]Thiazol), [1,2,3]Triazol, [1,2,4]Triazol, [1,2,4]Oxadiazol, [1,3,4]Oxadiazol, [1,2,4]Thiadiazol, [1,3,4]Thiadiazol, Tetrazol, Pyridin, Pyran, Thiopyran, Pyridazin, Pyrimidin, Pyrazin, [1,3]Oxazin, [1,4]Oxazin, [1,3]Thiazin, [1,4]Thiazin, [1,2,3]Triazin, [1,3]Dithiin, [1,4]Dithiin, [1,2,4]Triazin, [1,3,5]Triazin, [1,2,4,5]Tetrazin, Azepin, [1,3]Diazepin, [1,4]Diazepin, [1,3]Oxazepin, [1,4]Oxazepin, [1,3]Thiazepin, [1,4]Thiazepin, Azocin, Azecin, Cyclopenta[b]pyrrol, 2-Azabicyclo[3.1.0]hexan, 3-Azabicyclo[3.1.0]hexan, 2-Oxa-5-azabicyclo[2.2.1]heptan, Indol, Isoindol, Benzothiophen, Benzofuran, [1,3]Benzodioxol (= 1,2-Methylendioxybenzol), [1,3]Benzoxazol, [1,3]Benzothiazol, Benzoimidazol, Thieno[3,2-c]pyridin, Chromen, Isochromen, [1,4]Benzodioxin, [1,4]Benzoxazin, [1,4]Benzothiazin, Chinolin, Isochinolin, Cinnolin, Chinazolin, Chinoxalin, Phthalazin, Thienothiophen, [1,8]Naphthyridin und andere Naphtyridine, Pteridin und die jeweiligen gesättigten und teilweise gesättigten Heterocyclen, in denen eine oder mehrere, beispielsweise eine, zwei, drei, vier oder alle Doppelbindungen im Ringsystem einschließlich der Doppelbindungen im aromatischen Ring durch Einfachbindungen ersetzt sind, wie beispielsweise Azetidin, Oxetan, Pyrrolidin, Tetrahydrofuran, Tetrahydrothiophen, Imidazolidin, Oxazolidin, Thiazolidin, Dihydropyridin, Piperidin, Tetrahydropyran, Piperazin, Morpholin, Thiomorpholin, Azepan, Chroman, Isochroman, [1,4]Benzodioxan (= 1,2-Ethylendioxybenzol), 2,3-Dihydrobenzofuran, 1,2,3,4-Tetrahydrochinolin, 1,2,3,4-Tetrahydroisochinolin.

Beispiele für Reste von aromatischen Heterocyclen, die in den Verbindungen der Formel I vorkommen können, sind Thiophenyl (= Thienyl) einschließlich Thiophen-2-yl und Thiophen-3-yl, Pyridinyl (= Pyridyl) einschließlich Pyridin-2-yl (= 2-Pyridyl), Pyridin-3-yl (= 3-Pyridyl) und Pyridin-4-yl (= 4-Pyridyl), Imidazolyl einschließlich beispielsweise 1H-Imidazol-1-yl, 1H-Imidazol-2-yl, 1H-Imidazol-4-yl und 1H-Imidazol-5-yl, [1,2,4]Triazolyl einschließlich 1H-[1,2,4]-Triazol-1-yl und 4H-[1,2,4]-Triazol-3-yl, Tetrazolyl einschließlich 1H-Tetrazol-1-yl und 1H-Tetrazol-5-yl, Chinolinyl (= Chinolyl) einschließlich Chinolin-2-yl, Chinolin-3-yl, Chinolin-4-yl, Chinolin-5-yl, Chinolin-6-yl, Chinolin-7-yl und Chinolin-8-yl, die alle gegebenenfalls wie in der Definition der jeweiligen Gruppe angegeben substituiert sind. Beispiele für Reste von gesättigten und teilweise ungesättigten Heterocyclen, die in den Verbindungen der Formel I vorkommen können, sind Azetidinyl, Pyrrolidinyl einschließlich Pyrrolidin-1-yl, Pyrrolidin-2-yl und Pyrrolidin-3-yl, 2,5-Dihydro-1H-pyrrolyl, Piperidinyl einschließlich Piperidin-1-yl, Piperidin-2-yl, Piperidin-3-yl und Piperidin-4-yl, 1,2,3,4-Tetrahydropyridinyl, 1,2,5,6-Tetrahydropyridinyl, 1,2-Dihydropyridinyl, Azepanyl, Azocanyl, Azecanyl, Octahydrocyclopenta[b]pyrrolyl, 2,3-Dihydrobenzofuranyl einschließlich 2,3-Dihydrobenzofuran-7-yl, 2,3-Dihydro-1H-indolyl, Octahydro-1 H-indolyl, 2,3-Dihydro-1 H-isoindolyl, Octahydro-1 H-isoindolyl, 1,2-Dihydrochinolinyl, 1,2,3,4-Tetrahydrochinolinyl, Decahydrochinolinyl, 1,2-Dihydroisochinolinyl, 1,2,3,4-Tetrahydroisochinolinyl, 1,2,3,4-Tetrahydroisochinolinyl, Decahydroisochinolinyl, Decahydroisochinolinyl, 4,5,6,7-Tetrahydrothieno[3,2-c]pyridinyl, Pyrazolidinyl, Imidazolidinyl, Hexahydropyrimidinyl, 1,2-Dihydropyrimidinyl, Piperazinyl, [1,3]Diazepanyl, [1,4]Diazepanyl, Oxazolidinyl, [1,3]Oxazinanyl, [1,3]Oxazepanyl, Morpholinyl einschließlich Morpholin-2-yl, Morpholin-3-yl und Morpholin-4-yl, [1,4]Oxazepanyl, Thiazolidinyl, [1,3]Thiazinanyl, Thiomorpholinyl einschließlich Thiomorpholin-2-yl, Thiomorpholin-3-yl und Thiomorpholin-4-yl, 3,4-Dihydro-2H-[1,4]thiazinyl, [1,3]Thiazepanyl, [1,4]Thiazepanyl, [1,4]Thiazepanyl, Oxetanyl, Tetrahydrofuranyl, Tetrahydrothienyl, Isoxazolidinyl, Isothiazolidinyl, Oxazolidinyl, [1,2,4]-Oxadiazolidinyl, [1,2,4]-Thiadiazolidinyl, [1,2,4]Triazolidinyl, [1,3,4]Oxadiazolidinyl, [1,3,4]Thiadiazolidinyl, [1,3,4]Triazolidinyl, 2,3-Dihydrofuranyl, 2,5-Dihydrofuranyl, 2,3-Dihydrothienyl, 2,5-Dihydrothienyl, 2,3-Dihydropyrrolyl, 2,3-Dihydroisoxazolyl, 4,5-Dihydroisoxazolyl, 2,5-Dihydroisoxazolyl, 2,3-Dihydroisothiazolyl, 4,5-Dihydroisothiazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Tetrahydropyridazinyl, Tetrahydropyrimidinyl, Tetrahydropyrazinyl, Tetrahydro[1,3,5]triazinyl, [1,3]Dithianyl, Tetrahydropyranyl, Tetrahydrothiopyranyl, [1,3]Dioxolanyl, 3,4,5,6-Tetrahydropyridinyl, 4H-[1,3]Thiazinyl, 1,1-Dioxo-2,3,4,5-tetrahydrothienyl, 2-Azabicyclo[3.1.0]hexyl einschließlich 2-Azabicyclo[3.1.0]hex-2-yl, 3-Azabicyclo[3.1.0]hexyl einschließlich 3-Azabicyclo[3.1.0]hex-3-yl, 2-Oxa-5-azabicyclo[2.2.1]heptyl einschließlich 2-Oxa-5-azabicyclo[2.2.1]hept-5-yl, die alle über ein geeignetes Ringkohlenstoffatom oder Ringstickstoffatom gebunden sind und gegebenenfalls wie in der Definition der jeweiligen Gruppe angegebenen substituiert sind.
Halogen steht für Fluor, Chlor, Brom oder Iod. In einer Ausführungsform der Erfindung ist jedes Halogen in einer Verbindung der Formel I unabhängig von jedem anderen Halogen aus Fluor, Chlor und Brom ausgewählt, in einer anderen Ausführungsform aus Fluor und Chlor.

Wenn eine Oxogruppe an ein Kohlenstoffatom gebunden ist, ersetzt sie zwei Wasserstoffatome an einem Kohlenstoffatom des zugrundeliegenden Systems. Somit wird eine CH₂-Gruppe in einer Kette oder einem Ring dann, wenn sie durch Oxo, d.h. durch ein doppelt gebundenes Sauerstoffatom, substituiert ist, zu einer C(O)-Gruppe (=C(=O)-Gruppe). Offensichtlich kann eine Oxogruppe nicht als Substituent an einem Kohlenstoffatom in einem aromatischen Ring wie beispielsweise in einer Phenylgruppe vorkommen. Wenn ein Ringschwefelatom in einer heterocyclischen Gruppe eine oder zwei Oxogruppen tragen kann, handelt es sich in dem Fall, daß es keine Oxogruppe trägt, um ein nichtoxidiertes Schwefelatom S, oder in dem Fall, daß es eine Oxogruppe trägt, um eine S(O)-Gruppe (= Sulfoxidgruppe, S-Oxid-Gruppe) oder in dem Fall, daß es zwei Oxogruppen trägt, um eine S(O)₂-Gruppe (= Sulfongruppe, S,S-Dioxid-Gruppe).

Die vorliegende Erfindung schließt alle stereoisomeren Formen der Verbindungen der Formel I und ihre Salze und Solvate ein. Bezüglich jedes chiralen Zentrums können die Verbindungen der Formel I unabhängig von jedem anderen chiralen Zentrum in S-Konfiguration oder weitgehend S-Konfiguration oder in R-Konfiguration oder weitgehend R-Konfiguration oder als Mischung des S-Isomers und des R-Isomers in beliebigen Verhältnissen vorliegen. Die Erfindung schließt alle möglichen Enantiomere und Diastereomere und Mischungen von zwei oder mehr Stereoisomeren, zum Beispiel Gemische von Enantiomeren und/oder Diastereomeren, in allen Verhältnissen ein. Somit können erfindungsgemäße Verbindungen, die als Enantiomere existieren können, in enantiomerenreiner Form sowohl als linksdrehende als auch rechtsdrehende Antipoden und in Form von Mischungen der beiden Enantiomere in allen Verhältnissen einschließlich Racematen vorliegen. Im Fall einer E/Z-Isomerie bzw. cis/trans-Isomerie, beispielsweise an Doppelbindungen oder Ringen wie Cycloalkylringen, schließt die Erfindung sowohl die E-Form als auch die Z-Form bzw. die cis-Form und die trans-Form sowie Mischungen dieser Formen in allen Verhältnissen ein. In einer Ausführungsform der Erfindung handelt es sich bei einer Verbindung, die in zwei oder mehr stereoisomeren Formen vorkommen kann, um ein reines oder weitgehend reines einzelnes Stereoisomer. Die Herstellung von einzelnen Stereoisomeren kann beispielsweise durch Trennung einer Mischung von Isomeren nach üblichen Methoden, beispielsweise durch Chromatographie oder Kristallisation, durch Verwendung von stereochemisch einheitlichen Ausgangsstoffen bei der Synthese oder durch stereoselektive Synthese erfolgen. Gegebenenfalls kann vor einer Trennung von Stereoisomeren eine Derivatisierung durchgeführt werden. Die Trennung einer Mischung von Stereoisomeren kann auf der Stufe der Verbindung der Formel I oder auf der Stufe eines Ausgangsstoffs oder eines Zwischenprodukts im Verlauf der Synthese durchgeführt werden. Die vorliegende Erfindung schließt auch alle tautomeren Formen der Verbindungen der Formel I und ihre Salze und Solvate ein.

Wenn die Verbindungen der Formel I eine oder mehrere saure und/oder basische Gruppen, d.h. salzbildende Gruppen, enthalten, schließt die Erfindung auch ihre entsprechenden physiologisch oder toxikologisch akzeptablen Salze, d.h. nichttoxischen Salze, insbesondere ihre pharmazeutisch akzeptablen Salze, ein. Somit können die Verbindungen der Formel I, die eine saure Gruppe, wie eine Hydroxycarbonylgruppe (= Carboxygruppe = C(O)-OH-Gruppe), enthalten, an derartigen Gruppen vorliegen und erfindungsgemäß verwendet werden, beispielsweise als Alkalimetallsalze, Erdalkalimetallsalze oder als Ammoniumsalze. Genauere Beispiele für derartige Salze sind Natriumsalze, Kaliumsalze, Calciumsalze, Magnesiumsalze, quartäre Ammoniumsalze wie Tetraalkylammoniumsalze oder Säureadditionssalze mit Ammoniak oder organischen Aminen wie beispielsweise Ethylamin, Ethanolamin, Triethanolamin oder Aminosäuren. Verbindungen der Formel I, die eine basische Gruppe, d.h. eine protonierbare Gruppe wie eine Aminogruppe oder einen Stickstoffheterocyclus, enthalten, können in Form ihrer Additionssalze mit anorganischen und organischen Säuren an derartigen Gruppen vorliegen und erfindungsgemäß verwendet werden. Beispiele für geeignete Säuren sind Chlorwasserstoff, Bromwasserstoff, Phosphorsäure, Schwefelsäure, Methansulfonsäure, Oxalsäure, Essigsäure, Trifluoressigsäure, Weinsäure, Milchsäure, Benzoesäure, Malonsäure, Fumarsäure, Maleinsäure, Citronensäure und andere dem Fachmann bekannten Säuren. Wenn eine Verbindung der Formel I gleichzeitig eine saure Gruppe und eine basische Gruppe im Molekül enthält, so schließt die Verbindung zusätzlich zu den erwähnten Salzformen auch innere Salze (= Betaine, Zwitterionen) ein. Die Salze der Verbindungen der Formel I sind nach dem Fachmann bekannten üblichen Methoden erhältlich, beispielsweise durch Inberührungbringen der Verbindung der Formel I in einem Lösungsmittel oder Verdünnungsmittel mit einer organischen oder anorganischen Säure oder Base oder durch Anionenaustausch oder Kationenaustausch aus einem anderen Salz. Die Erfindung schließt auch alle Salze der Verbindungen der Formel I ein, die aufgrund geringer physiologischer Verträglichkeit der salzbildenden Säure oder Base nicht direkt für eine Verwendung in Pharmazeutika geeignet sind, aber zum Beispiel als Zwischenprodukte für chemische Reaktionen oder zur Herstellung von physiologisch akzeptablen Salzen verwendet werden können.

Die vorliegende Erfindung schließt alle Solvate von Verbindungen der Formel I, beispielsweise Hydrate oder Addukte mit Alkoholen wie (C₁-C₄)-Alkanolen, aktive Metaboliten der Verbindungen der Formel I und auch Prodrugs und Derivate der Verbindungen der Formel I, die in vitro nicht unbedingt pharmakologische Wirkung zeigen, aber in vivo in pharmakologisch wirksame Verbindungen umgewandelt werden, beispielsweise Ester oder Amide von Carbonsäuregruppen, ein.

In einer Ausführungsform der Erfindung ist A aus NH und O ausgewählt, in einer anderen Ausführungsform ist A unter NH und S ausgewählt, in einer anderen Ausführungsform ist A unter O und S ausgewählt, in einer anderen Ausführungsform steht A für NH, in einer anderen Ausführungsform steht A für O, in einer anderen Ausführungsform steht A für S.

Die Alkandiyl-, Alkendiyl- und Alkindiylgruppen, die in der Gruppe X vorkommen, können linear oder verzweigt sein, wie bereits bezüglich derartiger Gruppen im allgemeinen angegeben, und diese Gruppen sowie Cycloalkandiylgruppen, die X repräsentieren, können über beliebige Positionen an die benachbarten Gruppen, d.h. den Heterocyclus Y und die Gruppe R² oder im Fall der Gruppe Alkandiyloxy an das Sauerstoffatom der Alkandiyloxygruppe, gebunden sein. Die benachbarten Gruppen können an das gleiche Kohlenstoffatom oder verschiedene Kohlenstoffatome in der Gruppe X gebunden sein. In einer Ausführungsform besteht die Kette von Kohlenstoffatomen in einer Alkandiyl-, Alkendiyl- und Alkindiylgruppe, die in der Gruppe X vorkommt, die den Heterocyclus Y direkt mit der Gruppe R² oder im Fall der Alkandiyloxygruppe mit dem Sauerstoffatom der Alkandiyloxygruppe verbindet, aus 1, 2, 3 oder 4 Kohlenstoffatomen, in einer anderen Ausführungsform aus 1, 2 oder 3 Kohlenstoffatomen, in einer anderen Ausführungsform aus 1 oder 2 Kohlenstoffatomen, in einer anderen Ausführungsform aus 1 Kohlenstoffatom. Im Fall einer X repräsentierenden Cycloalkandiylgruppe sind in einer Ausführungsform der Heterocyclus Y und R² an zwei Ringkohlenstoffatome gebunden, die in 1,2-Position, 1,3-Position oder 1,4-Position zueinander stehen, in einer anderen Ausführungsform in 1,2-Position oder 1,3-Position zueinander, in einer anderen Ausführungsform in 1,2-Position zueinander, in einer anderen Ausführungsform in 1,4-Position zueinander. In einer Ausführungsform ist X ausgewählt aus (C₁-C₆)-Alkandiyl, (C₂-C₆)-Alkendiyl, (C₃-C₇)-Cycloalkandiyl und (C₁-C₆)-Alkandiyloxy, in einer anderen Ausführungsform aus (C₁-C₆)-Alkandiyl, (C₂-C₆)-Alkendiyl und (C₁-C₆)-Alkandiyloxy, in einer anderen Ausführungsform aus (C₁-C₆)-Alkandiyl, (C₃-C₇)-Cycloalkandiyl und (C₁-C₆)-Alkandiyloxy, in einer Ausführungsform aus (C₁-C₆)-Alkandiyl und (C₁-C₆)-Alkandiyloxy, in einer anderen Ausführungsform aus (C₁-C₆)-Alkandiyl, (C₂-C₆)-Alkendiyl, (C₂-C₆)-Alkindiyl und (C₃-C₇)-Cycloalkandiyl, in einer anderen Ausführungsform aus (C₁-C₆)-Alkandiyl, (C₂-C₆)-Alkendiyl und (C₃-C₇)-Cycloalkandiyl, in einer anderen Ausführungsform aus (C₁-C₆)-Alkandiyl und (C₂-C₆)-Alkendiyl, in einer anderen Ausführungsform steht X für (C₁-C₆)-Alkandiyl, in einer anderen Ausführungsform steht X für (C₂-C₆)-Alkendiyl, in einer anderen Ausführungsform steht X für (C₃-C₇)-Cycloalkandiyl, und in einer anderen Ausführungsform steht X für (C₁-C₆)-Alkandiyloxy, die alle gegebenenfalls wie angegeben substituiert sind. In einer Ausführungsform ist eine (C₁-C₆)-Alkandiylgruppe, die in X vorkommt, eine (C₁-C₄)-Alkandiylgruppe, in einer anderen Ausführungsform eine (C₁-C₃)-Alkandiylgruppe, in einer anderen Ausführungsform eine (C₁-C₂)-Alkandiylgruppe. In einer Ausführungsform sind die (C₂-C₆)-Alkendiyl- und (C₂-C₆)-Alkindiylgruppen, die X repräsentieren, (C₂-C₄)-Alkendiyl- und (C₂-C₄)-Alkindiylgruppen, in einer anderen Ausführungsform (C₂-C₃)-Alkendiyl- und (C₂-C₃)-Alkindiylgruppen. In einer Ausführungsform ist eine (C₃-C₇)-Cycloalkandiylgruppe, die X repräsentiert, eine (C₃-C₆)-Cycloalkandiylgruppe, in einer anderen Ausführungsform eine (C₃-C₄)-Cycloalkandiylgruppe, in einer anderen Ausführungsform eine Cyclopropandiylgruppe, in einer anderen Ausführungsform eine Cyclohexandiylgruppe. Beispiele für Gruppen X, aus denen die jeweilige X repräsentierende Gruppe in den oben aufgeführten Ausführungsformen gewählt sein kann oder aus denen in einer anderen Ausführungsform der Erfindung X ausgewählt sein kann, sind Methylen,-CH(CH₃)- (Ethan-1,1-diyl), -CH₂-CH₂- (Ethan-1,2-diyl, 1,2-Ethylen), -C(CH₃)₂- (1-Methylethan-1,1-diyl), -CH₂-CH₂-CH₂- (Propan-1,3-diyl, 1,3-Propylen), -CH₂-CH(CH₃)- und -CH(CH₃)-CH₂- (Propan-1,2-diyl, 1,2-Propylen), die die Gruppe (C₁-C₆)-Alkandiyl exemplifizieren, -CH=CH- (Ethen-1,2-diyl), -CH=CH-CH₂- und -CH₂-CH=CH- (Prop-1-en-1,3-diyl und Prop-2-en-1,3-diyl) und -CH=C(CH₃)- und -C(CH₃)=CH- (Prop-1-en-1,2-diyl), die die Gruppe (C₂-C₆)-Alkendiyl exemplifizieren, -C=C- (Ethindiyl) und -CH₂-C≡C- und -C≡C-CH₂- (Prop-1-in-1,3-diyl und Prop-2-in-1,3-diyl), die die Gruppe (C₂-C₆)-Alkindiyl exemplifizieren, Cyclopropan-1,1-diyl, Cyclopropan-1,2-diyl und Cyclohexan-1,4-diyl, die die Gruppe (C₃-C₇)-Cycloalkandiyl exemplifizieren, -CH₂-O- (Methylenoxy), -CH₂-CH₂-O- (Ethan-1,2-diyloxy), -CH(CH₃)-O- (Ethan-1,1-diyloxy), -C(CH₃)₂-O- (1-Methylethan-1,1-diyloxy), -CH₂-CH₂-CH₂-O- (Propan-1,3-diyloxy) und -CH₂-CH₂-CH₂-CH₂-O- (Butan-1,4-diyloxy), die die Gruppe (C₁-C₆)-Alkandiyloxy exemplifizieren, wobei alle diese Gruppen gegebenenfalls wie angegeben substituiert sind. So ist in einer Ausführungsform X ausgewählt aus -CH₂-O-, -CH₂-CH₂-O-, -CH(CH₃)-O- und -C(CH₃)₂-O-, in einer anderen Ausführungsform aus -CH₂-O-, -CH₂-CH₂-O- und -CH(CH₃)-O-, in einer anderen Ausführungsform aus -CH₂-O- und -CH(CH₃)-O-, und in einer anderen Ausführungsform steht X für -CH₂-O-, wobei alle diese Gruppen gegebenenfalls wie angegeben substituiert sind und wobei das Sauerstoffatom an die Gruppe R² gebunden ist. In einer Ausführungsform ist die Zahl der Substituenten, die gegebenenfalls in X vorliegen, 1, 2, 3 oder 4, in einer anderen Ausführungsform 1, 2 oder 3, in einer anderen Ausführungsform 1 oder 2, in einer anderen Ausführungsform 1, und in einer anderen Ausführungsform ist die Gruppe X nicht durch aus Fluor und Hydroxy ausgewählte Substituenten substituiert. In einer Ausführungsform ist die Zahl der Hydroxysubstituenten in X nicht größer als 2, in einer anderen Ausführungsform nicht größer als 1. In einer Ausführungsform liegt an einem einzelnen Kohlenstoffatom in X nicht mehr als ein Hydroxysubstituent vor. In einer Ausführungsform liegen an Kohlenstoffatomen, die Teil einer Doppelbindung in der Gruppe (C₂-C₆)-Alkendiyl sind, keine Hydroxysubstituenten vor. In einer Ausführungsform liegen an dem Kohlenstoffatom in der Gruppe (C₁-C₆)-Alkandiyloxy, die an das Sauerstoffatom gebunden sind, keine Hydroxysubstituenten vor, in einer anderen Ausführungsform liegen an dem Kohlenstoffatom in der Gruppe (C₁-C₆)-Alkandiyloxy, die an das Sauerstoffatom gebunden ist, keine Substituenten vor, d.h. in dieser letztgenannten Ausführungsform sind alle Kohlenstoffatome, die nicht an das Sauerstoffatom gebunden sind, gegebenenfalls durch einen oder mehrere gleiche oder verschiedene Substituenten substituiert, die aus Fluor und Hydroxy ausgewählt sind. Die Doppelbindung in der Gruppe (C₂-C₆)-Alkendiyl kann E-Konfiguration oder Z-Konfiguration haben. In einer Ausführungsform hat sie E-Konfiguration, in einer anderen Ausführungsform hat sie Z-Konfiguration.

Der 4-gliedrige bis 7-gliedrige gesättigte oder teilweise ungesättigte, monocyclische oder bicyclische Heterocyclus Y enthält das in Formel I dargestellte Ringstickstoffatom, das die an die Gruppe X gebundene Carbonylgruppe trägt, als Ringheteroatom und zusätzlich 0, 1, 2 oder 3, in einer Ausführungsform der Erfindung 0, 1 oder 2, in einer anderen Ausführungsform 0 oder 1, in einer anderen Ausführungsform 1 oder 2, in einer anderen Ausführungsform 1, in einer anderen Ausführungsform 0 gleiche oder verschiedene weitere Ringheteroatome, die aus N, O und S ausgewählt sind, wobei ein oder zwei der zusätzlichen Ringstickstoffatome ein Wasserstoffatom oder einen (C₁-C₄)-Alkylsubstituenten tragen können und eines der Ringschwefelatome eine oder zwei Oxogruppen tragen kann und wobei der Heterocyclus gegebenenfalls an einem oder mehreren Ringkohlenstoffatomen durch gleiche oder verschiedene (C₁-C₄)-Alkylsubstituenten substituiert ist. In einer Ausführungsform sind die zusätzlichen Heteroatome, die in Y vorliegen können, aus N und O ausgewählt, in einer anderen Ausführungsform aus N und S, in einer anderen Ausführungsform aus O uns S, in einer anderen Ausführungsform sind sie N-Atome, in einer anderen Ausführungsform sind sie O-Atome, und in einer anderen Ausführungsform sind sie S-Atome. Wie für die Verbindungen der Formel I im allgemeinen gilt, können Ringheteroatome in Y in beliebigen Positionen stehen, mit der Maßgabe, daß das heterocyclische System in der Technik bekannt und stabil und als Untergruppe für den gewünschten Zweck der Verbindung der Formel I wie die Verwendung als Arzneistoff geeignet ist. In einer Ausführungsform der Erfindung können zwei Ringsauerstoffatome in Y nicht in benachbarten Ringpositionen stehen, in einer anderen Ausführungsform können zwei Ringheteroatome in Y, die aus Sauerstoff und Schwefel ausgewählt sind, nicht in benachbarten Ringpositionen stehen, und in einer anderen Ausführungsform können zwei Ringheteroatome in Y einschließlich des in Formel I dargestellten Ringstickstoffatoms nicht in benachbarten Ringpositionen vorliegen. Wenn Y gesättigt ist, enthält der Heterocyclus Y keine Doppelbindungen im Ring. Wenn Y teilweise ungesättigt ist, d.h. der Heterocyclus Y eine oder mehrere Doppelbindungen im Ring enthält, aber nichtaromatisch ist, enthält der Heterocyclus in einer Ausführungsform eine oder zwei, in einer anderen Ausführungsform eine, Doppelbindungen im Ring, die in beliebigen geeigneten Positionen vorliegen können. In einer Ausführungsform ist Y gesättigt, in einer anderen Ausführungsform ist Y teilweise ungesättigt.

In einer Ausführungsform ist Y ein 4-gliedriger bis 6-gliedriger, in einer anderen Ausführungsform ein 4-gliedriger bis 5-gliedriger, in einer anderen Ausführungsform ein 5-gliedriger bis 6-gliedriger, in einer anderen Ausführungsform ein 4-gliedriger, in einer anderen Ausführungsform ein 5-gliedriger, in einer anderen Ausführungsform ein 6-gliedriger Heterocyclus, der gesättigt oder teilweise ungesättigt und monocyclisch oder bicyclisch ist und ansonsten wie in bezug auf Y im allgemeinen angegeben definiert ist, soweit anwendbar. In einer Ausführungsform ist die Zahl der Ringglieder in einem monocyclischen Heterocyclus, der Y repräsentiert, wie in der allgemeinen Definition von Y oder in den oben aufgeführten Ausführungsformen angegeben, und ein bicyclischer Heterocyclus, der Y repräsentiert, ist 6-gliedrig bis 7-gliedrig, in einer anderen Ausführungsform 6-gliedrig, in einer anderen Ausführungsform 7-gliedrig. In bicyclischen Heterocyclen, die Y repräsentieren, kann es sich bei den Brückenkopfatomen um Stickstoffatome, bei denen es sich um ein beliebiges der Ringstickstoffatome, die neben dem in Formel I dargestellten Ringstickstoffatom vorliegen können, handeln kann, und/oder Ringkohlenstoffatome handeln. In einer Ausführungsform ist eines der Brückenkopfatome in einem bicyclischen Heterocyclus, der Y repräsentiert, ein Kohlenstoffatom, und das andere ist ein Kohlenstoffatom oder ein Stickstoffatom, in einer anderen Ausführungsform sind beide Brückenkopfatome Kohlenstoffatome. Beispiele für bicyclische Ringsysteme, aus denen in einer Ausführungsform bicyclische Heterocyclen, die Y repräsentieren, ausgewählt sind, sind Bicyclo[3.1.0]-, Bicyclo[3.2.0]-, Bicyclo[4.1.0]-, Bicyclo[2.2.1]- und Bicyclo[2.1.1]-Ringsysteme, die das in Formel I dargestellte Ringstickstoffatom und etwaige zusätzliche Ringheteroatome, die gegebenenfalls in Y vorliegen, enthalten und gesättigt oder teilweise ungesättigt sind und ansonsten wie in bezug auf Y im allgemeinen angegeben definiert sind, soweit anwendbar. In einer Ausführungsform ist ein bicyclischer Heterocyclus, der Y repräsentiert, gesättigt. In einer Ausführungsform ist Y ein monocyclischer Heterocyclus, in einer anderen Ausführungsform ist Y ein bicyclischer Heterocyclus. In einer Ausführungsform ist die Zahl der (C₁-C₄)-Alkylsubstituenten, die gegebenenfalls an Ringkohlenstoffatomen in Y vorliegen, 1, 2, 3 oder 4, in einer anderen Ausführungsform oder 2, und in einer anderen Ausführungsform ist Y an Ringkohlenstoffatomen nicht durch (C₁-C₄)-Alkylsubstituenten substituiert.

In einer Ausführungsform ist Y ein 4-gliedriger bis 7-gliedriger, gesättigter, monocyclischer oder bicyclischer Heterocyclus, der neben dem in Formel I dargestellten Ringstickstoffatom 0 oder 1 Ringheteroatome, die aus N, O und S ausgewählt sind, enthält, wobei ein zusätzliches Ringstickstoffatom ein Wasserstoffatom oder einen (C₁-C₄)-Alkylsubstituenten tragen kann und ein Ringschwefelatom eine oder zwei Oxogruppen tragen kann und wobei der Heterocyclus gegebenenfalls an einem oder mehreren Ringkohlenstoffatomen durch gleiche oder verschiedene (C₁-C₄)-Alkylsubstituenten substituiert ist. In einer anderen Ausführungsform ist Y ein 4-gliedriger bis 6-gliedriger, gesättigter, monocyclischer Heterocyclus, der keine weiteren Ringheteroatome neben dem in Formel I dargestellten Ringstickstoffatom enthält, wobei der Ring gegebenenfalls in einem oder mehreren Ringkohlenstoffatomen durch gleiche oder verschiedene (C₁-C₄)-Alkylsubstituenten substituiert ist. In einer anderen Ausführungsform ist Y ausgewählt aus einem oder mehreren der Heterocyclen Azetidin, Pyrrolidin und Piperidin, deren Ringstickstoffatom das in Formel I dargestellte Ringstickstoffatom von Y ist, das die an die Gruppe X gebundene Carbonylgruppe trägt, wobei diese Heterocyclen gegebenenfalls an Ringkohlenstoffatomen durch einen oder mehrere gleiche oder verschiedene (C₁-C₄)-Alkylsubstituenten substituiert sind, und in einer anderen Ausführungsform tragen diese Heterocyclen keine Alkylsubstituenten.

Die Gruppe R¹-O-C(O)- kann in einer beliebigen geeigneten Position an dem Heterocyclus Y gebunden sein. In einer Ausführungsform ist die Gruppe R¹-O-C(O)- an ein Ringkohlenstoffatom von Y in einer beliebigen Position gebunden, in einer anderen Ausführungsform ist sie an ein Ringkohlenstoffatom in Y gebunden, das in einer dem in Formel I dargestellten Ringstickstoffatom in Y benachbarten Ringposition steht, in einer anderen Ausführungsform ist sie an ein Ringkohlenstoffatom in Y gebunden, das dem Ringstickstoffatom benachbart ist oder von dem Ringstickstoffatom durch ein oder zwei Ringglieder getrennt ist, in einer anderen Ausführungsform ist sie an ein Ringkohlenstoffatom gebunden, das dem Ringstickstoff benachbart ist oder von dem Ringstickstoffatom durch mindestens ein Ringglied getrennt ist, in einer anderen Ausführungsform ist sie an ein Ringkohlenstoffatom in Y gebunden, das von dem Ringstickstoffatom durch ein oder zwei Ringglieder getrennt ist, und in einer anderen Ausführungsform ist sie an ein Ringkohlenstoffatom gebunden, das von dem Ringstickstoffatom durch mindestens ein Ringglied getrennt ist. So ist beispielsweise dann, wenn ein Heterocyclus Y, der keine weiteren Ringheteroatome neben dem in Formel I dargestellten Stickstoffatom enthält und in dem das Ringstickstoffatom somit in Position 1 des Heterocyclus steht, in einer Ausführungsform die Gruppe R¹-O-C(O)- an ein Ringkohlenstoffatom in beliebiger Position gebunden, in einer anderen Ausführungsform ist sie in Position 2 gebunden, in einer anderen Ausführungsform in Position 3, in einer anderen Ausführungsform in Position 4, in einer anderen Ausführungsform in Position 2 oder 3, in einer anderen Ausführungsform in Position 3 oder 4, in einer anderen Ausführungsform in Position 2, 3 oder 4. In einer Ausführungsform ist die Gruppe R¹-O-C(O)- in einem teilweise ungesättigten Heterocyclus Y an ein Ringatom gebunden, das nicht Teil einer Doppelbindung ist. In einer Ausführungsform ist die Gruppe R¹-O-C(O)- im Fall eines bicyclischen Heterocyclus Y an ein Ringatom gebunden, das kein Brückenkopfatom ist. Die Gruppe Y kann am Heterocyclus Y in einer beliebigen stereochemischen Position vorliegen, beispielsweise in endo-Position oder exo-Position im Fall eines bicyclischen Rings Y, oder in cis-Position oder in trans-Position bezüglich eines Substituenten an Y oder an einem Kohlenstoffatom mit S-Konfiguration oder einem Kohlenstoffatom mit R-Konfiguration. So liegt beispielsweise im Fall eines Rings Y, der aus Azetidin, Pyrrolidin, Piperidin und Perhydroazepin ausgewählt ist, dessen Ringstickstoffatom das in Formel I dargestellte Ringstickstoffatom von Y ist und die Gruppe R¹-O-C(O)- in Position 2 trägt, das die Gruppe R¹-O-C(O)- tragende Kohlenstoffatom in einer Ausführungsform in S-Konfiguration vor. Beispiele für die Gruppierung R¹-O-C(O)-Y-, aus denen diese Gruppierung in einer Ausführungsform in den Verbindungen der Formel I ausgewählt ist, sind 2-Hydroxycarbonylazetidin-1-yl, 2-((C₁-C₄)-Alkyloxycarbonyl)azetidin-1-yl, (S)-2-Hydroxycarbonylazetidin-1-yl, (S)-2-((C₁-C₄)-Alkyloxycarbonyl)azetidin-1-yl, 3-Hydroxycarbonylazetidin-1-yl, 3-((C₁-C₄)-Alkyloxycarbonyl)azetidin-1-yl, 2-Hydroxycarbonylpyrrolidin-1-yl, 2-((C₁-C₄)-Alkyloxycarbonyl)pyrrolidin-1-yl, (S)-2-Hydroxycarbonylpyrrolidin-1-yl, (S)-2-((C₁-C₄)-Alkyloxycarbonyl)pyrrolidin1-yl, 3-Hydroxycarbonylpyrrolidin-1-yl, 3-((C₁-C₄)-Alkyloxycarbonyl)pyrrolidin-1-yl, 2-Hydroxycarbonylpiperidin-1-yl, 2-((C₁-C₄)-Alkyloxycarbonyl)piperidin-1-yl, (S)-2-Hydroxycarbonylpiperidin-1-yl, (S)-2-((C₁-C₄)-Alkyloxycarbonyl)piperidin-1-yl, 3-Hydroxycarbonylpiperidin-1-yl, 3-((C₁-C₄)-Alkyloxycarbonyl)piperidin-1-yl, 4-Hydroxycarbonylpiperidin-1-yl, 4-((C₁-C₄)-Alkyloxycarbonyl)piperidin-1-yl, 2-Hydroxycarbonylperhydroazepin-1-yl, 2-((C₁-C₄)-Alkyloxycarbonyl)perhydroazepin-1-yl, (S)-2-Hydroxycarbonylperhydroazepin-1-yl, (S)-2-((C₁-C₄)-Alkyloxycarbonyl)perhydroazepin-1-yl, 3-Hydroxycarbonylperhydroazepin-1-yl, 3-((C₁-C₄)-Alkyloxycarbonyl)perhydroazepin-1-yl, 4-Hydroxycarbonylperhydroazepin-1-yl, 4-((C₁-C₄)-Alkyloxycarbonyl)perhydroazepin-1-yl, 2-Hydroxycarbonylmorpholin-4-yl, 2-((C₁-C₄)-Alkyloxycarbonyl)morpholin-4-yl, 3-Hydroxycarbonylmorpholin-4-yl, 3-((C₁-C₄)-Alkyloxycarbonyl)morpholin-4-yl, 2-Hydroxycarbonylthiomorpholin-4-yl, 2-((C₁-C₄)-Alkyloxycarbonyl)thiomorpholin-4-yl, 3-Hydroxycarbonylthiomorpholin-4-yl, 3-((C₁-C₄)-Alkyloxycarbonyl)thiomorpholin-4-yl, 2-Hydroxycarbonyl-1,1-dioxothiomorpholin-4-yl, 2-((C₁-C₄)-Alkyloxycarbonyl)-1,1-dioxothiomorpholin-4-yl, 3-Hydroxycarbonyl-1,1-dioxothiomorpholin-4-yl, 3-((C₁-C₄)-Alkyloxycarbonyl)-1,1-dioxothiomorpholin-4-yl, 2-Hydroxycarbonyl-4-methylpiperazin-1-yl, 2-((C₁-C₄)-Alkyloxycarbonyl)-4-methylpiperazin-1-yl, 3-Hydroxycarbonyl-4-methylpiperazin-1-yl, 3-((C₁-C₄)-Alkyloxycarbonyl)-1-methylpiperazin-1-yl.

In einer Ausführungsform der Erfindung ist die Zahl z aus 0 und 1 ausgewählt, in einer anderen Ausführungsform steht sie für 0, in einer anderen Ausführungsform steht sie für 1. In einer Ausführungsform der Erfindung ist die Gruppe R¹ aus Wasserstoff und (C₁-C₄)-Alkyl ausgewählt, in einer anderen Ausführungsform ist R¹ aus Wasserstoff, Methyl, Ethyl, n-Propyl, n-Butyl und Isopropyl ausgewählt, in einer anderen Ausführungsform aus Wasserstoff, Methyl und Ethyl, in einer anderen Ausführungsform steht R¹ für Wasserstoff, in einer anderen Ausführungsform steht R¹ für (C₁-C₄)-Alkyl, in einer anderen Ausführungsform steht R¹ für Methyl, und in einer anderen Ausführungsform steht R¹ für Ethyl. In einer Ausführungsform ist eine (C₃-C₇)-Cycloalkylgruppe, die in R¹ vorliegt, (C₃-C₆)-Cycloalkyl, in einer anderen Ausführungsform Cyclopropyl.

In einer Ausführungsform der Erfindung ist die Zahl der Ringheteroatome in einem aromatischen Heterocyclus, der R² repräsentiert, 1 oder 2, in einer anderen Ausführungsform 1. In einer Ausführungsform der Erfindung ist R² aus Phenylen und einem zweiwertigen Rest eines aromatischen, 6-gliedrigen monocyclischen Heterocyclus, der 1, 2 oder 3 Ringstickstoffatome, in einer anderen Ausführungsform 1 oder 2 Ringstickstoffatome, in einer anderen Ausführungsform 1 Ringstickstoffatom enthält, ausgewählt, wobei eines der Ringstickstoffatome einen Substituenten R²¹ tragen kann, der Oxy ist, d.h. wobei eines der Ringstickstoffatome zum N-Oxid oxidiert sein kann, und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind. In einer anderen Ausführungsform steht R² für Phenylen, wobei das Phenylen gegebenenfalls an einem oder mehreren Ringatomen durch gleiche oder verschiedene Substituenten R²² substituiert ist, und in einer anderen Ausführungsform steht R² für Pyridindiyl, wobei das Ringstickstoffatom einen Substituenten R²¹, der Oxy ist, tragen kann, d.h. wobei das Ringstickstoffatom zum N-Oxid oxidiert sein kann, und wobei das Pyridindiyl gegebenenfalls an einem oder mehreren Ringkohlenstoffatomen durch gleiche oder verschiedene Substituenten R²² substituiert ist. In einer anderen Ausführungsform steht R² für einen zweiwertigen Rest eines aromatischen 5-gliedrigen Heterocyclus, der 1, 2 oder 3 gleiche oder verschiedene Ringheteroatome, die aus N, O und S ausgewählt sind, enthält, wobei eines der Ringstickstoffatome ein Wasserstoffatom oder einen Substituenten R²¹ tragen kann und wobei der zweiwertige Rest eines aromatischen Heterocyclus gegebenenfalls an einem oder mehreren Ringkohlenstoffatomen durch gleiche oder verschiedene Substituenten R²² substituiert ist. In einer Ausführungsform ist ein zweiwertiger Rest einer aromatischen heterocyclischen Gruppe, die R² repräsentiert, aus Furandiyl, Thiophendiyl, Oxazoldiyl, Thiazoldiyl, Pyridindiyl, Pyridazindiyl, Pyrimidindiyl und Pyrazindiyl ausgewählt, in einer anderen Ausführungsform aus Furandiyl, Thiophendiyl, Thiazoldiyl, Pyridindiyl, Pyridazindiyl, Pyrimidindiyl und Pyrazindiyl, in einer anderen Ausführungsform aus Furandiyl, Thiophendiyl, Pyridindiyl, Pyridazindiyl, Pyrimidindiyl und Pyrazindiyl, in einer anderen Ausführungsform aus Furandiyl, Thiophendiyl, Pyridindiyl und Pyrimidindiyl, in einer anderen Ausführungsform aus Furandiyl, Thiophendiyl und Pyridindiyl, die alle gegebenenfalls wie in bezug auf R² angegeben substituiert sind.

Die Ringkohlenstoffatome, über die die Phenylengruppe und der zweiwertige Rest eines aromatischen Heterocyclus, die bzw. der R² repräsentieren, an den Oxazolopyrimidinring und die Gruppe X gebunden sind, können in beliebigen Positionen vorliegen. Eine R² repräsentierende Phenylengruppe kann 1,2-Phenylen sein, d.h. der Oxazolopyrimidinring und die Gruppe X können in 1,2-Position oder ortho-Position zueinander gebunden sein, sie kann 1,3-Phenylen sein, d.h. der Oxazolopyrimidinring und die Gruppe X können in 1,3-Position oder meta-Position zueinander gebunden sein, und sie kann 1,4-Phenylen sein, d.h. der Oxazolopyrimidinring und die Gruppe X können in 1,4-Position oder para-Position zueinander gebunden sein. In einer Ausführungsform ist eine R² repräsentierende Phenylengruppe ausgewählt aus 1,3-Phenylen und 1,4-Phenylen, in einer anderen Ausführungsform ist sie 1,3-Phenylen, und in einer anderen Ausführungsform ist sie 1,4-Phenylen, wobei alle diese Gruppen gegebenenfalls wie in bezug auf R² angegeben substituiert sind. In einer Ausführungsform ist R² ausgewählt aus einer oder mehreren der Gruppen Phenylen, Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus den Gruppen Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus Pyridin-2,4-diyl, Pyridin-2,5-diyl, Pyridin-3,5-diyl und Pyridin-2,6-diyl, in einer anderen Ausführungsform aus Phenylen, Pyridin-2,4-diyl, Pyridin-2,5-diyl, Pyridin-3,5-diyl und Pyridin-2,6-diyl, wobei alle diese Gruppen gegebenenfalls wie in bezug auf R² angegeben substituiert sind. In einer Ausführungsform ist die Zahl der Substituenten R²², die gegebenenfalls an Ringkohlenstoffatomen in R² vorliegen, 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Ringkohlenstoffatome in R², die keinen Substituenten R²² tragen, tragen ein Wasserstoffatom.

In einer Ausführungsform der Erfindung ist R³ aus (C₁-C₆)-Alkyl, (C₂-C₆)-Alkenyl und (C₂-C₆)-Alkinyl ausgewählt, in einer anderen Ausführungsform steht R³ für (C₁-C₆)-Alkyl, in einer anderen Ausführungsform steht R³ für (C₂-C₅)-Alkyl, und in einer anderen Ausführungsform steht R³ für (C₁-C₄)-Alkyl. In einer anderen Ausführungsform ist R³ aus (C₁-C₆)-Alkyl, (C₃-C₇)-Cycloalkyl-CᵤH₂ᵤ- und Het-CᵥH₂ᵥ- ausgewählt, in einer anderen Ausführungsform aus (C₃-C₇)-Cycloalkyl-CᵤH₂ᵤ- und Het-CᵥH₂ᵥ-, in einer anderen Ausführungsform ist R³ (C₃-C₇)-Cycloalkyl-CᵤH₂ᵤ-, und in einer anderen Ausführungsform steht R³ für Het-CᵥH₂ᵥ-, wobei in dieser Ausführungsform u und v unabhängig voneinander aus 1 und 2 ausgewählt sind. In einer Ausführungsform steht u für 1, in einer anderen Ausführungsform steht u für 2. In einer Ausführungsform steht v für 1, in einer anderen Ausführungsform steht v für 2. In einer Ausführungsform ist die Gruppe (C₃-C₇)-Cycloalkyl-CᵤH₂ᵤ-, die R³ repräsentiert, aus Cyclopropyl-CᵤH₂ᵤ-, Cyclobutyl-CᵤH₂ᵤ- und Cyclopentyl-CᵤH₂ᵤ- ausgewählt, und die Gruppe Het-CᵥH₂ᵥ-, die R³ repräsentiert, steht für Tetrahydrofuranyl-CᵥH₂ᵥ-. In einer Ausführungsform ist R³ ausgewählt aus Cyclopropyl-CᵤH₂ᵤ-, Cyclobutyl-CᵤH₂ᵤ- and Cyclopentyl-CᵤH₂ᵤ-.

In einer Ausführungsform ist R³ aus (C₃-C₇)-Cycloalkyl-CᵤH₂ᵤ- und Het-CᵥH₂ᵥ- ausgewählt, oder R³ steht für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die unter N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, und in einer anderen Ausführungsform steht R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist. In einer Ausführungsform ist die Zahl der Ringheteroatome im Ring, der R³ repräsentiert, 0, 1, 2 oder 3, in einer anderen Ausführungsform 0, 1 oder 2, in einer anderen Ausführungsform 0 oder 1, in einer anderen Ausführungsform 0, in einer anderen Ausführungsform ist sie 1, 2, 3 oder 4, in einer anderen Ausführungsform 1, 2 oder 3, in einer anderen Ausführungsform 1 oder 2, in einer anderen Ausführungsform 1. Der Rest des Rings, der R³ repräsentiert, kann somit carbocyclisch oder heterocyclisch sein. In einer Ausführungsform sind die Ringheteroatome in R³ aus N und O ausgewählt, in einer anderen Ausführungsform aus N und S, in einer anderen Ausführungsform aus O und S, in einer anderen Ausführungsform stehen sie für N, wobei Ringstickstoffatome ein Wasserstoffatom oder einen (C₁-C₄)-Alkylsubstituenten tragen können, wie es beispielsweise in gesättigten oder teilweise ungesättigten Heterocyclen oder in 5-gliedrigen aromatischen Ringen in Heterocyclen wie Pyrrol oder Benzoimidazol vorkommt, oder kein Wasserstoffatom und keinen (C₁-C₄)-Alkylsubstituenten tragen, wie es in aromatischen Heterocyclen wie beispielsweise Imidazol oder Pyridin vorkommt. In einem Rest eines R³ repräsentierenden Heterocyclus, der ein oder mehrere Ringschwefelatome enthält, ist in einer Ausführungsform eines der Ringschwefelatome nicht oxidiert oder trägt eine oder zwei Oxogruppen, und alle anderen Ringschwefelatome sind nicht oxidiert. Der Rest eines monocyclischen oder bicyclischen Rings, der R³ repräsentiert, kann über ein beliebiges geeignetes Ringkohlenstoffatom oder Ringstickstoffatom an die Gruppe A gebunden sein. In einer Ausführungsform ist er über ein Ringkohlenstoffatom gebunden, in einer anderen Ausführungsform ist er über ein Ringkohlenstoffatom oder dann, wenn A für NH steht, über ein Ringstickstoffatom gebunden, und in einer anderen Ausführungsform ist er über ein Ringstickstoffatom gebunden. Der Rest eines monocyclischen oder bicyclischen Rings, der R³ repräsentiert, kann ungesättigt sein und in diesem Fall 1, 2, 3, 4 oder 5 oder 1, 2, 3 oder 4 oder 1, 2 oder 3 oder 1 oder 2 oder 1 Doppelbindungen im Ring enthalten und kann in jedem der beiden Ringe aromatisch oder nichtaromatisch sein, oder er kann gesättigt sein und in diesem Fall keine Doppelbindungen im Ring enthalten. In einer Ausführungsform ist der Rest des Rings, der R³ repräsentiert, gesättigt oder aromatisch, in einer anderen Ausführungsform ist er gesättigt, und in einer anderen Ausführungsform ist er aromatisch. In einer Ausführungsform ist der Rest des 3-gliedrigen oder 4-gliedrigen Rings, der R³ repräsentiert, gesättigt. Wenn R³ Ringstickstoffatome enthält, die ein Wasserstoffatom oder einen (C₁-C₄)-Alkylsubstituenten tragen können, kann ein derartiges Ringstickstoffatom bzw. können zwei derartige Ringstickstoffatome vorliegen. In einer Ausführungsform ist die Zahl der fakultativen Substituenten R³¹ an Ringkohlenstoffatomen in dem R³ repräsentierenden Ring 1, 2, 3, 4, 5 oder 6, in einer anderen Ausführungsform 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1.

Der Ring, der R³ repräsentieren kann, kann 3-gliedrig, 4-gliedrig, 5-gliedrig, 6-gliedrig, 7-gliedrig, 8-gliedrig, 9-gliedrig oder 10-gliedrig sein. In einer Ausführungsform ist R³ 4-gliedrig bis 10-gliedrig, in einer anderen Ausführungsform 4-gliedrig bis 9-gliedrig, in einer anderen Ausführungsform 4-gliedrig bis 8-gliedrig, in einer anderen Ausführungsform 4-gliedrig bis 7-gliedrig, in einer anderen Ausführungsform 5-gliedrig bis 7-gliedrig, in einer anderen Ausführungsform 5-gliedrig oder 6-gliedrig, in einer anderen Ausführungsform 6-gliedrig, in einer anderen Ausführungsform 8-gliedrig bis 10-gliedrig, in einer anderen Ausführungsform 9-gliedrig bis 10-gliedrig. In einer Ausführungsform enthält ein 3-gliedriger Ring, der R³ repräsentiert, keine Ringheteroatome. In einer Ausführungsform ist R³ monocyclisch, in einer anderen Ausführungsform bicyclisch. In einer Ausführungsform ist eine bicyclische Gruppe, die R³ repräsentiert, mindestens 7-gliedrig. Unter anderem kann der Rest eines Rings, der R³ repräsentiert, eine Cycloalkylgruppe, eine Phenylgruppe, eine Naphthylgruppe, ein Rest einer ungesättigten, aromatischen oder nichtaromatischen heterocyclischen Gruppe oder ein Rest einer gesättigten heterocyclischen Gruppe sein, die alle gegebenenfalls an Ringkohlenstoffatomen und Ringstickstoffatomen wie in bezug auf R³ angegeben substituiert sind. Soweit anwendbar, gelten alle oben angegebenen Erklärungen in bezug auf derartige Gruppen entsprechend für R³. Ein anderes Beispiel für Gruppen, die R³ repräsentieren können, sind Cycloalkenylgruppen wie (C₅-C₇)-Cycloalkenylgruppen, die über ein beliebiges Ringkohlenstoffatom gebunden sein können und gegebenenfalls wie in bezug auf R³ angegeben substituiert sind. In einer Ausführungsform sind fakultative Substituenten R³¹ an einer R³ repräsentierenden Cycloalkenylgruppe aus Fluor und (C₁-C₄)-Alkyl ausgewählt. In einer Ausführungsform enthalten Cycloalkenylgruppen eine Doppelbindung im Ring, die in einer beliebigen Position vorliegen kann. Beispiele für Cycloalkenyl sind Cyclopentenyl einschließlich Cyclopent-1-enyl, Cyclopent-2-enyl und Cyclopent-3-enyl, Cyclohexenyl einschließlich Cyclohex-1-enyl, Cyclohex-2-enyl und Cyclohex-3-enyl und Cycloheptenyl einschließlich Cyclohept-1-enyl, Cyclohept-2-enyl, Cyclopent-3-enyl und Cyclohept-4-enyl. Beispiele für Reste von Ringen, aus denen R³ in einer Ausführungsform der Erfindung ausgewählt ist, sind Cyclobutyl, Cyclopentyl, Cyclohexyl, Phenyl, Oxetanyl einschließlich Oxetan-3-yl, Tetrahydrofuranyl einschließlich Tetrahydrofuran-3-yl, Tetrahydrothiophenyl einschließlich Tetrahydrothiophen-3-yl, Tetrahydropyranyl einschließlich Tetrahydropyran-4-yl, Azetidinyl einschließlich Azetidin-1-yl, Pyrrolidinyl, Piperidinyl, Imidazolidinyl, Piperazinyl, Morpholinyl einschließlich Morpholin-1-yl, Thiomorpholinyl, Furanyl einschließlich Furan-3-yl, Thiophenyl einschließlich Thiophen-3-yl, Pyrazolyl einschließlich Pyrazol-3-yl, Imidazolyl, Thiazolyl einschließlich Thiazol-2-yl, Pyridinyl einschließlich Pyridin-2-yl, Pyridin-3-yl und Pyridin-4-yl, Pyridazinyl einschließlich Pyridazin-3-yl, wobei in allen davon, sofern anwendbar, ein oder zwei der Ringstickstoffatome ein Wasserstoffatom oder (C₁-C₄)-Alkyl tragen können und wobei alle davon gegebenenfalls an einem oder mehreren Ringkohlenstoffatomen durch gleiche oder verschiedene Substituenten R³¹ substituiert sind und wobei in allen davon, sofern anwendbar, ein Ringschwefelatom nichtoxidiert sein kann, d.h. als Schwefelatom vorliegen kann, oder eine oder zwei Oxogruppen tragen kann, d.h. in Form eines Sulfoxids oder Sulfons vorliegen kann.

In einer Ausführungsform ist R³ aus Phenyl und einem Rest eines gesättigten oder ungesättigten 3-gliedrigen bis 7-gliedrigen, monocyclischen Rings ausgewählt, in einer anderen Ausführungsform aus Phenyl und einem Rest eines gesättigten oder ungesättigten 5-gliedrigen bis 7-gliedrigen, monocyclischen Rings, in einer anderen Ausführungsform aus Phenyl, Pyridinyl und einem Rest eines gesättigten 3-gliedrigen bis 7-gliedrigen, monocyclischen Rings, in einer anderen Ausführungsform aus Phenyl, Pyridinyl und einem Rest eines gesättigten 5-gliedrigen bis 7-gliedrigen, monocyclischen Rings, in einer anderen Ausführungsform aus Phenyl und Pyridinyl, in einer anderen Ausführungsform aus Phenyl und einem Rest eines gesättigten 3-gliedrigen bis 7-gliedrigen, monocyclischen Rings, in einer anderen Ausführungsform aus Phenyl und einem Rest eines gesättigten 5-gliedrigen bis 7-gliedrigen, monocyclischen Rings, in einer anderen Ausführungsform aus einer oder mehreren der Gruppen Phenyl, Cyclobutyl, Cyclopentyl, Cyclohexyl und Pyridinyl, in einer anderen Ausführungsform aus einer oder mehreren der Gruppen Phenyl, Cyclopentyl und Cyclohexyl, in einer anderen Ausführungsform aus einer oder beiden Gruppen Phenyl und Cyclohexyl, wobei in allen diesen Ausführungsformen der monocyclische Ring 1 oder 2 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei das Phenyl, das Pyridinyl, der Rest eines Rings, Cyclobutyl, Cyclopentyl und Cyclohexyl gegebenenfalls an einem oder mehreren Ringkohlenstoffatomen durch gleiche oder verschiedene Substituenten R³¹ substituiert sind und wobei Pyridinyl Pyridin-2-yl, Pyridin-3-yl und Pyridin-4-yl einschließt. In einer anderen Ausführungsform ist R³ Phenyl, das gegebenenfalls durch eine oder mehrere gleiche oder verschiedene Substituenten R³¹ substituiert ist.

In einer Ausführungsform der Erfindung ist die Zahl w aus 0 und 1 ausgewählt, in einer anderen Ausführungsform steht sie für 0, in einer anderen Ausführungsform steht sie für 1. In einer Ausführungsform ist eine (C₃-C₇)-Cycloalkylgruppe, die in R²¹ vorliegt, (C₃-C₆)-Cycloalkyl, in einer anderen Ausführungsform (C₃-C₅)-Cycloalkyl, in einer anderen Ausführungsform Cyclopropyl. In einer Ausführungsform ist R²¹ aus (C₁-C₄)-Alkyl und Oxy ausgewählt, in einer anderen Ausführungsform steht R²¹ für (C₁-C₄)-Alkyl, in einer anderen Ausführungsform für (C₁-C₃)-Alkyl, in einer anderen Ausführungsform für Methyl und in einer anderen Ausführungsform für Oxy.

In einer Ausführungsform der Erfindung sind die Substituenten R²², die gegebenenfalls an der Gruppe R² vorliegen, aus Halogen, Hydroxy, (C₁-C₄)-Alkyl-, (C₁-C₄)-Alkyloxy-, (C₁-C₄)-Alkyl-S(O)ₘ-, Amino, Nitro und Cyano ausgewählt, in einer anderen Ausführungsform aus Halogen, Hydroxy, (C₁-C₄)-Alkyl-, (C₁-C₄)-Alkyloxy-, Amino und Cyano, in einer anderen Ausführungsform aus Halogen, Hydroxy, (C₁-C₄)-Alkyl- und (C₁-C₄)-Alkyloxy-, in einer anderen Ausführungsform aus Fluor, Chlor, Hydroxy, (C₁-C₄)-Alkyl- und (C₁-C₄)-Alkyloxy-, in einer anderen Ausführungsform aus Fluor, Chlor und (C₁-C₄)-Alkyl-, und in einer anderen Ausführungsform sind sie (C₁-C₄)-Alkylsubstituenten.

In einer Ausführungsform sind 1, 2 oder 3 der Substituenten R²², in einer anderen Ausführungsform 1 oder 2 der Substituenten R²² und in einer anderen Ausführungsform 1 der Substituenten R²², die gegebenenfalls an der Gruppe R² vorliegen, wie in der allgemeinen Definition von R²² definiert und somit aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl ausgewählt, und jegliche weiteren Substituenten R²², die gegebenenfalls an der Gruppe R² vorliegen, beispielsweise 1, 2 oder 3 weitere Substituenten R²², oder 1 oder 2 weitere Substituenten R²² oder 1 weiterer Substituent R²², sind aus Halogen, Hydroxy, (C₁-C₄)-Alkyl-, (C₁-C₄)-Alkyloxy-, (C₁-C₄)-Alkyl-S(O)ₘ-, Amino, Nitro und Cyano ausgewählt, wobei alle Alkylgrupppen unabhängig voneinander gegebenenfalls durch einen oder mehrere Fluorsubstituenten substituiert sind, wie es allgemein für Alkylgruppen gilt. In einer Ausführungsform sind die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², aus Halogen, Hydroxy, (C₁-C₄)-Alkyl-, (C₁-C₄)-Alkyloxy-, (C₁-C₄)-Alkyl-S(O)ₘ-, Amino und Cyano ausgewählt. In einer Ausführungsform befinden sich die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², nicht an Ringkohlenstoffatomen in der Gruppe R², die dem Atom, über das die Gruppe R² an den in Formel I dargestellten Oxazolopyrimidinring gebunden ist, benachbart ist. In einer Ausführungsform sind die weiteren Substituenten R²², die gegebenenfalls an der Gruppe R² vorliegen, beispielsweise 1, 2 oder 3 weitere Substituenten R²² oder 1 oder 2 weitere Substituenten R²² oder 1 weiterer Substituent R²², aus Halogen, Hydroxy, (C₁-C₄)-Alkyl- und (C₁-C₄)-Alkyloxy-, Amino und Cyano ausgewählt, in einer anderen Ausführungsform aus Halogen, Hydroxy, (C₁-C₄)-Alkyl- und (C₁-C₄)-Alkyloxy-, in einer anderen Ausführungsform aus Halogen, (C₁-C₄)-Alkyl- und (C₁-C₄)-Alkyloxy-, in einer anderen Ausführungsform aus Halogen und (C₁-C₄)-Alkyl-, wobei in allen diesen Ausführungsformen alle Alkylgruppen unabhängig voneinander gegebenenfalls durch einen oder mehrere Fluorsubstituenten substituiert sind.

In einer Ausführungsform der Erfindung ist R³¹ aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)m-, Amino, (C₁-C₄)-Alkylamino, Di((C₁-C₄)-alkyl)amino, (C₁-C₄)-Alkylcarbonylamino, (C₁-C₄)-Alkyl-sulfonylamino, Cyano, (C₁-C₄)-Alkylcarbonyl, Aminosulfonyl, (C₁-C₄)-Alkyl-aminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines aromatischen Rings, der R³ repräsentiert, beispielsweise an einer Phenylgruppe oder Pyridinylgruppe, die R³ repräsentiert, unter Halogen, (C₁-C₄)-Alkyl, (C₃-C₇)-Cycloalkyl, Hydroxy, (C₁-C₄)-Alkyloxy, (C₁-C₄)-Alkyl-S(O)m-, Amino, (C₁-C₄)-Alkylamino, Di((C₁-C₄)-alkyl)amino, (C₁-C₄)-Alkylcarbonylamino, (C₁-C₄)-Alkylsulfonylamino, Cyano, (C₁-C₄)-Alkylcarbonyl, Aminosulfonyl, (C₁-C₄)-Alkylaminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)m-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines gesättigten oder nichtaromatischen ungesättigten Rings, der R³ repräsentiert, aus Halogen, (C₁-C₄)-Alkyl, (C₃-C₇)-Cycloalkyl, Hydroxy, (C₁-C₄)-Alkyloxy, Oxo, (C1-C₄)-Alkyl-S(O)ₘ-, Amino, (C₁-C₄)-Alkylamino, Di((C₁-C₄)-alkyl)amino, (C₁-C₄)-Alkyl-carbonylamino, (C₁-C₄)-Alkylsulfonylamino und Cyano ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino und Cyano, in einer anderen Ausführungsform aus Halogen, (C₁-C₄)-Alkyl, (C₃-C₇)-Cycloalkyl, Hydroxy, (C₁-C₄)-Alkyloxy und Oxo, in einer anderen Ausführungsform aus Halogen, (C₁-C₄)-Alkyl, Hydroxy, (C₁-C₄)-Alkyloxy und Oxo, in einer anderen Ausführungsform aus Fluor, Chlor, (C₁-C₄)-Alkyl, Hydroxy, (C₁-C₄)-Alkyloxy und Oxo, in einer anderen Ausführungsform aus (C₁-C₄)-Alkyl, Hydroxy und Oxo, in einer anderen Ausführungsform aus Alkyl und Hydroxy, und in einer anderen Ausführungsform stehen sie für (C₁-C₄)-Alkyl, wobei in allen diesen Ausführungsformen alle Alkylgruppen unabhängig voneinander gegebenenfalls durch einen oder mehrere Fluorsubstituenten substituiert sind. Wenn der Rest eines Rings, der R³ repräsentiert, Oxogruppen als Substituenten R³¹ enthält, liegen in einer Ausführungsform nicht mehr als zwei derartige Oxosubstituenten vor, und in einer anderen Ausführungsform liegt nicht mehr als ein derartiger Oxosubstituent vor.

In einer Ausführungsform der Erfindung sind die Ringheteroatome in Het aus N und O ausgewählt, in einer anderen Ausführungsform aus O und S, in einer anderen Ausführungsform stehen sie für O-Atome. In einer anderen Ausführungsform ist die Zahl der Ringheteroatome in Het 1. In einer Ausführungsform liegen zwei Ringsauerstoffatome in Het nicht in benachbarten Ringpositionen vor, in einer anderen Ausführungsform liegen zwei aus O und S ausgewählte Ringheteroatome nicht in benachbarten Ringpositionen vor, in einer anderen Ausführungsform liegen zwei Ringheteroatome nicht in benachbarten Ringpositionen vor. Ringstickstoffatome in Het tragen ein Wasserstoffatom oder einen Substituenten wie angegeben. In einer Ausführungsform sind fakultative Substituenten an Ringstickstoffatomen in Het (C₁-C₄)-Alkylsubstituenten. In einer Ausführungsform sind fakultative Substituenten an Ringstickstoffatomen und Ringkohlenstoffatomen in Het (C₁-C₄)-Alkylsubstituenten. In einer Ausführungsform ist die Zahl fakultativer Substituenten an Het 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Het kann über ein beliebiges geeignetes Ringkohlenstoffatom gebunden sein. In einer Ausführungsform ist Het über ein Ringkohlenstoffatom gebunden, das nicht einem Ringheteroatom benachbart ist. Het kann 4-gliedrig, 5-gliedrig, 6-gliedrig oder 7-gliedrig sein. In einer Ausführungsform ist Het 4-gliedrig oder 5-gliedrig, in einer anderen Ausführungsform 5-gliedrig bis 7-gliedrig, in einer anderen Ausführungsform 5-gliedrig oder 6-gliedrig, in einer anderen Ausführungsform 4-gliedrig. Beispiele für Het, aus denen Het in einer Ausführungsform ausgewählt ist, sind Oxetanyl einschließlich Oxetan-2-yl und Oxetan-3-yl, Tetrahydrofuranyl einschließlich Tetrahydrofuran-2-yl und Tetrahydrofuran-3-yl, Tetrahydropyranyl einschließlich Tetrahydropyran-2-yl, Tetrahydropyran-3-yl und Tetrahydropyran-4-yl, Oxepanyl einschließlich Oxepan-2-yl, Oxepan-3-yl und Oxepan-4-yl, [1,3]Dioxolanyl einschließlich [1,3]Dioxolan-2-yl und [1,3]Dioxolan-4-yl, [1,4]Dioxanyl einschließlich [1,4]Dioxan-2-yl, Thietanyl einschließlich Thietan-2-yl und Thietan-3-yl, Tetrahydrothiophenyl einschließlich Tetrahydrothiophen-2-yl und Tetrahydrothiophen-3-yl, Tetrahydrothiopyranyl einschließlich Tetrahydrothiopyran-2-yl, Tetrahydrothiopyran-3-yl und Tetrahydrothiopyran-4-yl, [1,4]Dithianyl einschließlich [1,4]Dithian-2-yl, Azetidinyl einschließlich Azetidin-2-yl und Azetidin-3-yl, Pyrrolidinyl einschließlich Pyrrolidinyl-2-yl und Pyrrolidinyl-3-yl, Piperidinyl einschließlich Piperidinyl-2-yl, Piperidinyl-3-yl und Piperidinyl-4-yl, Azepanyl einschließlich Azepan-2-yl, Azepan-3-yl und Azepan-4-yl, Oxazolidinyl einschließlich Oxazolidin-2-yl, Oxazolidin-4-yl und Oxazolidin-5-yl, Thiazolidinyl einschließlich Thiazolidin-2-yl, Thiazolidin-4-yl und Thiazolidin-5-yl, Morpholinyl einschließlich Morpholin-2-yl und Morpholin-3-yl, Thiomorpholinyl einschließlich Thiomorpholin-2-yl und Thiomorpholin-3-yl, die alle gegebenenfalls wie in bezug auf Het angegeben substituiert sind.

Gegenstand der Erfindung sind alle Verbindungen der Formel I, worin ein oder mehrere Strukturelemente wie Gruppen, Substituenten und Zahlen wie in einer der angegebenen Ausführungsformen oder Definitionen der Elemente definiert sind oder eine oder mehrere der spezifischen Bedeutungen, die hier als Beispiele für Elemente angegeben sind, besitzen, wobei alle Kombinationen einer oder mehrerer angegebener Ausführungsformen und/oder Definitionen und/oder spezifischer Bedeutungen der Elemente Gegenstand der vorliegenden Erfindung sind. Auch in bezug auf alle derartigen Verbindungen der Formel I sind alle ihre stereoisomeren Formen und Mischungen von stereoisomeren Formen in beliebigem Verhältnis und ihre physiologisch annehmbaren Salze und die physiologisch annehmbaren Solvate davon Gegenstand der vorliegenden Erfindung.

Ein Beispiel für erfindungsgemäße Verbindungen, die in bezug auf beliebige Strukturelemente wie in angegebenen Ausführungsformen der Erfindung oder Definitionen derartiger Elemente definiert sind und die Gegenstand der Erfindung sind, sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin R³ ausgewählt ist aus (C₁-C₆)-Alkyl, (C₃-C₇)-Cycloalkyl-C₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Ein anderes derartiges Beispiel sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin A aus O und S ausgewählt ist;
X ausgewählt ist aus (C₁-C₆)-Alkandiyl, (C₂-C₆)-Alkendiyl und (C₁-C₆)-Alkandiyloxy;
Y für einen 4-gliedrigen bis 7-gliedrigen, gesättigten, monocyclischen oder bicyclischen Heterocyclus steht, der neben dem in Formel I dargestellten Ringstickstoffatom 0 oder 1 Ringheteroatome, die aus N, O und S ausgewählt sind, enthält, wobei ein zusätzliches Ringstickstoffatom ein Wasserstoffatom oder einen (C₁-C₄)-Alkylsubstituenten tragen kann und ein Ringschwefelatom eine oder zwei Oxogruppen tragen kann und wobei der Heterocyclus gegebenenfalls an einem oder mehreren Ringstickstoffatomen durch gleiche oder verschiedene (C₁-C₄)-Alkylsubstituenten substituiert ist;
R² ausgewählt ist aus Phenylen und Pyridindiyl, worin das Phenylen und das Pyridindiyl gegebenenfalls an einem oder mehreren Ringstickstoffatomen durch gleiche oder verschiedene Substituenten R²² substituiert sind;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Ein weiteres derartiges Beispiel sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin A für O steht;
X aus (C₁-C₆)-Alkandiyl und (C₁-C₆)-Alkandiyloxy ausgewählt ist;
Y für einen 4-gliedrigen bis 6-gliedrigen, gesättigten, monocyclischen Heterocyclus steht, der keine weiteren Ringheteroatome neben dem in Formel I dargestellten Ringstickstoffatom enthält, wobei der Ring gegebenenfalls an einem oder mehreren Ringkohlenstoffatomen durch gleiche oder verschiedene (C₁-C₄)-Alkylsubstituenten substituiert ist;
R¹ aus Wassertoff und (C₁-C₄)-Alkyl ausgewählt ist;
R² für Phenylen steht, das gegebenenfalls an einem oder mehreren Ringkohlenstoffatomen durch gleiche oder verschiedene Substituenten R²² substituiert ist;
R³ ausgewählt ist aus (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 7-gliedrigen, monocyclischen oder bicyclischen Rings, der 0, 1 oder 2 gleiche oder verschiedene Ringheteroatome, die unter N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
Het für einen Rest eines gesättigten, 4-gliedrigen bis 6-gliedrigen, monocyclischen Heterocyclus, der 1 Ringheteroatom, das aus O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind; wobei alle Alkyl-, Alkandiyl-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

Ein weiteres derartiges Beispiel sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin A für O steht;
X aus (C₁-C₆)-Alkandiyl und (C₁-C₆)-Alkandiyloxy ausgewählt ist;
Y für einen 4-gliedrigen bis 6-gliedrigen, gesättigten, monocyclischen Heterocyclus steht, der keine weiteren Ringheteroatome neben dem in Formel I dargestellten Ringstickstoffatom enthält, wobei der Ring gegebenenfalls an einem oder mehreren Ringkohlenstoffatomen durch gleiche oder verschiedene (C₁-C₄)-Alkylsubstituenten substituiert ist;
R¹ aus Wasserstoff und (C₁-C₄)-Alkyl ausgewählt ist;
R² für Phenylen steht, das gegebenenfalls an einem oder mehreren Ringkohlenstoffatomen durch gleiche oder verschiedene Substituenten R²² substituiert ist;
R³ für einen Rest eines gesättigten oder ungesättigten, 3-gliedrigen bis 7-gliedrigen, monocyclischen Rings, der 0 oder 1 Ringheteroatom, das unter N, O und S ausgewählt ist, enthält, steht, wobei ein Ringstickstoffatom ein Wasserstoffatom oder einen (C₁-C₄)-Alkylsubstituenten tragen kann und ein Ringschwefelatom eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind; wobei alle Alkyl-, Alkandiyl-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

Ebenso gilt auch in bezug auf alle hier offenbarten spezifischen Verbindungen, wie die Beispielverbindungen, die Ausführungsformen der Erfindung repräsentieren, in denen die verschiedenen Gruppen und Zahlen in der allgemeinen Definition der Verbindungen der Formel I die in der jeweiligen spezifischen Verbindung vorliegenden spezifischen Bedeutungen besitzen, daß sie in einer beliebigen ihrer stereoisomeren Formen und/oder einer Mischung von stereoisomeren Formen in beliebigem Verhältnis und in Form ihrer physiologisch akzeptablen Salze und in Form der physiologisch akzeptablen Solvate derartiger Verbindungen oder derartiger Salze Gegenstand der vorliegenden Erfindung sind. Unabhängig davon, ob eine spezifische Verbindung hier als freie Verbindung und/oder als spezifisches Salz offenbart wird, ist sie sowohl in Form der freien Verbindung als auch in Form aller ihrer physiologisch akzeptablen Salze und bei Offenbarung eines spezifischen Salzes zusätzlich in Form dieses spezifischen Salzes und in Form der physiologisch akzeptablen Solvate einer derartigen Verbindung oder derartiger Salze Gegenstand der Erfindung. Gegenstand der Erfindung ist somit auch eine Verbindung der Formel I, die aus einer oder mehreren der hier offenbarten spezifischen Verbindungen der Formel I einschließlich der nachstehend angeführten Beispielverbindungen ausgewählt ist, und die physiologisch akzeptablen Salze davon und die physiologisch akzeptablen Solvate einer derartigen Verbindung oder derartiger Salze, wobei die Verbindung der Formel I in einer beliebigen ihrer stereoisomeren Formen oder als Mischung von stereoisomeren Formen in beliebigem Verhältnis, sofern anwendbar, Gegenstand der Erfindung ist. Ein genanntes Beispiel ist eine Verbindung der Formel I oder ein physiologisch akzeptables Salz davon oder ein physiologisch akzeptables Solvat einer solchen Verbindung oder eines solchen Salzes, die ausgewählt ist aus:
(S)-1-(2-{4-[5-(2-Fluorphenoxy)oxazolo[5,4-d]pyrimidin-2-yl]-2,6-dimethylphenoxy}-acetyl)pyrrolidin-2-carbonsäure,
(S)-1-(2-{4-[5-(2-Fluorphenoxy)oxazolo[5,4-d]pyrimidin-2-yl]-2,6-dimethylphenoxy}-propionyl)pyrrolidin-2-carbonsäure,
(S)-1-((E)-3-{4-[5-(2-Fluorphenoxy)oxazolo[5,4-d]pyrimidin-2-yl]phenyl}acryloyl)-pyrrolidin-2-carbonsäure, und
(S)-1-(3-{4-[5-(2-Fluorphenoxy)oxazolo[5,4-d]pyrimidin-2-yl]phenyl}propionyl)pyrrolidin-2-carbonsäure,
(S)-1-(2-{4-[5-(2-Fluorphenoxy)oxazolo[5,4-d]pyrimidin-2-yl]-2-methylphenoxy}-acetyl)pyrrolidin-2-carbonsäure,
(S)-1-(2-{4-[5-(2-Fluorphenoxy)oxazolo[5,4-d]pyrimidin-2-yl]phenoxy}acetyl)pyrrolidin-2-carbonsäure,
1-(2-{4-[5-(2-Fluorphenoxy)oxazolo[5,4-d]pyrimidin-2-yl]phenoxy}acetyl)piperidin-4-carbonsäure, und
(S)-1-(2-{4-[5-(2-Fluorphenoxy)oxazolo[5,4-d]pyrimidin-2-yl]phenoxy}propionyl)-pyrrolidin-2-carbonsäure,
wobei die Verbindungen (S)-1-(2-{4-[5-(2-Fluorphenoxy)oxazolo[5,4-d]pyrimidin-2-yl]-2,6-dimethylphenoxy}propionyl)pyrrolidin-2-carbonsäure und (S)-1-(2-{4-[5-(2-Fluor-phenoxy)oxazolo[5,4-d]pyrimidin-2-yl]phenoxy}propionyl)pyrrolidin-2-carbonsäure in der 1-substituierten Propionylgruppierung in S-Konfiguration oder R-Konfiguration oder einer Mischung dieser beiden Formen der Gruppierung in beliebigem Verhältnis vorliegen können.

Ein weiterer Gegenstand der vorliegenden Erfindung sind Verfahren zur Herstellung der Verbindungen der Formel I und ihrer Salze und Solvate, nach denen die Verbindungen erhältlich sind und die im folgenden umrissen werden. Bei einem Verfahren setzt man eine Verbindung der Formel II mit einer Verbindung der Formel III zu einer Verbindung der Formel I um, 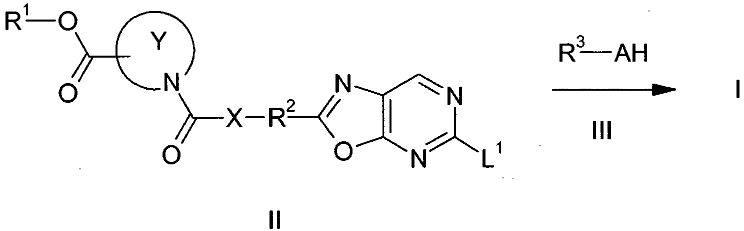 worin die Gruppen A, X, Y, R¹, R² und R³ in den Verbindungen der Formeln II und III wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in den Verbindungen der Formel II ist eine Abgangsgruppe, die in einer nukleophilen aromatischen Substitutionsreaktion ersetzt werden kann, wie ein Halogenatom, beispielsweise Chlor oder Brom, oder eine Sulfoxidgruppe oder eine Sulfongruppe, beispielsweise eine Gruppe der Formel-S(O)-Alk oder -S(O)₂-Alk, worin Alk für eine (C₁-C₄)-Alkylgruppe, beispielsweise Methyl oder Ethyl, steht.

Die Reaktion der Verbindungen der Formeln II und III ist eine nukleophile aromatische Substitutionsreaktion an dem Kohlenstoffatom in der 5-Position des Oxazolo[5,4-d]pyrimidinrings, d.h. in der Pyrimidingruppierung, und kann unter Standardbedingungen für derartige Reaktionen, die dem Fachmann gut bekannt sind, durchgeführt werden. Im allgemeinen wird die Reaktion in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie Tetrahydrofuran (THF), Dioxan, Dibutylether, Diisopropylether oder 1,2-Dimethoxyethan (DME), einem Keton wie Aceton oder Butan-2-on, einem Ester wie Essigsäureethylester oder Essigsäurebutylester, einem Nitril wie Acetonitril, einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa 20°C bis etwa 160°C, beispielsweise bei Temperaturen von etwa 40°C bis etwa 100°C, je nach den Besonderheiten des jeweiligen Falls, durchgeführt. Im allgemeinen ist es günstig, zur Erhöhung der Nukleophilie der Verbindung der Formel III eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, - hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III kann auch vor der Reaktion mit der Verbindung der Formel II separat mit einer Base behandelt und in ein Salz umgewandelt werden.

Die Ausgangsverbindungen der Formeln II und III sind nach in der Literatur beschriebenen Verfahrensweisen oder in Anlehnung daran erhältlich und in vielen Fällen im Handel erhältlich. Die Verbindungen der Formel II sind beispielsweise durch Umsetzung eines 5-Aminopyrimidinderivats der Formel IV mit einem aktivierten Carbonsäurederivat der Formel V zu einer Verbindung der Formel VI, Cyclisierung der letzteren Verbindung unter Bildung des Oxazolo[5,4-d]pyrimidinringsystems zu einer Verbindung der Formel VII, Eintragen der Gruppierung R"O-C(O)-X- in die Verbindung der Formel VII durch Umsetzung mit einer Verbindung der Formel VIII, Entschützen der erhaltenen Verbindung der Formel IX unter Erhalt der Carbonsäure der Formel X, Eintragen der Gruppierung R¹O-C(O)-Y- durch Umsetzung mit einer Verbindung der Formel XI unter Erhalt einer Verbindung der Formel XII, die bereits eine Verbindung der Formel II sein kann, in Abhängigkeit der Bedeutung von R' und L¹, und gegebenenfalls Modifizieren der Gruppe R' in der Verbindung der Formel XII unter Erhalt einer Verbindung der Formel II erhältlich.

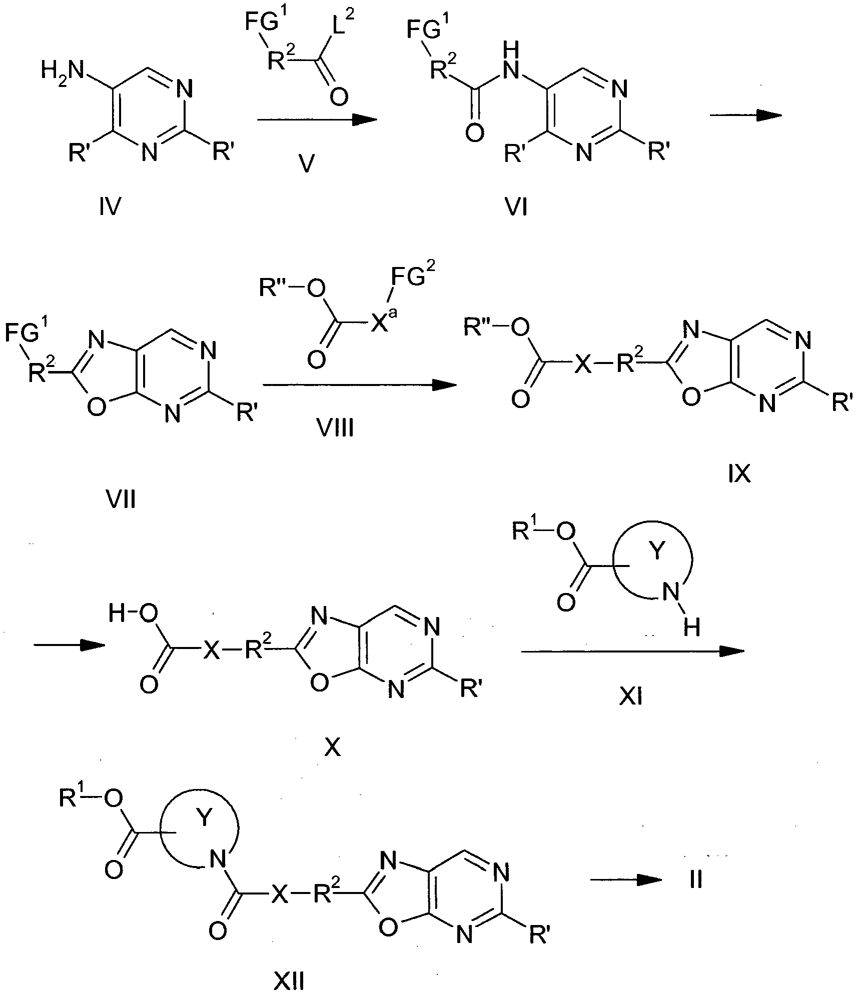

Die Reihenfolge, in der Struktureinheiten bei der Synthese einer Verbindung der Formel I eingeführt werden, kann auch anders sein; beispielsweise kann man in einer Verbindung der Formel IX oder einer Verbindung der Formel XIII, die durch Modifizierung der Gruppe R' aus einer Verbindung der Formel IX erhalten worden ist, zunächst die Gruppe -A-R³ durch Umsetzung mit einer Verbindung der Formel III einführen, die erhaltene Verbindung der Formel XIV entschützen, was die Carbonsäure der Formel XV ergibt, und dann die Gruppierung R¹O-C(O)-Y- durch Umsetzung mit einer Verbindung der Formel XI einführen.

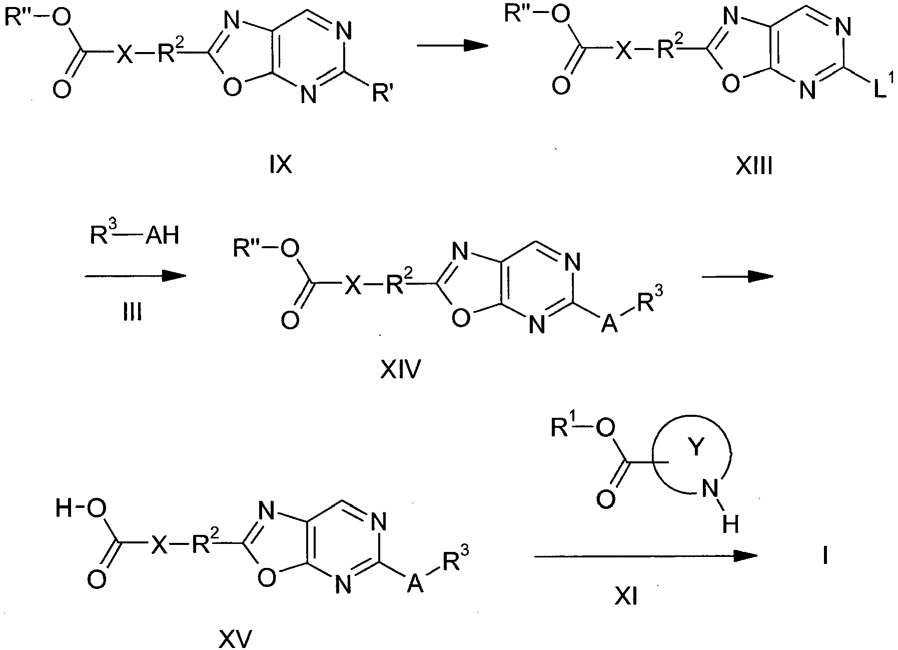

Bei einem weiteren Weg zur Synthese von Verbindungen der Formel I erfolgt die Einführung der Gruppe -A-R³ in eine Verbindung der Formel VII oder einer Verbindung der Formel XVI, die durch Modifizierung der Gruppe R' aus einer Verbindung der Formel VII erhalten worden ist, durch Umsetzung mit einer Verbindung der Formel III, wonach die erhaltene Verbindung der Formel XVII mit einer Verbindung der Formel VIII umgesetzt wird und die erhaltene Verbindung der Formel XIV wie oben angegeben weiter umgesetzt wird.

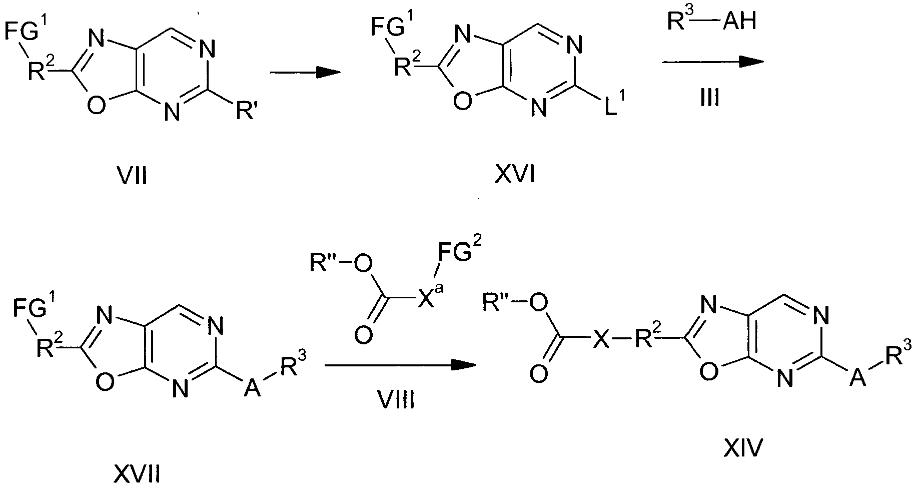

Die Gruppen A, X, Y, R¹, R² und R³ in den Verbindungen der Formeln V, VI, VII, VIII, IX, X, XI, XII, XIII, XIV, XV, XVI und XVII sind wie in den Verbindungen der Formel I definiert, und zusätzlich können funktionelle Gruppen in geschützter Form oder in Form einer Vorläufergruppe, die später in die endgültige Gruppe umgewandelt wird, vorliegen. Die Gruppe X^{a} in den Verbindungen der Formel VIII ist wie die Gruppe X in den Verbindungen der Formel I definiert oder umfaßt einen Teil der Gruppe X in der gewünschten Verbindung der Formel II, so daß nach der Umsetzung der Verbindungen der Formeln VII und VIII oder der Verbindungen der Formeln VIII und XVII die Gruppe X^{a} und jegliche in der Verbindung der Formeln IX und XIV verbleibende Teile der Gruppen FG¹ und FG² zusammen die gewünschte Gruppe X bilden. So kann es sich beispielsweise dann, wenn die Gruppe X eine Alkandiyloxygruppe ist, bei der Gruppe X^{a} in der Verbindung der Formel VIII um die gewünschte Alkandiyloxygruppe handeln, und die Gruppe FG² kann ein an das Sauerstoffatom gebundenes Wasserstoffatom sein, oder die Gruppe X^{a} kann der Alkandiylteil sein, die Gruppe FG² ist eine Abgangsgruppe, und die Gruppe FG¹ in den Verbindungen der Formeln VII und XVII ist eine Hydroxygruppe, deren Sauerstoffatom zusammen mit dem Alkandiylteil dann nach Umsetzung der Verbindung der Formel VII oder der Formel XVII mit der Verbindung der Formel VIII die gewünschte Alkandiyloxygruppe bildet.

Die Gruppen FG¹ und FG² in den Verbindungen der Formeln V, VI, VII, VIII, XVI und XVII sind funktionelle Gruppen, die für den zur Bildung der gewünschten Gruppe X aus der Gruppe X^{a} und jeglichem in den Verbindungen der Formeln IX und XIV verbleibenden Teil der Gruppen FG¹ und FG² verwendeten Kupplungstyp geeignet sind. Beispielsweise kann es sich dann, wenn die Gruppe X^{a} über eine nukleophile Substitutionsreaktion an die Gruppe R² oder an ein Atom in der Gruppe FG¹, wie ein Sauerstoffatom in einer Hydroxygruppe, die FG¹ repräsentiert, wie oben erwähnt, gebunden wird, bei FG² um eine Abgangsgruppe wie ein Halogenatom wie Chlor, Brom oder lod oder eine Sulfonyloxygruppe wie Methansulfonyloxy, Trifluormethansulfonyloxy oder Toluolsulfonyloxy handeln. Wenn die Gruppe X^{a} über eine übergangsmetallkatalysierte Reaktion an die Gruppe R² gebunden wird, kann es sich bei FG² um eine Abgangsgruppe wie eine Boronsäure-, Boronsäureester-, Dialkylboran- oder Stannangruppe handeln, und in diesem Fall kann FG¹ Halogen sein. Bei FG² kann es sich auch um ein Wasserstoffatom oder ein Kohlenstoffatom, das Teil einer Doppelbindung in einer Alkendiylgruppe ist, die X^{a} repräsentiert, handeln, wenn zur Verknüpfung von X^{a} mit R² eine Heck-Reaktion verwendet wird, und in diesem Fall kann FG¹ Halogen sein. Bei Verwendung einer Wittig-Reaktion oder Wittig-Horner-Reaktion zur Verknüpfung von X^{a} mit R² kann es sich bei FG² um eine Phosphoniogruppe wie Triphenylphosphonio oder eine Phosphonylgruppe wie Diethylphosphonyl handeln, und die Verbindung der Formel VIII kann ein Phosphoniumsalz oder ein Phosphonsäureester sein, und in diesem Fall kann FG¹ eine Aldehydgruppe -C(O)-H oder Ketongruppe -C(O)-Alkyl sein, und umgekehrt. Im allgemeinen liegt die Gruppe FG¹ an dem Kohlenstoffatom in der Phenylengruppe oder heterocyclischen Gruppe, die R² repräsentiert, die in den Verbindungen der Formeln II und I die Gruppe X trägt, vor. Die Gruppe FG¹ in den Verbindungen der Formeln V, VI, VII und XVI kann auch in geschützter Form oder in Form einer Vorläufergruppe, die später in die Gruppe umgewandelt wird, die in der Verbindung der Formel VII bzw. der Formel XVII mit der Verbindung der Formel VIII reagiert, vorliegen. So kann beispielsweise eine Hydroxygruppe, die in der Verbindung der Formeln VII und XVII FG¹ repräsentiert, in den Verbindungen der Formeln V, VI, VII und XVI in geschützter Form vorliegen, beispielsweise in Form einer veretherten Hydroxygruppe wie eines Benzylethers oder eines Alkylethers wie eines Methylethers.

Derartige Ether können dann vor der Umsetzung mit der Verbindung der Formel VIII nach dem Fachmann gutbekannten Methoden gespalten werden. Eine Zusammenfassung von Methoden zur Abspaltung von Schutzgruppen findet sich in der Literatur, beispielsweise in P. J. Kocienski, Protecting Groups (Thieme Verlag, 1994), oder T. W. Greene und P. G. M. Wuts, Protective Groups in Organic Synthesis (John Wiley & Sons, 1999).

Die Gruppe L¹ in den Verbindungen der Formeln XIII und XVI ist wie oben angegeben definiert. Die Gruppe L² in den Verbindungen der Formel V ist eine nukleophil substituierbare Abgangsgruppe und kann insbesondere ein Halogenatom, wie Chlor oder Brom, sein, und die Verbindung der Formel V kann somit ein Carbonsäurehalogenid sein. L² kann auch eine Gruppe der Formel FG¹-R²-C(O)-O sein, und die Verbindung der Formel V kann somit beispielsweise ein Carbonsäureanhydrid sein. Bei den Gruppen R' in den Verbindungen der Formeln IV, VI, VII, IX, und XII kann es sich um eine Hydroxygruppe oder ein Halogenatom, wie Chlor oder Brom, handeln.

Die Gruppe R" in den Verbindungen der Formeln VIII, IX, XIII und XIV ist zur Schützung der Carbonsäurefunktion als einen Ester, beispielsweise eine (C₁-C₄)-Alkylgruppe wie Methyl, Ethyl oder tert.-Butyl oder eine Benzylgruppe geeignet. Bei der Synthese der Verbindungen der Formel I auftretende Verbindungen, wie die Verbindung der Formel IV, können auch in einer anderen tautomeren Form vorliegen, beispielsweise in der Ketoform, für den Fall, daß die Gruppen R' in der Verbindung der Formel IV Hydroxygruppen sind. Bei der Synthese der Verbindungen der Formel I auftretende Verbindungen, einschließlich Ausgangsverbindungen, Vorläufer und Produkte, können auch in Salzform eingesetzt oder erhalten werden. Verbindungen der Formeln XI können beispielsweise in Form eines Säureadditionssalzes wie Hydrochlorid eingesetzt werden.

Die Umsetzung der Verbindungen der Formeln IV und V kann unter Standardbedingungen für die Acylierung eines Amins mit einem aktivierten Carbonsäurederivat wie einem Säurehalogenid oder -anhydrid durchgeführt werden. Im allgemeinen wird die Umsetzung in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Keton wie Aceton oder Butan-2-on, einem Ester wie Essigsäureethylester oder Essigsäurebutylester oder Wasser, oder einer Mischung von Lösungsmitteln, bei Temperaturen von etwa -10°C bis etwa 40°C, beispielsweise bei Temperaturen von etwa 0°C bis etwa 30°C durchgeführt. Im allgemeinen wird die Umsetzung unter Zugabe einer Base, beispielsweise eines tertiären Amins, wie Triethylamin, Ethyldiisopropylamin oder N-Methylmorpholin oder einer anorganischen Base wie eines Alkalimetallhydroxids, -carbonats oder - hydrogencarbonats wie Natriumhydroxid, Kaliumhydroxid, Natriumcarbonat, Kaliumcarbonat oder Natriumhydrogencarbonat durchgeführt.

Wenn die Gruppe R' in der Verbindung der Formel VI für Hydroxy steht, kann die Cyclisierung der Verbindung der Formel VI zu der Verbindung der Formel VII günstigerweise in Gegenwart eines Halogenierungsmittels wie eines Phosphorhalogenids, wie Phosphorpentachlorid oder Phosphoroxidchlorid oder einer Mischung davon, in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan bei Temperaturen von etwa 20°C bis etwa 100°C, beispielsweise Temperaturen von etwa 50°C bis etwa 80°C, durchgeführt werden. Wenn die Gruppe R' in der Verbindung der Formel VI für Halogen wie Chlor steht, kann die Cyclisierung der Verbindung der Formel VI zur Verbindung der Formel VII thermisch durchgeführt werden, beispielsweise durch Erhitzen der Verbindung der Formel VI in einem inerten Lösungsmittel wie einem Kohlenwasserstoff oder chloriertem Kohlenwasserstoff, beispielsweise Toluol, Xylol oder Chlorbenzol, oder einem Amid, beispielsweise DMF, DMA oder NMP, oder einem Nitril, beispielsweise Acetonitril, auf Temperaturen von etwa 100°C bis etwa 200°C, beispielsweise auf Temperaturen von etwa 120°C bis etwa 180°C, gegebenenfalls unter Druck und gegebenenfalls in Gegenwart einer Base, wie eines tertiären Amins, beispielsweise Triethylamin, Ethyldiisopropylamin oder N-Methylmorpholin, oder einer anorganischen Base, beispielsweise eines Alkalimetallhydroxids, -carbonats oder -hydrogencarbonats wie Natriumhydroxid, Kaliumhydroxid, Natriumcarbonat, Kaliumcarbonat oder Natriumhydrogencarbonat. Die thermische Cyclisierung kann günstigerweise in einem Mikrowellenreaktor durchgeführt werden.

Die Kupplung von Verbindungen der Formel VIII mit Verbindungen der Formeln VII und XVII kann durch Reaktionen verschiedener Typen durchgeführt werden, wie oben bereits angegeben, beispielsweise über eine Alkylierungsreaktion. So kann die Gruppe R² beispielsweise dann, wenn sie eine Hydroxygruppe, die FG¹ repräsentiert, trägt, unter Verwendung einer Verbindung der Formel VIII, in der FG² für eine für nukleophile Substitutionsreaktionen geeignete Abgangsgruppe wie ein Halogenatom wie Chlor, Brom oder lod oder eine Sulfonyloxygruppe wie Methansulfonyloxy oder Toluolsulfonyloxy steht, alkyliert werden. Die nukleophile Substitutionsreaktion an dem Kohlenstoffatom in der Verbindung der Formel VIII, die die Gruppe FG² trägt, kann unter Standardbedingungen für derartige Reaktionen, die dem Fachmann gut bekannt sind, durchgeführt werden. Im allgemeinen wird die Umsetzung je nach den Besonderheiten des jeweiligen Falls in einem inerten Lösungsmittel, beispielsweise einem Kohlenwasserstoff oder chlorierten Wasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie N,N-Dimethylformamid oder N-Methylpyrrolidin-2-on, oder einer Mischung von Lösungsmitteln bei Temperaturen von etwa 20°C bis etwa 100°C, beispielsweise bei Temperaturen von etwa 40°C bis etwa 80°C durchgeführt. Im Allgemeinen ist es günstig, zur Erhöhung der Nukleophilie der Verbindung der Formel VII oder XVII und/oder zur Bindung einer Säure, die bei der Umsetzung freigesetzt wird, eine Base, beispielsweise ein Base, beispielsweise ein tertiäres Amin, wie Triethylamin, Ethyldiisopropylamin oder N-Methylmorpholin, oder eine anorganische Base wie Alkalimetallhydrid, -hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel VII oder XVII, worin FG¹ für Hydroxy steht, kann auch vor der Umsetzung mit der Verbindung der Formel VIII separat mit einer Base behandelt und in ein Salz umgewandelt werden. Eine Verbindung der Formel VII oder VXII, worin FG¹ für Hydroxy steht, kann nicht nur durch Umsetzung mit einer Verbindung der Formel VIII, worin FG² für eine Abgangsgruppe wie angegeben steht, in eine Verbindung der Formel IX oder XIV umgewandelt werden, sondern auch durch Umsetzung mit dem entsprechenden Alkohol, d.h. mit einer Verbindung der Formel VIII, worin FG² für Hydroxy steht, unter den Bedingungen der Mitsunobu-Reaktion in Gegenwart eines Azodicarboxylats wie Diethylazodicarboxylat oder Diisopropylazodicarboxylat und eines Phosphins wie Triphenylphosphin oder Tributylphosphin in einem inerten aprotischen Lösungsmittel, beispielsweise einem Ether wie THF oder Dioxan (s. O. Mitsunobu, Synthesis (1981), 1-28). Die Kupplung von Verbindungen der Formel VIII mit Verbindungen der Formeln VII und XVII über eine übergangsmetallkatalysierte Reaktion kann auch unter den Bedingungen von palladiumkatalysierten Kreuzkupplungsreaktionen wie der Heck-, Stille- oder Suzuki-Kupplungsreaktion durchgeführt werden (siehe A. de Meijere und F. Diederich (Hrsg.), Metal-Catalyzed Cross-Coupling Reactions (Wiley-VCH, 2004)).

Die Entschützung der R"-geschützten Carbonsäuregruppe in den Verbindungen der Formeln IX und XIV zu den Verbindungen der Formeln X und XV kann nach Standardmethoden erreicht werden, die dem Fachmann gut bekannt und in den oben aufgeführten Büchern von P.J. Kocienski und T.W. Grene und P.G.M. Wuts zusammengefaßt sind, beispielsweise unter basischen Bedingungen durch Behandlung mit einem Alkalimetallhydroxid wie Natriumhydroxid oder Lithiumhydroxid im Fall von Verbindungen, in denen R" für eine Alkylgruppe wie Methyl oder Ethyl steht, durch Behandlung mit Trifluoressigsäure im Fall von Verbindungen, in denen R" für einer tert.-Butylgruppe steht, oder durch Hydrierung in Gegenwart eines Übergangsmetallkatalysators wie Palladium auf Kohle im Fall von Verbindungen, in denen R" für eine Benzylgruppe steht.

Zur Einführung der Gruppierung R¹O-C(O)-Y- wird die Carbonsäuregruppe HO-C(O)-in den Verbindungen der Formeln X und XV im allgemeinen in situ mit Hilfe eines üblichen Amidkupplungsreagens aktiviert oder in ein reaktives Carbonsäurederivat umgewandelt, das in situ hergestellt oder isoliert werden kann. So kann beispielsweise die Verbindung der Formel oder XV durch Behandlung mit Thionylchlorid, Phosphorpentachlorid, Phosphortribromid oder Oxalylchlorid in ein Säurehalogenid umgewandelt werden oder mit einem Chlorameisensäurealkylester wie Chlorameisensäureethylester oder Chlorameisensäureisobutylester behandelt werden, was ein gemischtes Anhydrid ergibt. Übliche Kupplungsreagentien, die eingesetzt werden können, sind Propanphosphonsäureanhydrid, N,N'-Carbonyldiazole wie N,N'-Carbonyldiimidazol (CDI), Carbodiimide wie 1,3-Diisopropylcarbodiimid (DIC), 1,3-Dicyclohexylcarbodiimid (DCC) oder 1-(3-Dimethylaminopropyl)-3-ethylcarbodiimid-hydrochlorid (EDC), Carbodiimide zusammen mit Additiven wie 1-Hydroxybenzotriazol (HOBT) oder 1-Hydroxy-7-azabenzotriazol (HOAT), auf Uronium basierende Kupplungsreagentien wie O-(7-Azabenzotriazol-1-yl)-N,N,N',N'-tetramethyluroniumhexafluorophosphat (HATU), O-(Benzotriazol-1-yl)-N,N,N',N'-tetramethyluroniumhexafluorophosphat (HBTU) oder O-(Cyano(ethoxycarbonyl)methylenamino)-N,N,N',N'-tetramethyluroniumtetrafluoroborat (TOTU) und auf Phosphonium basierende Kupplungsreagentien wie (Benzotriazol-1-yloxy)tris(dimethylamino)phosphoniumhexafluorophosphat (BOP), (Benzotriazol-1-yloxy)tripyrrolidinophosphoniumhexafluorophosphat (PyBOP) oder Bromtripyrrolidinophosphoniumhexafluorophosphat (PyBroP).

Die Reaktionsbedingungen für die Herstellung der Verbindungen der Formeln XII und I aus Verbindungen der Formeln X und XV und den Verbindungen der Formel XI hängen von den Besonderheiten des jeweiligen Falls ab, beispielsweise von dem eingesetzten Kupplungsreagens, und sind dem Fachmann gut bekannt. So wird die Kupplungsreaktion mit dem cyclischen Amin der Formel XI beispielsweise im Fall einer Aktivierung der Verbindung der Formel X oder XV über ein Säurechlorid oder Säurebromid im allgemeinen in einem inerten Lösungsmittel, beispielsweise einem Kohlenwasserstoff oder chlorierten Kohlenwasserstoff oder einem Ether wie den oben aufgeführten, einem Ester wie Essigsäureethylester oder Essigsäurebutylester, einem Nitril wie Acetonitril oder Wasser oder einer Mischung von Lösungsmitteln einschließlich einer Mischung von Wasser und einem organischen Lösungsmittel, das mit Wasser mischbar oder unmischbar ist, bei Temperaturen von etwa -10°C bis etwa 100°C, insbesondere bei Temperaturen von etwa 0°C bis etwa 80°C, beispielsweise bei etwa Raumtemperatur, durchgeführt. Günstigerweise wird die Umsetzung eines von der Carbonsäure der Formel oder XV abgeleiteten Säurehalogenids mit einer Verbindung der Formel XI in Gegenwart einer Base wie eines tertiären Amins wie Triethylamin, Ethyldiisopropylamin, N-Methylmorpholin oder Pyridin oder einer anorganischen Base wie eines Alkalimetallhydroxids, -carbonats oder -hydrogen-carbonats, beispielsweise Natriumhydroxid, Kaliumhydroxid, Natriumcarbonat oder Natriumhydrogencarbonat, durchgeführt. Im Fall der Aktivierung der Carbonsäuregruppe in den Verbindungen der Formel X oder XV mit Hilfe eines Amidkupplungsreagens, wie beispielsweise eines Carbodiimids oder TOTU, wird die Umsetzung im allgemeinen unter wasserfreien Bedingungen in einem inerten aprotischen Lösungsmittel, beispielsweise einem Ether wie THF, Dioxan oder DME. oder einem Amid wie DMF oder NMP, bei Temperaturen von etwa -10°C bis etwa 80°C, insbesondere bei Temperaturen von etwa 0°C bis etwa 60°C, in Gegenwart einer Base wie eines tertiären Amins, wie Triethylamin, Ethyldiisopropylamin oder N-Methylmorpholin, durchgeführt. Im Fall des Einsatzes der Verbindung der Formel XI in Form eines Säureadditionssalzes bei der Umsetzung mit der Verbindung der Formel X oder XV wird in der Regel eine ausreichende Menge einer Base zugegeben, um die freie Verbindung der Formel XI freizusetzen.

Die Verbindung der Formel XII kann bereits eine Verbindung der Formel II sein und bei der Umsetzung mit der Verbindung der Formel III eingesetzt werden, wenn sie aus einer Verbindung der Formel VI, worin R' für Halogen, wie Chlor, steht, erhalten worden ist und das Halogenatom im Cyclisierungsprodukt im Verlauf der Synthese nicht ersetzt worden ist, beispielsweise durch eine Hydroxygruppe während der Aufarbeitung, oder wenn sie aus einer Verbindung der Formel VI, worin R' für Hydroxy steht, erhalten worden ist und gleichzeitig mit der Cyclisierung die zweite Hydroxygruppe in der Verbindung der Formel VII halogeniert wird, beispielsweise durch ein Chloratom ersetzt wird, wie es bei der Cyclisierung mit Hilfe eines Phosphorhalogenids vorkommen kann. Wenn eine Verbindung der Formel VII, worin R' für Hydroxy steht, als Cyclisierungsprodukt erhalten wird, kann die Hydroxygruppe in der Verbindung der Formel XII unter Standardbedingungen in eine Abgangsgruppe umgewandelt werden, beispielsweise durch Behandlung mit einem Halogenierungsmittel wie einem Phosphorhalogenid in ein Halogenatom wie ein Chloratom oder durch Behandlung mit einem Sulfonylchlorid oder Sulfonsäureanhydrid in eine Sulfonyloxygruppe gemäß obigen Angaben. Je nach den Besonderheiten des speziellen Falls, wie der Reaktivität der spezifischen Verbindung der Formel III, die mit der Verbindung der Formel II umzusetzen ist, kann es auch vorteilhaft sein, die Gruppe R' in einer Verbindung der Formel XII oder in einer Verbindung der Formel IX, die in eine Verbindung der Formel XIII umgewandelt wird, zu modifizieren, oder in einer Verbindung der Formel VII, die in eine Verbindung der Formel XVI umgewandelt wird, selbst wenn es sich dabei bereits um eine Abgangsgruppe handelt. So kann man beispielsweise eine Verbindung der Formel VII, IX oder XII, worin R' für Halogen, wie Chlor, steht, durch Behandlung mit einer Alkansulfinsäure der Formel Alk-S(O)-OH, worin Alk für (C₁-C₄)-Alkyl steht, in eine Verbindung der Formel XVI, XIII bzw. II, worin L¹ für die Gruppe -S(O)₂-Alk steht und die dann mit einer Verbindung der Formel III umgesetzt wird, umwandeln. Eine derartige Umwandlung wird im allgemeinen in Gegenwart einer Base, wie eines Alkalimetallhydrids, -hydroxids, -carbonats oderhydrogencarbonats wie Natriumhydrid, Natriumhydroxid, Kaliumhydroxid, Natriumcarbonat, Kaliumcarbonat, Cäsiumcarbonat oder Natriumhydrogencarbonat, in einem inerten Lösungsmittel, wie einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Amid wie DMF oder NMP, oder einer Mischung von Lösungsmitteln bei Temperaturen von etwa 20°C bis etwa 150°C, beispielsweise bei Temperaturen von etwa 50°C bis etwa 120°C, durchgeführt. Eine Alkansulfinsäure kann auch vor der Umsetzung mit der Verbindung der Formel VII, IX oder XII separat mit einer Base behandelt und in ein Salz umgewandelt werden.

Die obigen Ausführungen zur Umsetzung der Verbindungen der Formeln II und III und für die einzelnen Reaktionsschritte bei der Synthese der Verbindungen der Formel II aus den Verbindungen der Formel VII gelten entsprechend für entsprechende Reaktionsschritte bei der Synthese der Verbindungen der Formel I aus den Verbindungen der Formel IX über die Verbindungen der Formeln XIII, XIV und XV und für die Synthese der Verbindungen der Formel XIV aus den Verbindungen der Formel VII über die Verbindungen der Formeln XVI und XVII, sofern nicht oben bereits erwähnt. Die Reihenfolge der Schritte bei der Herstellung der Verbindungen der Formel I kann auch anderweitig verändert werden.

Weitere Verbindungen der Formel I sind aus geeigneten, nach den obenbeschriebenen Verfahren hergestellten Verbindungen durch Funktionalisierung oder Modifizierung von enthaltenen funktionellen Gruppen nach Standardverfahrensweisen erhältlich, beispielsweise durch Veresterung, Amidierung, Hydrolyse, Veretherung, Alkylierung, Acylierung, Sulfonylierung, Reduktion, Oxidation, Umwandlung in Salze u.a. So kann beispielsweise eine Hydroxygruppe, die aus einer Ethergruppe durch Etherspaltung, beispielsweise mit Hilfe von Bortribromid, oder aus einer geschützten Hydroxygruppe durch Entschützung freigesetzt werden kann, zu einem Carbonsäureester oder einem Sulfonsäureester verestert oder verethert werden. Veretherungen von Hydroxygruppen können günstigerweise durch Alkylierung mit der jeweiligen Halogenverbindung, beispielsweise einem Bromid oder Iodid, in Gegenwart einer Base, beispielsweise eines Alkalimetallcarbonats wie Kaliumcarbonat oder Cäsiumcarbonat, in einem inerten Lösungsmittel, beispielsweise einem Amid wie DMF oder NMP oder einem Keton wie Aceton oder Butan-2-on oder mit dem jeweiligen Alkohol unter den oben angesprochenen Bedingungen der Mitsunobu-Reaktion durchgeführt werden. Eine Hydroxygruppe kann durch Behandlung mit einem Halogenierungsmittel in ein Halogenid umgewandelt werden. Ein Halogenatom kann in einer Substitutionsreaktion, bei der es sich auch um eine übergangsmetallkatalysierte Reaktion handeln kann, durch verschiedene Gruppen ersetzt werden. Eine Nitrogruppe kann zu einer Aminogruppe reduziert werden, beispielsweise durch katalytische Hydrierung. Eine Aminogruppe kann unter Standardbedingungen für die Alkylierung, beispielsweise durch Umsetzung mit einer Halogenverbindung oder durch reduktive Aminierung einer Carbonylverbindung, oder für die Acylierung oder Sulfonylierung, beispielsweise durch Umsetzung mit einem reaktiven Carbonsäurederivat wie einem Säurechlorid oder Anhydrid oder einem Sulfonsäurechlorid oder mit einer aktivierten Carbonsäure, die aus der Carbonsäure beispielsweise durch Behandlung mit einem Kupplungsmittel wie CDI, einem Carbodiimid wie DCC oder EDC, HATU, TOTU, TBTU erhältlich ist, modifiziert werden. Eine Carbonsäureestergruppe kann unter sauren oder basischen Bedingungen zu einer Carbonsäure hydrolysiert werden. Eine Carbonsäuregruppe kann wie oben erwähnt aktiviert oder in ein reaktives Derivat umgewandelt und mit einem Alkohol oder einem Amin oder Ammoniak zu einem Ester oder Amid umgesetzt werden. Ein primäres Amid kann zu einem Nitril dehydratisiert werden. Ein Schwefelatom, beispielsweise in einer Alkyl-S-Gruppe oder in einem heterocyclischen Ring, kann mit einem Peroxid wie Wasserstoffperoxid oder einer Persäure zu einer Sulfoxidgruppierung S(O) oder einer Sulfongruppierung S(O)₂ oxidiert werden. Eine Carbonsäuregruppe, eine Carbonsäureestergruppe und eine Ketongruppe können zu einem Alkohol reduziert werden, beispielsweise mit Hilfe eines komplexen Hydrids wie Lithiumaluminiumhydrid, Lithiumborhydrid oder Natriumborhydrid. Eine Verbindung der Formel I oder ein Zwischenprodukt wie eine Verbindung der Formel II, die bzw. das eine Doppelbindung oder eine Dreifachbindung in der Gruppe X enthält, die leicht über eine übergangsmetallkatalysierte Kupplungsreaktion aus einer Verbindung der Formel XIV mit einer Doppel- oder Dreifachbindung in der Gruppe X^{a} und einer Verbindung der Formel XIII gemäß obiger Beschreibung erhältlich ist, kann durch Hydrierung in Gegenwart von Hydrierkatalysator wie eines Palladiumkatalysators in eine Verbindung, in der für eine gesättigte Gruppe steht, umgewandelt werden.

Alle bei den oben beschriebenen Synthesen der Verbindungen der Formel I verwendeten Reaktionen sind dem Fachmann an sich gut bekannt und können unter Standardbedingungen gemäß oder in Analogie zu in der Literatur, beispielsweise in Houben-Weyl, Methoden der Organischen Chemie (Methods of Organic Chemistry), Thieme-Verlag, Stuttgart, oder Organic Reactions, John Wiley & Sons, New York, beschriebenen Verfahrensweisen durchgeführt werden. Falls gewünscht, können die erhaltenen Verbindungen der Formel I sowie etwaige Zwischenverbindungen nach herkömmlichen Reinigungsverfahrensweisen gereinigt werden, beispielsweise durch Umkristallisieren oder Chromatographie. Wie bereits erwähnt, können alle bei den oben beschriebenen Synthesen eingesetzten Ausgangsverbindungen und Zwischenprodukte, die eine saure oder basische Gruppe enthalten, auch in Form von Salzen eingesetzt werden und alle Zwischenprodukte und entgültigen Zielverbindungen können auch in Form von Salzen erhalten werden. Wie ebenfalls oben erwähnt, kann es je nach den Umständen des jeweiligen Falls zur Vermeidung eines unerwünschten Verlaufs einer Reaktion oder von Nebenreaktionen im Lauf der Synthese einer Verbindung im allgemeinen erforderlich oder vorteilhaft sein, funktionelle Gruppen durch die Einführung von Schutzgruppen zeitweilig zu blockieren und sie in einer späteren Stufe der Synthese wieder zu entschützen oder funktionelle Gruppen in Form von Vorläufergruppen, die später in die gewünschten funktionellen Gruppen umgewandelt werden, einzuführen. Als Beispiele für Schutzgruppen seien Aminoschutzgruppen genannt, bei denen es sich um Acylgruppen oder Alkyloxycarbonylgruppen, beispielsweise eine tert.-Butyloxycarbonylgruppe (=Boc), die durch Behandung mit Trifluoressigsäure (=TFA) abgespalten werden kann, eine Benzyloxycarbonylgruppe, die durch katalytische Hydrierung abgespalten werden kann, oder eine Fluoren-9-ylmethoxycarbonylgruppe,die durch Behandlung mit Piperidin abgespalten werden kann, handeln kann, und Schutzgruppen von Carbonsäuregruppen, die als Estergruppen, wie tert.-Butylester, die durch Behandlung mit Trifluoressigsäure entschützt werden können, oder Benzylester, die durch katalytische Hydrierung entschützt werden können, geschützt werden können. Als Beispiel für eine Vorläufergruppe sei die Nitrogruppe genannt, die durch Reduktion, beispielsweise durch katalytische Hydrierung, in eine Aminogruppe umgewandelt werden kann. Derartige Synthesestrategien und Schutzgruppen und Vorläufergruppen, die in einem bestimmten Fall geeignet sind, sind dem Fachmann bekannt.

Ein weiterer Gegenstand der vorliegenden Erfindung sind die neuen Ausgangsverbindungen und Zwischenprodukte, die bei der Synthese der Verbindungen der Formel I vorkommen, einschließlich der Verbindungen der Formeln II, III, IV, V, VI, VII, VIII, IX, X, XI, XII, XIII, XIV, XV, XVI und XVII, worin A, X, X^{a}, Y, R¹, R², R³, R', R", FG¹, FG², L¹ und L² wie oben definiert sind, in einer beliebigen ihrer stereoisomeren Formen oder einer Mischung von stereoisomeren Formen in beliebigem Verhältnis und ihre Salze und Solvate derartiger Verbindungen oder derartiger Salze und ihre Verwendung als Zwischenprodukte. Die Erfindung schließt auch alle tautomeren Formen der Zwischenprodukte und Ausgangsverbindungen ein. Alle oben bezüglich der Verbindungen der Formel I angegebenen Erklärungen und Ausführungsformen gelten entsprechend auch für die Zwischenprodukte und Ausgangsverbindungen. Gegenstand der Erfindung sind insbesondere die hier offenbarten neuen spezifischen Ausgangsverbindungen und Zwischenprodukte. Unabhängig davon, ob sie als freie Verbindung und/oder als spezifisches Salz offenbart sind, sind sie sowohl in Form der freien Verbindungen als auch in Form ihrer Salze und im Fall der Offenbarung eines spezifischen Salzes zusätzlich in Form dieses spezifischen Salzes und in Form von Solvaten derartiger Verbindungen oder derartiger Salze Gegenstand der Erfindung.

Die Verbindungen der Formel I, gegebenenfalls in Kombination mit anderen pharmakologisch wirksamen Verbindungen, können Tieren, vorzugsweise Säugetieren einschließlich Menschen, als Pharmazeutika für sich alleine, in Mischungen miteinander oder in Form pharmazeutischer Zusammensetzungen verabreicht werden. Die Verabreichung kann oral, beispielsweise in Form von Tabletten, Filmtabletten, Dragees, Granulaten, Hart- und Weichgelatinekapseln, Lösungen einschließlich wäßriger, alkoholischer und öliger Lösungen, Säften, Tropfen, Sirupen, Emulsionen oder Suspensionen, rektal, beispielsweise in Form von Suppositorien, oder parenteral, beispielsweise in Form von Lösungen zur subkutanen, intramuskulären oder intravenösen Injektion oder Infusion, insbesondere wäßrigen Lösungen, durchgeführt werden. Die Verbindungen der Formel I können des weiteren in Modi der lokalen Arzneistoffzufuhr verwendet werden, beispielsweise in beschichteten Stents zur Verhinderung oder Verringerung der In-Stent-Restenose oder durch lokale Anwendung mit Hilfe eines Katheters. Die geeignete Verabreichungsform hängt u.a. von der zu behandelnden Erkrankung und ihrer Schwere ab.

Die Menge einer Verbindung der Formel I und/oder ihrer physiologisch akzeptablen Salze und/oder Solvate in den pharmazeutischen Zusammensetzungen liegt normalerweise im Bereich von etwa 0,2 bis etwa 800 mg, beispielsweise von etwa 0,5 bis etwa 500 mg, beispielsweise von etwa 1 bis etwa 200 mg, pro Einheitsdosis, kann aber je nach Art der pharmazeutischen Zusammensetzung auch höher sein. Die pharmazeutischen Zusammensetzungen enthalten in der Regel etwa 0,5 bis etwa 90 Gew.-% der Verbindung der Formel I und/oder ihrer physiologisch akzeptablen Salze und/oder Solvate. Die pharmazeutischen Zusammensetzungen können auf an sich bekannte Art und Weise hergestellt werden. Hierzu bringt man eine oder mehrere Verbindungen der Formel I und/oder ihre physiologisch akzeptablen Salze und/oder Solvate zusammen mit einer oder mehreren festen oder flüssigen pharmazeutischen Trägersubstanzen oder Vehikeln und/oder Additiven oder Hilfssubstanzen und dann, wenn ein Kombinationsmedikament gewünscht ist, anderen pharmakologisch wirksamen Verbindungen mit therapeutischer oder prophylaktischer Wirkung in eine für die Verabreichung und Dosierung geeignete Form, die dann in der Human- oder Tiermedizin verwendet werden kann. Als Trägersubstanzen und Additive können geeignete organische und anorganische Substanzen verwendet werden, die mit den Verbindungen der Formel I oder ihren physiologisch akzeptablen Salzen oder Solvaten nicht in unerwünschter Weise reagieren. Als Beispiele für Additivtypen, die in den pharmazeutischen Zusammensetzungen und Medikamenten enthalten sein können, seien Gleitmittel, Konservierungsstoffe, Verdicker, Stabilisatoren, Sprengmittel, Netzmittel, Mittel zur Erzielung eines Depoteffekts, Emulgatoren, Salze, beispielsweise zur Beeinflussung des osmotischen Drucks, Puffersubstanzen, Farbmittel, Geschmacksstoffe und aromatische Substanzen genannt. Beispiele für Trägersubstanzen und Additive sind Wasser, physiologische Natriumchloridlösung, Pflanzenöle, Wachse, Alkohole wie Ethanol, Isopropanol, 1,2-Propandiol, Benzylalkohole oder Glycerin, Polyole, Mannit, Polyethylenglykole, Polypropylenglykole, Glycerintriacetat, Polyvinylpyrrolidon, Gelatine, Cellulose, Kohlenhydrate wie Lactose, Glucose, Saccharose oder Stärke wie Maisstärke, Stearinsäure und Stearinsäuresalze wie Magnesiumstearat, Talk, Lanolin, Vaseline oder Mischungen davon, beispielsweise Mischungen von Wasser mit einem oder mehreren organischen Lösungsmitteln wie Mischungen von Wasser mit Alkoholen. Man kann die Verbindungen der Formel I und ihre physiologisch akzeptablen Salze und Solvate auch lyophilisieren und die erhaltenen Lyophilisate beispielsweise zur Herstellung von Injektionszusammensetzungen verwenden.

Die Dosierung einer zu verabreichenden Verbindung der Formel I und/oder eines physiologisch akzeptablen Salzes und/oder Solvats davon hängt vom Einzelfall ab und ist wie üblich zur Erzielung einer optimalen Wirkung vom Arzt nach den üblichen Regeln und Verfahrensweisen den individuellen Gegebenheiten anzupassen. So hängt sie beispielsweise ab von der Art und Schwere der zu behandelnden Störung, von Geschlecht, Alter, Gewicht und individueller Ansprechbarkeit des zu behandelnden Menschen oder Tiers, von der Effizienz und Wirkdauer der verwendeten Verbindung, davon, ob die Behandlung für die Therapie einer akuten oder chronischen Erkrankung oder prophylaktisch ist, oder davon, ob neben einer Verbindung der Formel I weitere Wirkstoffe verabreicht werden. Im allgemeinen ist eine Tagesdosis von beispielsweise etwa 0,01 mg/kg bis etwa 100 mg/kg oder von etwa 0,1 mg/kg bis etwa 10 mg/kg oder von etwa 0,3 mg/kg bis etwa 5 mg/kg (jeweils mg pro kg Körpergewicht) zur Verabreichung an einen 75 kg schweren Erwachsenen zur Erzielung der gewünschten Ergebnisse angemessen. Die Tagesdosis kann dabei als Einzeldosis verabreicht oder, insbesondere bei Verabreichung größerer Mengen, in mehrere, beispielsweise zwei, drei oder vier, Einzeldosen aufgeteilt werden. Die Verabreichung kann auch kontinuierlich durchgeführt werden, beispielsweise durch kontinuierliche Infusion oder Injektion. Im Einzelfall kann es je nach dem individuellen Verhalten erforderlich sein, von den angegebenen Dosierungen nach oben oder nach unten abzuweichen.

Die folgenden Beispiele erläutern die Erfindung.

Wenn Beispielsverbindungen mit einer basischen Gruppe durch präparative Hochdruck-Flüssigkeitschromatographie (HPLC) an Umkehrphasen-Säulenmaterial (RP-Säulenmaterial) gereinigt wurden und es sich bei dem Elutionsmittel wie üblich um eine Gradientenmischung von Wasser und Acetonitril mit Trifluoressigsäure (TFA) handelte, wurden sie je nach den Einzelheiten der Aufarbeitung wie Verdampfungs-oder Lyophilisierungsbedingungen zum Teil in Form ihres Säureadditionssalzes mit Trifluoressigsäure erhalten. In den Namen der Beispielverbindungen und ihren Strukturformeln ist jegliche derartige enthaltene Trifluoressigsäure nicht angegeben.

Die hergestellten Verbindungen wurden im allgemeinen durch spektroskopische Daten und chromatografische Daten, insbesondere Massenspektrum (MS) und HPLC-Retentionszeiten (Rt; in min), die durch kombinierte analytische HPLC/MS-Charakterisierung (LC/MS) erhalten wurden, und/oder NMR-Spektren (NMR=kernmagnetische Resonanz), charakterisiert. Bei der NMR-Charakterisierung sind die chemische Verschiebung δ (in ppm), die Zahl der Wasserstoffatome und die Multiplizität (s = Singulett, d = Dublett, dd = doppeltes Dublett, t = Triplett, dt = doppeltes Triplett, q = Quartett, m = Multiplett; br = breit) der Signale angegeben. Bei der MS-Charakterisierung ist im allgemeinen die Massenzahl (m/z) des Peaks des Molekülions M, z.B. M⁺, oder eines verwandten Ions wie des Ions M+1, z.B. [M+1]⁺, d.h. des protonierten Molekülions [M+H]⁺, das je nach der verwendeten Ionisierungsmethode gebildet wurde, angegeben. Bei der Ionisierungsmethode handelte es sich im allgemeinen um Elektrospray-Ionisierung (ESI). Es wurden die folgenden LC/MS-Bedingungen verwendet.

### Methode LC1

Säule: UPLC BEH C18, 50 x 2,1 mm, 1,7 µm; Durchfluß: 0,9 ml/min; Elutionsmittel A: Acetonitril + 0,08 % Ameisensäure; Elutionsmittel B: Wasser + 0,1 % Ameisensäure; Gradient: von 5% A + 95% B bis 95% A + 5% B in 1,1 min, dann von 95% A + 5% B über einen Zeitraum von 0,6 min; MS-Ionisierungsmethode: ESI⁺

### Methode LC2

Säule: Merck Chromolith FastGrad. RP-18e, 50 x 2 mm; Durchfluß: 2 ml/min; Elutionsmittel A: Acetonitril + 0,05% TFA; Elutionsmittel B: Wasser + 0,05 % TFA; Gradient: 2% A + 98% B über einen Zeitraum von 0,2 min, dann von 2% A + 98% B bis 98% A + 2% B in 2,2 min, dann 98% A + 2% B über einen Zeitraum von 0,8 min; MS-Ionisationsmethode: ESI⁺

### Methode LC3

Säule: YMC-Pack J'sphere H80, 33 x 2,1 mm, 4 µm; Durchfluß: 1,0 ml/min; Elutionsmittel A: Acetonitril + 0,05% TFA; Elutionsmittel B: Wasser + 0,05% TFA; Gradient: von 2% A + 98% B bis 95% A + 5% B in 5 min, dann 95% A + 5% B über einen Zeitraum von 1,25 min; MS-Ionisationsmethode: ESI⁺

### Method LC4

Säule: Waters XBridge C18, 50 x 4,6 mm, 2,5 µm; Durchfluß: 1,3 ml/min; Elutionsmittel A: Acetonitril + 0,05% TFA; Elutionsmittel B: Wasser + 0,05% TFA; Gradient: 5% A + 95% B über einen Zeitraum von 0,3 min, dann von 5% A + 95% B bis 95% A + 5% B in 3,2 min, dann 95% A + 5% B über einen Zeitraum von 0,5 min; MS-Ionisationsmethode: ESI⁺

### Methode LC5

Säule: UPLC BEH C18, 50 x 2,1 mm, 1,7 µm; Durchfluß: 0,9 ml/min; Elutionsmittel A: Acetonitril + 0,035 % Ameisensäure; Elutionsmittel B: Wasser + 0,05% Ameisensäure; Gradient: von 5% A + 95% B bis 95% A + 5% B in 1,1 min, dann 95% A + 5% B für 0,6 min; MS-Ionisationsmethode: ESI⁺

### Methode LC6

Säule: Waters Xbridge C18, 50 x 4,6 mm, 2,5 µm; Durchfluß: 1,3 ml/min; Elutionsmittel A: Acetonitril + 0,1 % Ameisensäure; Elutionsmittel B: Wasser + 0,1% Ameisensäure; Gradient: von 3% A + 97% B bis 60% A + 40% B in 3,5 min, von 60% A + 40% B bis 98% A + 2% B in 0,5 min, dann bis 98% A + 2% B frü 1 min; MS-Ionisationsmethode: ESI⁺

### Beispiel 1

### (S)-1-(2-{4-[5-(2-Fluorphenoxy)oxazolo[5,4-d]pyrimidin-2-yl]-2,6-dimethyl-phenoxy}acetyl)pyrrolidin-2-carbonsäure

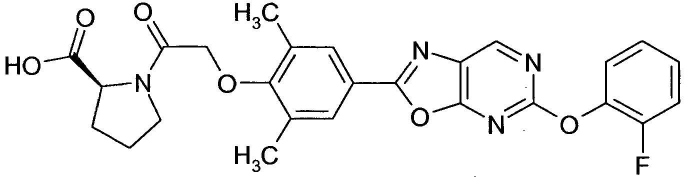

(a) N-(2,4-Dichlorpyrimidin-5-yl)-4-methoxy-3,5-dimethylbenzamid Eine Mischung von 25 ml gesättigter wäßriger Natriumhydrogencarbonatlösung in 25 ml Wasser wurde mit einer Lösung von 3,2 g 5-Amino-2,4-dichlorpyrimidin und 50 ml Essigsäureethylester versetzt. Dann wurde über einen Zeitraum von 15 min bei Raumtemperatur eine Lösung von 4,9 g 3,5-Dimethyl-4-methoxybenzoylchlorid zugegeben. Die Mischung wurde 4 h intensiv gemischt. Dann wurden die Schichten getrennt, wonach die wäßrige Schicht zweimal mit Essigsäureethylester extrahiert wurde. Nach Trocknen über Natriumsulfat und Filtrieren wurde das Lösungsmittel im Vakuum abgezogen, was 7,54 g Rohprodukt ergab. Das Rohprodukt wurde mit 25 ml Isopropanol trituriert. Nach Filtrieren und Waschen mit 10 ml Isopropanol wurden 2,74 g der Titelverbindung in Form eines weißen Feststoffs erhalten.
(b) 5-Chlor-2-(4-methoxy-3,5-dimethylphenyl)oxazolo[5,4-d]pyrimidin Eine Lösung von 2,74 g N-(2,4-Dichlorpyrimidin-5-yl)-4-methoxy-3,5-dimethyl-benzamid und 3,2 ml N,N-Diisopropylethylamin in 17 ml Acetonitril wurde in zwei Chargen aufgeteilt, die jeweils in einem Mikrowellenreaktor 1 h auf 160°C erhitzt wurden. Nach Wiedervereinigung der Chargen wurde der Niederschlag abfiltriert, was 600 mg der Titelverbindung in Form eines dunklen, aber recht reinen Feststoffs (600 mg) ergab. Nach Abziehen der Lösungsmittel von der Mutterlauge im Vakuum wurde der Rückstand Kieselgelchromatographie (Heptan/Essigsäureethylester-Gradient) unterworfen, was weitere 600 mg der Titelverbindung in Form eines blaßgelben Feststoffs ergab.
(c) 5-(2-Fluorphenoxy)-2-(4-methoxy-3,5-dimethylphenyl)oxazolo[5,4-d]pyrimidin Eine Lösung von 418 mg of 2-Fluorphenol in 15 ml Dimethylacetamid wurde unter Argonatmosphäre mit 149 mg Natriumhydrid versetzt. Nach 30 min Rühren bei Raumtemperatur wurde langsam eine Lösung von 900 mg 5-Chlor-2-(4-methoxy-3,5-dimethylphenyl)oxazolo[5,4-d]pyrimidin in 20 ml Dimethylacetamid zugegeben. Die Mischung wurde 1,5 h bei Raumtemperatur rühren gelassen. Nach Verbrauch des als Ausgangsstoff dienenden Oxazolo[5,4-d]pyrimidins wurde wäßrige Citronensäurelösung (100 g/l) zugegeben, bis der pH-Wert neutral war. Die wäßrige Schicht wurde zweimal mit 15 ml Essigsäureethylester extrahiert. Die vereinigten organischen Schichten wurden über Natriumsulfat getrocknet, filtriert und im Vakuum von den Lösungsmitteln befreit. Die Titelverbindung wurde durch Kieselgelchromatographie (Heptan/Essigsäureethylester-Gradient) in Form eines weißen Feststoffs isoliert, Ausbeute: 820 mg.
   LC/MS (Methode LC4): Rt = 3,56 min; m/z = 366,0 [M+H]⁺
(d) 4-[5-(2-Fluorphenoxy)oxazolo[5,4-d]pyrimidin-2-yl]-2,6-dimethylphenol Eine Lösung von 510 mg 5-(2-Fluorphenoxy)-2-(4-methoxy-3,5-dimethylphenyl)-oxazolo[5,4-d]pyrimidin in 20 ml Dichlormethan wurde auf 0°C abgekühlt und über einen Zeitraum von 10 min mit 4,2 ml einer 1 M Lösung von Bortribromid in Dichlormethan versetzt. Die Mischung wurde 1 h bei 0°C gerührt und dann mit weiteren 2 ml einer 1 M Lösung von Bortribromid in Dichlormethan versetzt. Nach einer weiteren Stunde Rühren wurden langsam 10 ml gesättigte wäßrige Natriumhydrogencarbonatlösung zugegeben. Die wäßrige Schicht wurde zweimal mit 15 ml Dichlormethan extrahiert. Die vereinigten organischen Schichten wurden über Natriumsulfat getrocknet, filtriert und im Vakuum von den Lösungsmitteln befreit, was 445 mg der Titelverbindung in Form eines weißen Feststoffs ergab.
   LC/MS (Methode LC3): Rt = 3,62 min; m/z = 352,13 [M+H]⁺
(e) {4-[5-(2-Fluorphenoxy)oxazolo[5,4-d]pyrimidin-2-yl]-2,6-dimethylphenoxy}-essigsäure
   Eine Suspension von 430 mg 4-[5-(2-Fluorphenoxy)oxazolo[5,4-d]pyrimidin-2-yl]-2,6-dimethylphenol, 0,2 ml Bromessigsäure-tert.-butylester und 423 mg Kaliumcarbonat in 5 ml Dimethylformamid wurde 2 h bei 60°C gerührt. Nach Abkühlen wurden die Feststoffe abfiltriert und das Lösungsmittel im Vakuum abdestilliert. Der Rückstand wurde mit 1,3 ml Trifluoressigsäure versetzt, wonach die Lösung 1 h bei Raumtemperatur gerührt wurde. Nach Zugabe von 10 ml Toluol wurden die Lösungsmittel im Vakuum abgezogen. Die Titelverbindung wurde durch Kieselgelchromatographie (Heptan/Essigsäureethylester-Gradient) in Form eines weißen Feststoffs (135 mg) isoliert.
   LC/MS (Methode LC1): Rt = 1,26 min; m/z = 410,21 [M+H]⁺
(f) {4-[5-(2-Fluorphenoxy)oxazolo[5,4-d]pyrimidin-2-yl]-2,6-dimethylphenoxy}acetyl-chlorid
   336 mg {4-[5-(2-Fluorphenoxy)oxazolo[5,4-d]pyrimidin-2-yl]-2,6-dimethylphenoxy}-essigsäure wurden langsam mit 3 ml Thionylchlorid versetzt. Die Mischung wurde 2 Tage reagieren gelassen. Dann wurde Toluol zugegeben, wonach die Lösungsmittel im Vakuum abgezogen wurden. Die Zugabe und Entfernung von Toluol wurde wiederholt, was 350 mg der Titelverbindung in Form eines braunen Öls ergab.
(g) (S)-1-(2-{4-[5-(2-Fluorphenoxy)oxazolo[5,4-d]pyrimidin-2-yl]-2,6-dimethyl-phenoxy}acetyl)pyrrolidin-2-carbonsäure
   Eine Suspension von 102 mg Prolin-tert.-butylester-hydrochlorid und 170 µl Triethylamin in 5 ml Dichlormethan wurde langsam mit einer Lösung von 175 mg {4-[5-(2-Fluorphenoxy)oxazolo[5,4-d]pyrimidin-2-yl]-2,6-dimethylphenoxy}acetylchlorid in 2 ml Dichlormethan versetzt. Nach 2 h Rühren wurde das Lösungsmittel im Vakuum abgezogen. Nach Zugabe von 1,6 ml Trifluoressigsäure wurde die Mischung 2 h bei Raumtemperatur gerührt. Dann wurden 10 ml Wasser zugegeben, wonach die wäßrige Schicht dreimal mit 15 ml einer Mischung von Dichlormethan und Isopropanol im Verhältnis 3:1 extrahiert wurde. Nach Trocknen mit Natriumsulfat und Filtrieren wurden die Lösungsmittel im Vakuum abgezogen, wonach die Titelverbindung mittels präparativer HPLC isoliert wurde. Ausbeute: 56 mg weißer Feststoff.
   LC/MS (Methode LC2): Rt = 1,64 min; m/z = 507,16 [M+H]⁺

### Beispiel 2

### (S)-1-(2-{4-[5-(2-Fluorphenoxy)oxazolo[5,4-d]pyrimidin-2-yl]-2,6-dimethylphenoxy}-propionyl)pyrrolidin-2-carbonsäure

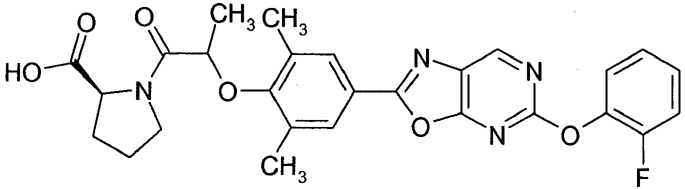

Die Titelverbindung wurde in Analogie zu Beispiel 1 unter Verwendung von 2-Brompropionsäure-tert.-butylester anstelle von Bromessigsäure-tert.-butylester in Schritt (e) hergestellt.
LC/MS (Methode LC1): Rt = 1,24 min; m/z = 521,37 [M+H]⁺

### Beispiel 3

### (S)-1-((E)-3-{4-[5-(2-Fluorphenoxy)oxazolo[5,4-d]pyrimidin-2-yl]phenyl}acryloyl)pyrrolidin-2-carbonsäure

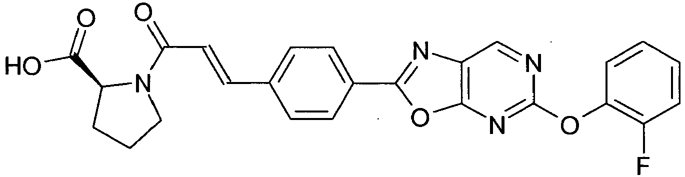

(a) 2-(4-Bromphenyl)-5-chloroxazolo[5,4-d]pyrimidin
   Die Titelverbindung wurde wie in Beispiel 1, Schritte (a) und (b) beschrieben unter Verwendung von 4-Brombenzoylchlorid in Schritt (a) hergestellt.
(b) 2-(4-Bromphenyl)-5-(2-fluorphenoxy)oxazolo[5,4-d]pyrimidin Eine Lösung von 162 µl 2-Fluorphenol in 10 ml Dimethylacetamid wurde unter Argonatmosphäre mit 70 mg Natriumhydrid versetzt. Nach 30 min Rühren bei Raumtemperatur wurde langsam eine Suspension von 450 mg 2-(4-Bromphenyl)-5-chloroxazolo[5,4-d]pyrimidin in 5 ml Dimethylacetamid zugegeben. Die Mischung wurde 2 h bei Raumtemperatur rühren gelassen. Nach Verbrauch des als Ausgangsstoff dienenden Oxazolo[5,4-d]pyrimidins wurde wäßrige Citronensäurelösung (100 g/l) zugegeben, bis der pH-Wert neutral war. Der bei Zugabe der Citronensäurelösung gebildete Niederschlag wurde abfiltriert und mit Wasser gewaschen, was 472 mg der Titelverbindung ergab.
   LC/MS (Methode LC1): Rt = 1,38 min; m/z = 386,1 [M+H]⁺
(c) (E)-3-{4-[5-(2-Fluorphenoxy)oxazolo[5,4-d]pyrimidin-2-yl]phenyl}arcylsäure-tert.-butylester
   300 mg 2-(4-Bromphenyl)-5-(2-fluorphenoxy)oxazolo[5,4-d]pyrimidin, 36 mg Tris(dibenzylidenaceton)dipalladium(0) und 23 mg Tri-tert.-butylphosphonium-tetrafluoroborat wurden in ein Reaktionsfläschchen gegeben, das mit einem Septum verschlossen und dreimal evakuiert und mit Argon gefüllt wurde. Die Feststoffe wurden in 5,5 ml entgastem 1,4-Dioxan gelöst, das mit Hilfe einer Spritze zugegeben wurde. Danach wurden über eine Spritze 0,124 ml Acrylsäure-tert.-butylester und 0,181 ml N,N-Dicyclohexylmethylamin zugegeben, wonach die Mischung in einem Mikrowellenreaktor 6 h auf 120°C erhitzt wurde. Nach Abkühlen wurde die Mischung mit 100 ml Essigsäureethylester verdünnt, über Kieselgel filtriert und im Vakuum aufkonzentriert. Der Rückstand wurde mittels präparativer HPLC gereinigt. Es wurden 147 mg der Titelverbindung erhalten.
   LC/MS (Methode LC1): Rt = 1,44 min; m/z = 434,14 [M+H]⁺
(d) (E)-3-{4-[5-(2-Fluorphenoxy)oxazolo[5,4-d]pyrimidin-2-yl]phenyl}essigsäure Eine Lösung von 145 mg (E)-3-{4-[5-(2-Fluorphenoxy)oxazolo[5,4-d]pyrimidin-2-yl]-phenyl}arcylsäure-tert-butylester in 2 ml Dichlormethan und 1 ml Trifluoressigsäure wurde 1,5 h bei Raumtemperatur gerührt. Dann wurde die Mischung im Vakuum aufkonzentriert und gefriergetrocknet. Es wurden 154 mg der Titelverbindung erhalten. LC/MS (Methode LC1): Rt = 1,25 min; m/z = 378,08 [M+H]⁺
(e) (S)-1-((E)-3-{4-[5-(2-Fluorphenoxy)oxazolo[5,4-d]pyrimidin-2-yl]phenyl}acryloyl)-pyrrolidin-2-carbonsäure-tert.-butylester
   140 mg {(E)-3-{4-[5-(2-Fluorphenoxy)oxazolo[5,4-d]pyrimidin-2-yl]phenyl}acrylsäure wurden in 2 ml N,N-Dimethylformamid gelöst und mit 98 mg 1-Ethyl-3-(3-dimethylaminopropyl)carbodiimid-hydrochlorid, 19 mg 1-Hydroxy-7-azabenzotriazol und 397 µl N,N-Diisopropylethylamin und 5 min danach mit 49 mg L-Prolin-tert.-butylester versetzt. Nach 16 h bei Raumtemperatur wurden 10 ml Wasser zugegeben, wonach die Mischung durch Zugabe von 2 M wäßriger Salzsäure auf pH 3 gebracht und zweimal mit Essigsäureethylester extrahiert wurde. Die vereinigten organischen Schichten wurden mit gesättigter Natriumhydrogencarbonatlösung und Kochsalzlösung gewaschen, getrocknet und im Vakuum aufkonzentriert. Der Rückstand wurde mittels präparativer HPLC gereinigt. Es wurden 60 mg der Titelverbindung erhalten.
   LC/MS (Methode LC1): Rt = 1,36 min; m/z = 531,20 [M+H]⁺
(f) (S)-1-((E)-3-{4-[5-(2-Fluorphenoxy)oxazolo[5,4-d]pyrimidin-2-yl]phenyl}acryloyl)-pyrrolidin-2-carbonsäure
   Eine Lösung von 55 mg (S)-1-((E)-3-{4-[5-(2-Fluorphenoxy)oxazolo[5,4-d]pyrimidin-2-yl]phenyl}acryloyl)pyrrolidin-2-carbonsäure-tert.-butylester in 2 ml Dichlormethan und 1 ml Trifluoressigsäure wurde 1 h bei Raumtemperatur gerührt. Dann wurde die Mischung im Vakuum aufkonzentriert und gefriergetrocknet. Es wurden 37 mg der Titelverbindung erhalten.
   LC/MS (Methode LC1): Rt = 1,23 min; m/z = 475,3 [M+H]⁺

### Beispiel 4

### (S)-1-(3-{4-[5-(2-Fluorphenoxy)oxazolo[5,4-d]pyrimidin-2-yl]phenyl}propionyl)pyrrolidin-2-carbonsäure

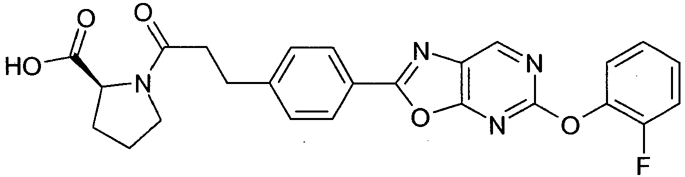

30 mg (S)-1-((E)-3-{4-[5-(2-Fluorphenoxy)oxazolo[5,4-d]pyrimidin-2-yl]phenyl}-acryloyl)pyrrolidin-2-carbonsäure wurden in 2 ml Essigsäureethylester gelöst und in Gegenwart von 30 mg Palladium auf Kohle (10%) 16 h bei 5 bar hydriert. Die Mischung wurde über Celite filtriert und im Vakuum aufkonzentriert. Es wurden 25 mg der Titelverbindung erhalten.
LC/MS (Methode LC1): Rt = 1,11 min; m/z = 477,11 [M+H]⁺

In Analogie zur Herstellung der oben beschriebenen Beispielverbindungen wurden die folgenden Verbindungen der Formel I hergestellt.

| Beispiel | Name | LC/MS | m/z [M+H]⁺ | Rt [min] |
|---|---|---|---|---|
| 5 | (S)-1-(2-{4-[5-(2-Fluorphenoxy)oxazolo[5,4-d]-pyrimidin-2-yl]-2-methylphenoxy}acetyl)pyrrolidin-2-carbonsäure | LC1 | 493,21 | 1,24 |
| 6 | (S)-1-(2-{4-[5-(2-Fluorphenoxy)oxazolo[5,4-d]-pyrimidin-2-yl]phenoxy}acetyl)pyrrolidin-2-carbonsäure | LC1 | 479,15 | 1,20 |
| 7 | 1-(2-{4-[5-(2-Fluorphenoxy)oxazolo[5,4-d]pyrimidin-2-yl]phenoxy}acetyl)piperidin-4-carbonsäure | LC1 | 493,18 | 1,20 |
| 8 | (S)-1-(2-{4-[5-(2-Fluorphenoxy)oxazolo[5,4-d]-pyrimidin-2-yl]phenoxy}propionyl)pyrrolidin-2-carbonsäure | LC1 | 493,16 | 1,23 |
| 9 | (S)-1-(2-{4-[5-(2-Fluorphenoxy)-oxazolo[5,4-d]pyrimidin-2-yl]-2-trifluormethylphenoxy}-acetyl)-pyrrolidin-2-carbonsäure | LC1 | 547,13 | 1,27 |
| 10 | 1-(2-{4-[5-(2-Fluorphenoxy)-oxazolo[5,4-d]pyrimidin-2-yl]-2-trifluormethylphenoxy}-acetyl)-piperidin-4-carbonsäure | LC1 | 561,16 | 1,27 |
| 11 | (S)-1-(2-{4-[5-(3-Chlorphenoxy)-oxazolo[5,4-d]pyrimidin-2-yl]-2,6-dimethylphenoxy}-acetyl)-pyrrolidin-2-carbonsäure | LC5 | 523,15 | 1,29 |
| 12 | 1-(2-{4-[5-(3-Chlorphenoxy)-oxazolo[5,4-d]pyrimidin-2-yl]-2,6-dimethylphenoxy}-acetyl)-piperidin-4-carbonsäure | LC5 | 537,15 | 1,29 |
| 13 | (S)-1-((E)-3-{4-[5-(2-Fluorphenoxy)-oxazolo[5,4-d]pyrimidin-2-yl]-2-methylphenyl}-acryloyl)-pyrrolidin2-carbonsäure | LC5 | 489,15 | 1,25 |
| 14 | (S)-1-(3-{4-[5-(2-Fluorphenoxy)-oxazolo[5,4-d]pyrimidin-2-yl]-2-methylphenyl}-propionyl)-pyrrolidin-2-carbonsäure | LC6 | 491,15 | 4,35 |
| 15 | (S)-1-(2-{2,6-Dimethyl-4-[5-(thiazol-2-ylsulfanyl)-oxazolo[5,4-d]pyrimidin-2-yl]-phenoxy}-acetyl)-pyrrolidin-2-carbonsäure | LC1 | 512,12 | 1,21 |
| 16 | (S)-1-(2-{2-Fluoro-4-[5-(2-fluorphenoxy)-oxazolo[5,4-d]pyrimidin-2-yl]-phenoxy}-acetyl)-pyrrolidin-2-carbonsäure | LC1 | 497,09 | 1,22 |
| 17 | (S)-1-(3-{4-[5-(3-Chlorphenoxy)-oxazolo[5,4-d]pyrimidin-2-yl]-2-methylphenyl}-propionyl)-pyrrolidin-2-carbonsäure | LC1 | 507,17 | 1,29 |
| 18 | (S)-1-(2-{2-Chlor-4-[5-(3-chlorphenoxy)-oxazolo[5,4-d]pyrimidin-2-yl]-phenoxy}-acetyl)-pyrrol id in-2-carbonsäure | LC6 | 529,02 | 4,59 |
| 19 | 1-(2-{2-Chlor-4-[5-(3-chlorphenoxy)-oxazolo[5,4-d]pyrimidin-2-yl]-phenoxy}-acetyl)-piperidin-4-carbonsäure | LC5 | 543,06 | 1,29 |
| 20 | (S)-1-(2-{4-[5-(2-Fluorphenoxy)-oxazolo[5,4-d]pyrimidin-2-yl]-2,6-dimethylphenoxy}-2-methylpropionyl)-pyrrolidin-2-carbonsäure | LC1 | 535,29 | 1,33 |
| 21 | (S)-1-(2-{4-[5-(3-Chlorphenoxy)-oxazolo[5,4-d]pyrimidin-2-yl]-2,6-dimethylphenoxy}-2-methylpropionyl)-pyrrolidin-2-carbonsäure | LC1 | 551,25 | 1,38 |
| 22 | 1-(2-{4-[5-(3-Chlorphenoxy)-oxazolo[5,4-d]pyrimidin-2-yl]-2,6-dimethylphenoxy}-2-methylpropionyl)-piperidin-4-carbonsäure | LC1 | 565,27 | 1,36 |
| 23 | 1-(2-{4-[5-(2-Fluorphenoxy)-oxazolo[5,4-d]pyrimidin-2-yl]-2,6-dimethylphenoxy}-acetyl)-piperidin-4-carbonsäure | LC1 | 521,27 | 1,24 |
| 24 | (S)-1-(2-{3-Fluor-4-[5-(2-fluorphenoxy)-oxazolo[5,4-d]pyrimidin-2-yl]-phenoxy}-acetyl)-pyrrolidin-2-carbonsäure | LC1 | 497,21 | 1,2 |
| 25 | 1-(2-{3-Fluor-4-[5-(2-fluorphenoxy)-oxazolo[5,4-d]pyrimidin-2-yl]-phenoxy}-acetyl)-piperidin-4-carbonsäure | LC1 | 511,21 | 1,2 |
| 26 | 1-{2-[2,6-Dimethyl-4-(5-{2-methylphenoxy}-oxazolo[5,4-d]pyrimidin-2-yl)-phenoxy]-acetyl}-piperidin-4-carbonsäure | LC1 | 517,32 | 1,26 |
| 27 | (S)-1-{2-[2,6-Dimethyl-4-(5-{2-methylphenoxy}-oxazolo[5,4-d]pyrimidin-2-yl)-phenoxy]-acetyl}-pyrrolidin-2-carbonsäure | LC1 | 503,32 | 1,27 |
| 28 | (S)-1-(2-{4-[5-(3-Methoxyphenoxy)-oxazolo[5,4-d]pyrimidin-2-yl]-2,6-dimethylphenoxy}-acetyl)-pyrrolidin-2-carbonsäure | LC5 | 519,24 | 1,11 |
| 29 | 1-(2-{4-[5-(3-Methoxyphenoxy)-oxazolo[5,4-d]pyrimidin-2-yl]-2,6-dimethylphenoxy}-acetyl)-piperidin-4-carbonsäure | LC5 | 533,37 | 1,11 |
| 30 | 1-(2-{4-[5-(2-Fluor-4-methylphenoxy)-oxazolo[5,4-d]pyrimidin-2-yl]-2,6-dimethylphenoxy}-acetyl)-piperidin-4-carbonsäure | LC5 | 535,39 | 1,15 |
| 31 | 1-(2-{4-[5-(2-Fluorphenoxy)-oxazolo[5,4-d]pyrimidin-2-yl]-2,6-dimethylphenoxy}-acetyl)-azetidin-3-carbonsäure | LC1 | 493,34 | 1,21 |
| 32 | 1-(2-{4-[5-(3-Chlorphenoxy)-oxazolo[5,4-d]pyrimidin-2-yl]-2,6-dimethyl phenoxy}-acetyl)-azetid in-3-carbonsäure | LC1 | 509,33 | 1,26 |
| 33 | 1-(2-{4-[5-(2-Fluor-4-methylphenoxy)-oxazolo[5,4-d]pyrimidin-2-yl]-2,6-dimethyl-phenoxy}-acetyl)-azetidin-3-carbonsäure | LC1 | 507,19 | 1,25 |
| 34 | 1-{2-[2,6-Dimethyl-4-(5-{2methylphenoxy}-oxazolo[5,4-d]pyrimidin-2-yl)-phenoxy]-acetyl}-azetidin-3-carbonsäure | LC1 | 489,2 | 1,23 |
| 35 | 1-(2-{4-[5-(3-Methoxyphenoxy)-oxazolo[5,4-d]pyrimidin-2-yl]-2,6-dimethylphenoxy}-acetyl)-azetidin-3-carbonsäure | LC1 | 505,19 | 1,2 |
| 36 | 1-(2-{4-[5-(2-Fluorphenoxy)-oxazolo[5,4-d]pyrimidin-2-yl]-2,6-dimethylphenoxy}-acetyl)-pyrrolidin-3-carbonsäure | LC1 | 507,21 | 1,22 |
| 37 | 1-(2-{4-[5-(3-Chlorphenoxy)-oxazolo[5,4-d]pyrimidin-2-yl]-2,6-dimethyl-phenoxy}-acetyl)-pyrrolidin-3-carbonsäure | LC1 | 523,15 | 1,25 |

### Bestimmung der pharmakologischen Wirkung

### A) GTP-γ-S-Assay mit humanen Edg-1-Rezeptoren

Zur Bestimmung der Edg-1-Rezeptor-Aktivierung durch die erfindungsgemäßen Verbindungen wurde ein GTP-γ-S-Assay (GTP-γ-S = Guanosin-5'-[thio]triphosphat) auf die Bindung an G-Protein gekoppeltem Rezeptor auf Basis des Szintillationsproximitätsassay-Prinzips verwendet, wobei ein Zellmembranpräparat einer CHO-Flp-In-Zelllinie, die den humanen Edg-1-Rezeptor konstitutiv überexprimiert, eingesetzt wurde.

### (a) Erzeugung der Zelllinie

Das Flp-In™-Expressionssystem (Invitrogen, Kat.-Nr. K6010-01) erlaubt die Erzeugung von stabilen Säugetierzelllinien, in die das interessierende Gen durch homologe Rekombination an einem spezifischen Genomort, der als FRT-Ort (FRT = Flp Recombination Target) bezeichnet wird, mit Hilfe einer durch das pOG44-Expressionsplasmid codierten Flp-Rekombinase integriert worden ist. Die Integration des pcDNA5/FRT-Expressionskonstrukts in das Flp-In-Wirtszellliniengenom führt zur Transkription des interessierenden Gens. Die stabil transfizierten Zellen werden hygromycinresistent.

Einen Tag vor der Transfektion wurden 200 000 Flp-In-CHO-Zellen in Ham-F-12-Medium (Invitrogen, Kat.-Nr. 31765) mit 10% fötalem Kälberserum (FCS; Perbio Science, Kat.-Nr. SH30068.03) in einer Platte mit 6 Vertiefungen ausgesät und über Nacht bei 37°C/5% CO₂ inkubiert. Unter Verwendung von FuGENE^{®}-6-Transfektionsreagens (Roche, Kat.-Nr. 11988387001) wurden Zellen mit dem Flp-Rekombinase-Expressionsplasmid pOG44 und einem modifizierten Plasmid, das zusätzlich das edg-1-Gen (Zugangsnummer NM_001400) enthält und als pcDNA5-FRT-TO_nFLAG_DEST-EDG-1 bezeichnet wird, mit einem Verhältnis von 9:1 kotransfiziert. Zum Erhalt des modifizierten pcDNA5-FRT-TO_nFLAG_DEST-Plasmids wurde das Invitrogen-Plasmid pcDNA5/FRT/TO (Invitrogen, Kat.-Nr. V6520-20) durch Insertierung einer Gateway-Kassette mit ein ccdB-Gen und ein Chloramphenicolresistenzgen flankierenden attR-Rekombinationsstellen (Gateway Conversion System, Invitrogen, Kat.-Nr. 11828-029) auf das Gateway^{®}-Kloniersystem (Invitrogen) angepasst. Außerdem wurde vor der 5'-att-Rekombinationsstelle ein FLAG-Tag-Epitop hinzugefügt, um eine rekombinante Expression von Proteinen mit N-terminalem FLAG-Tag zu ermöglichen.

Für die Transfektion einer Vertiefung wurden 1,08 µg pOG44 und 0,12 µg pcDNA5-FRT-TO_nFLAG_DEST-EDG-1 mit 100 µl serumfreiem Ham-F-12-Medium mit 6 µl FuGENE^{®}-6-Transfektionsreagens gemischt. Nach 20 min Inkubation wurde der Transfektionsreagens/DNA-Komplex tropfenweise auf den Zellen verteilt. Die Zellen wurden 24 h bei 37°C inkubiert. Dann wurden die Zellen aus drei Vertiefungen in eine T75-Flasche (Greiner Cellstar^{®}, Kat.-Nr. 658175) mit Ham-F-12-Medium mit 10% FCS, aber ohne Antibiotikum, überführt und noch 24 h inkubiert. 48 h nach der Transfektion wurde das Medium durch Selektionsmedium (Ham F-12 mit 10 % FCS und 300 µg/ml Hygromycin B (Invitrogen, Kat.-Nr. 10687-010)) ersetzt. Das Medium wurde alle 2 bis 3 Tage ausgetauscht, bis eine resistente Population von Zellen herangewachsen war. Zellen wurden mehrmals aufgeteilt und in eine neue Flasche ausgesät, so daß die Zellen nicht mehr als 25% Konfluenz erreichten. Nach 2 Wochen Selektion wurden die Zellen in T175-Flaschen (Greiner Cellstar^{®}, Kat.-Nr. 660175) überführt und für die Batchproduktion kultiviert. Die Zellen wurden durch kurze Behandlung (2 bis 5 min) mit Accutase (PAA, Kat.-Nr. L11-007) aus den Kulturflaschen geerntet, in Selektionsmedium (siehe oben) resuspendiert und 5 min bei 200 x g zentrifugiert. Die Zellen wurden in einer Mischung von 90% FCS und 10% Dimethylsulfoxid resuspendiert und in flüssigem Stickstoff gefroren gelagert.

### (b) Membranpräparat

Aus der obenbeschriebenen CHO-Flp-In-Zelllinie, die den humanen Edg-1-Rezeptor konstitutiv überexprimiert, wurde nach Standardmethoden ein Membranpräparat erhalten. Kurz gesagt, wurden die kryokonservierten Zellen in Kultur genommen und in T175-Zellkulturflaschen (Becton Dickinson, Kat.-Nr. 35 5001) bis zur Konfluenz angezogen. Die Zellkultur wurde durch Waschen mit calciumfreier phosphatgepufferter Kochsalzlösung (PBS; Gibco, Kat.-Nr. 14190) gestoppt, und die Zellen wurden mit einem Gummischaber in 4°C kaltem und calciumfreiem PBS mit einem Proteaseinhibitorcocktail (Complete Protease Inhibitor; Roche, Kat.-Nr. 1697498; 1 Tablette pro 50 ml) geerntet und danach bei 4°C 15 min bei 1100 x g zentrifugiert (Heraeus Minifuge T). Zur Zelllyse wurde das Pellett in 4°C kaltem hypotonischem Puffer aus 5 mM HEPES (Sigma-Aldrich, Kat.-Nr. H-0981), 1 mM EDTA (Dinatriumsalz; Merck, Kat.-Nr. 8418) mit Proteaseinhibitorcocktail (wie oben) resuspendiert, in dem die Zellen noch 15 min auf Eis gelagert wurden. Nach der Lyse wurden die Zellen bei 4°C 10 min bei 400 x g zentrifugiert (Heraeus Minifuge T). Das Pellet wurde in einem Dounce-Homogenisator auseinandergebrochen, mit dem Überstand der vorhergehenden Zentrifugation verdünnt und danach bei 4°C 10 min bei 500 x g zentrifugiert (Heraeus Minifuge T), um Nuklei und noch intakte Zellen von den hauptsächlich im Überstand vorliegenden Membranen abzutrennen. Der Überstand wurde dann in hypotonischem Puffer verdünnt und bei 4°C bei ungefähr 18600 x g 2 h zentrifugiert (Beckmann, Avanti J251). Danach wurde das Membranpellet in einem Lagerpuffer aus 20 mM HEPES; 150 mM NaCl (Merck, Kat.-Nr. 6400), 1 mM EDTA (wie oben) mit Proteaseinhibitorcocktail (wie oben) resuspendiert. Das Membranpräparat wurde aliquotiert und bei -80°C gelagert. Die Proteinkonzentration des Membranpräparats wurde in einer Probe mit Hilfe eines kommerziellen Proteinassays (Bio-Rad, DC Protein Assay, Kat.-Nr. 500-0113, 500-0114, 500-0115) bestimmt.

### (c) GTP-γ-S-Assay

Das in (b) erhaltene Edg-1-Membranpräparat wurde in einem kommerziell erhältlichen Szintillationsproximitätsassay-Kit (SPA-Kit) auf die Bindung am G-Protein-gekoppelten Rezeptor von Amersham Biosciences/GE Healthcare (Code RPNQ0210) eingesetzt, indem die ligandeninduzierte Bindung von ³⁵S-radiomarkiertem GTP-γ-S an die rezeptorhaltige Membran, die an Szintillationsperlen gebunden ist, die Emission von Licht induziert und die Quantifizierung der in-vitro-Wirkung der Edg-1-agonistischen Verbindung erlaubt. Der Assay wurde auf einer Platte mit 96 Vertiefungen weitgehend nach den Anweisungen des Herstellers durchgeführt. Vor dem Beginn der Experimente wurden Szintillationsperlen in einem Rekonstitutionspuffer aus Tris-HCl (pH 7,4) mit 0,1 % (w/v) Natriumazid suspendiert und dann auf Eis mit Assaypuffer (aus 20 mM HEPES, 100 mM NaCl, 1 mM EDTA (wie oben), 1 mM Dithiothreitol (DTT), auf pH 7,4 eingestellt) auf eine Perlenendkonzentration von 30 mg/ml verdünnt.

Die Vertiefungen wurden mit 10 µl des angegebenen Assaypuffers, 10 µl 100 µM Guanosindiphosphat-Lösung (GDP-Lösung) und 10 µl einer Lösung der Testverbindung in Assaypuffer/Dimethylsulfoxid versetzt, was eine Endkonzentration der Testverbindung von 10 µM ergibt. Anstelle der Lösung der Testverbindung wurden für die hohen Kontrollen 10 µl einer Lösung von Sphingosin-1-phosphat (S1P; Sigma, Kat.-Nr. S-9666), was eine S1P-Endkonzentration von 10 µM ergab, und für die niedrigen Kontrollen 10 µl Assaypuffer in die jeweiligen Vertiefungen gegeben. Alle Vertiefungen enthielten äquivalente Mengen von Dimethylsulfoxid. Dann wurden in jede Vertiefung 10 µl einer [³⁵S]GTP-γ-S-Lösung (4 nM) und das in (b) erhaltene Edg-1-Membranpräparat (15 µg Membranprotein in 100 µl Assaypuffer) gegeben. Nach Inkubation der Platten bei Raumtemperatur über einen Zeitraum von 5 min wurden 50 µl der angegebenen Szintillationsperlensuspension (30 mg/ml) zugegeben. Nach einem weiteren Inkubationszeitraum von 45 min bei Raumtemperatur wurden die Platten 10 min bei 500 x g zentrifugiert. Die Quantifizierung der [³⁵S]GTP-γ-S-Bindung und somit der Rezeptoraktivierung wurde mit Hilfe eines Beta-Zählers (MicroBeta, Wallac) über einen Zeitraum von 1 min gemessen. Die Werte wurden durch Subtraktion der jeweiligen niedrigen Kontrolle hintergrundkorrigiert. Alle Messungen wurden dreifach durchgeführt. Die Rezeptoraktivierung durch die Testverbindung wird in % der jeweiligen hohen Kontrolle (10 µM S1P; wird als 100% Aktivierung erachtet) ausgedrückt. Die mit Beispielverbindungen bei 10 µM beobachteten Aktivierungen sind in Tabelle 2 aufgeführt.

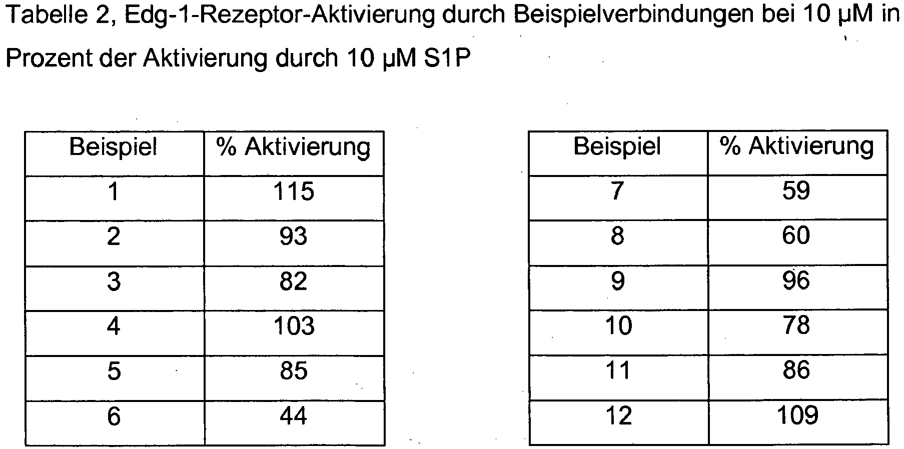

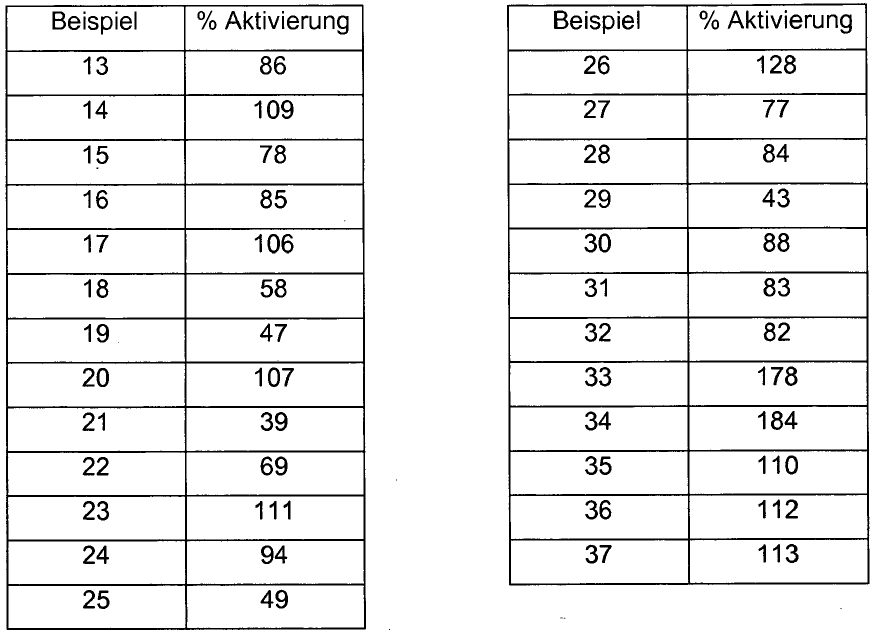

Aus den Messdaten ist ersichtlich, dass die Verbindungen gut zur Wundheilung und insbesondere zur Behandlung von Wundheilungsstörungen von Diabetes Patienten geeignet sind.

## Patentansprüche

1. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes, 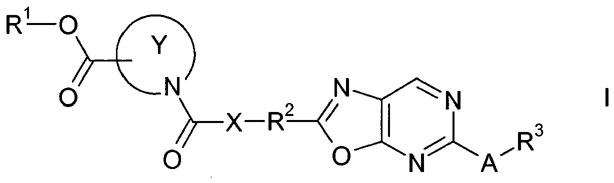 worin
A ausgewählt ist aus NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl und (C₁-C₆)-Alkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxygruppe an die Gruppe R² gebunden ist;
Y für einen 4-gliedrigen bis 7-gliedrigen, gesättigten oder teilweise ungesättigten, monocyclischen oder bicyclischen Heterocyclus steht, der neben dem in Formel I dargestellten Ringstickstoffatom 0,1, 2 oder 3 gleiche oder verschiedene Ringheteroatome, die unter N, O und S ausgewählt sind, enthält, wobei ein oder zwei der zusätzlichen Ringstickstoffatome ein Wasserstoffatom oder einen (C₁-C₄)-Alkylsubstituenten tragen können und eines der Ringschwefelatome eine oder zwei Oxogruppen tragen kann und wobei der Heterocyclus gegebenenfalls an einem oder mehreren Ringkohlenstoffatomen durch gleiche oder verschiedene (C₁-C₄-)-Alkylsubstituenten substituiert ist;
R¹ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R² ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R²¹ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₂-C₆)-Alkenyl, (C₂-C₆)-Alkinyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R²¹ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind;
wobei alle Cycloalkyl- und Cycloalkandiylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, Alkandiyl-, CᵤH₂ᵤ-, CᵥH₂ᵥ-, C_{w}H_{2w}-, C_{z}H_{2z}-, Alkenyl-, Alkendiyl-, Alkinyl- und Alkindiylgruppen unabhängig voneinander und unabhängig von anderen Substituenten gegebenenfalls durch einen oder mehrere Fluorsubstituenten substituiert sind.

2. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach Anspruch 1, worin A aus O und S ausgewählt ist.

3. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 und 2, worin X aus (C₁-C₆)-Alkandiyl, (C₂-C₆)-Alkendiyl und (C₁-C₆)-Alkandiyloxy ausgewählt ist.

4. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 3, worin Y für einen 4-gliedrigen bis 7-gliedrigen, gesättigten monocyclischen oder bicyclischen Heterocyclus, der neben dem in Formel I dargestellten Ringstickstoffatom 0 oder 1 Ringheteroatom enthält, das unter N, O und S ausgewählt ist, steht, wobei ein zusätzliches Ringstickstoffatom ein Wasserstoffatom oder einen (C₁-C₄)-Alkylsubstituenten tragen kann und ein Ringschwefelatom eine oder zwei Oxogruppen tragen kann und wobei der Heterocyclus gegebenenfalls an einem oder mehreren Ringkohlenstoffatomen durch gleiche oder verschiedene (C₁-C₄)-Alkylsubstituenten substituiert ist.

5. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 4, worin
R² ausgewählt ist aus Phenylen und Pyridindiyl, wobei das Phenylen und das Pyridindiyl gegebenenfalls an einem oder mehreren Ringkohlenstoffatomen durch gleiche oder verschiedene Substituenten R²² substituiert sind.

6. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 5, worin
R³ ausgewählt ist aus (C₁-C₆)-Alkyl, (C₃-C₇)-Cycloalkyl-CᵤH₂ᵤ- und Het-CᵥH₂ᵥ-, worin u und v unter 1 und 2 ausgewählt sind, oder R³ für einen Rest eines gesättigten oder ungesättigten, 3-gliedrigen bis 1 0-gliedrigen, monocyclischen oder bicyclischen Rings, der 0, 1 oder 2 gleiche oder verschiedene Ringheteroatome enthält, die unter N, O und S ausgewählt sind, steht, wobei ein oder zwei der Ringstickstoffatome ein Wasserstoffatom oder einen (C₁-C₄)-Alkylsubstituenten tragen können und ein Ringschwefelatom eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
Het für einen Rest eines gesättigten 4-gliedrigen bis 6-gliedrigen, monocyclischen Heterocyclus, der 1 Ringheteroatom enthält, das aus N, O und S ausgewählt ist und über ein Ringkohlenstoffatom gebunden ist, steht, wobei der Rest eines Heterocyclus gegebenenfalls durch einen oder mehrere gleiche oder verschiedene Substituenten, die aus Fluor und (C₁-C₄)-Alkyl ausgewählt sind, substituiert ist.

7. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 6, worin
A aus O und S ausgewählt ist;
X ausgewählt ist aus (C₁-C₆)-Alkandiyl, (C₂-C₆)-Alkendiyl und (C₁-C₆)-Alkandiyloxy;
Y für einen 4-gliedrigen bis 7-gliedrigen, gesättigten, monocyclischen oder bicyclischen Heterocyclus steht, der neben dem in Formel I dargestellten Ringstickstoffatom 0 oder 1 Ringheteroatom, das aus N, O und S ausgewählt ist, enthält, wobei ein zusätzliches Ringstickstoffatom ein Wasserstoffatom oder einen (C₁-C₄)-Alkylsubstituenten tragen kann und ein Ringschwefelatom eine oder zwei Oxogruppen tragen kann und wobei der Heterocyclus gegebenenfalls an einem oder mehreren Ringkohlenstoffatomen durch gleiche oder verschiedene (C₁-C₄)-Alkylsubstituenten substituiert ist;
R² ausgewählt ist aus Phenylen und Pyridindiyl, worin das Phenylen und das Pyridindiyl gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind.

8. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 7, worin
A für O steht;
X aus (C₁-C₆)-Alkandiyl und (C₁-C₆)-Alkandiyloxy ausgewählt ist;
Y für einen 4-gliedrigen bis 6-gliedrigen, gesättigten, monocyclischen Heterocyclus steht, der keine weiteren Ringheteroatome neben dem in Formel I dargestellten Ringstickstoffatom enthält, wobei der Ring gegebenenfalls an einem oder mehreren Ringkohlenstoffatomen durch gleiche oder verschiedene (C₁-C₄)-Alkylsubstituenten substituiert ist;
R¹ aus Wasserstoff und (C₁-C₄)-Alkyl ausgewählt ist;
R² für Phenylen steht, das gegebenenfalls an einem oder mehreren Ringkohlenstoffatomen durch gleiche oder verschiedene Substituenten R²² substituiert ist;
R³ ausgewählt ist aus (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 7-gliedrigen, monocyclischen oder bicyclischen Rings, der 0, 1 oder 2 gleiche oder verschiedene Ringheteroatome, die unter N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
Het für einen Rest eines gesättigten, 4-gliedrigen bis 6-gliedrigen, monocyclischen Heterocyclus, der 1 Ringheteroatom, das aus O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind; wobei alle Alkyl-, Alkandiyl-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

9. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 8, worin
A für O steht;
X aus (C₁-C₆)-Alkandiyl und (C₁-C₆)-Alkandiyloxy ausgewählt ist;
Y für einen 4-gliedrigen bis 6-gliedrigen, gesättigten, monocyclischen Heterocyclus steht, der keine weiteren Ringheteroatome neben dem in Formel I dargestellten Ringstickstoffatom enthält, wobei der Ring gegebenenfalls an einem oder mehreren Ringkohlenstoffatomen durch gleiche oder verschiedene (C₁-C₄)-Alkylsubstituenten substituiert ist;
R¹ aus Wasserstoff und (C₁-C₄)-Alkyl ausgewählt ist;
R² für Phenylen steht, das gegebenenfalls an einem oder mehreren Ringkohlenstoffatomen durch gleiche oder verschiedene Substituenten R²² substituiert ist;
R³ für einen Rest eines gesättigten oder ungesättigten, 3-gliedrigen bis 7-gliedrigen, monocyclischen Rings, der 0 oder 1 Ringheteroatom, das unter N, O und S ausgewählt ist, enthält, steht, wobei ein Ringstickstoffatom ein Wasserstoffatom oder einen (C₁-C₄)-Alkylsubstituenten tragen kann und ein Ringschwefelatom eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
wobei alle Cycloalkylgruppen unabhängig voneinander und unabhängig von anderen Substituenten gegebenenfalls durch einen oder mehrere gleiche ode verschiedene Substituenten substituiert sind, die unter Fluor und (C₁-C₄)-Alkyl ausgewählt sind; wobei alle Alkyl-, Alkandiyl-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

10. Verbindung der Formel I oder physiologisch akzeptables Salz davon oder physiologisch akzeptables Solvat einer derartigen Verbindung oder eines derartigen Salzes nach einem oder mehreren der Ansprüche 1 bis 9, ausgewählt aus
(S)-1-(2-{4-[5-(2-Fluorphenoxy)oxazolo[5,4-d]pyrimidin-2-yl]-2,6-dimethylphenoxy}-acetyl)pyrrolidin-2-carbonsäure,
(S)-1-(2-{4-[5-(2-Fluorphenoxy)oxazolo[5,4-d]pyrimidin-2-yl]-2,6-dimethylphenoxy}-propionyl)pyrrolidin-2-carbonsäure,
(S)-1-((E)-3-{4-[5-(2-Fluorphenoxy)oxazolo[5,4-d]pyrimidin-2-yl]phenyl}acryloyl)-pyrrolidin-2-carbonsäure,
(S)-1-(3-{4-[5-(2-Fluorphenoxy)oxazolo[5,4-d]pyrimidin-2-yl]phenyl}propionyl)pyrrolidin-2-carbonsäure,
(S)-1-(2-{4-[5-(2-Fluorphenoxy)oxazolo[5,4-d]pyrimidin-2-yl]-2-methylphenoxy}-acetyl)pyrrolidin-2-carbonsäure,
(S)-1-(2-{4-[5-(2-Fluorphenoxy)oxazolo[5,4-d]pyrimidin-2-yl]phenoxy}acetyl)pyrrolidin-2-carbonsäure,
1-(2-{4-[5-(2-Fluorphenoxy)oxazolo[5,4-d]pyrimidin-2-yl]phenoxy}acetyl)piperidin-4-carbonsäure und
(S)-1-(2-{4-[5-(2-Fluorphenoxy)oxazolo[5,4-d]pyrimidin-2-yl]phenoxy}propionyl)-pyrrolidin-2-carbonsäure.

11. Verfahren zur Herstellung einer Verbindung der Formel I nach einem der Ansprüche 1 bis 10, bei dem man eine Verbindung der Formel II mit einer Verbindung der Formel III umsetzt, 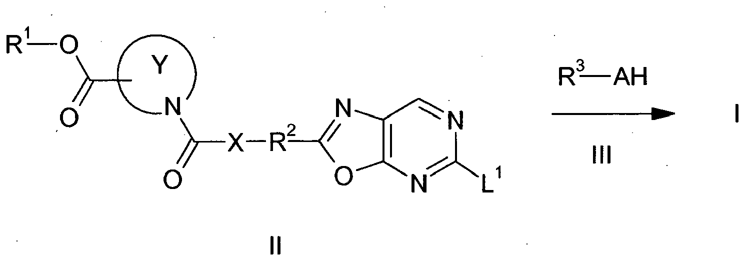 wobei die Gruppen A, X, Y, R¹, R² und R³ in den Verbindungen der Formeln II und III wie in den Verbindungen der Formel I definiert sind und außerdem funktionelle Gruppen in geschützter Form oder in Form einer Vorläufergruppe vorliegen können und L¹ für ein Halogenatom oder eine Gruppe der Formel -S(O)-Alk oder -S(O)₂-Alk steht, wobei Alk für (C₁-C₄)-Alkyl steht.

12. Pharmazeutische Zusammensetzung, enthaltend mindestens eine Verbindung der Formel I nach einem der Ansprüche 1 bis 10 oder physiologisch akzeptables Salz davon oder physiologisch akzeptables Solvat einer derartigen Verbindung oder eines derartigen Salzes und einen pharmazeutisch akzeptablen Träger.

13. Verbindung der Formel I nach einem der Ansprüche 1 bis 10 oder physiologisch akzeptables Salz davon oder physiologisch akzeptables Solvat einer derartigen Verbindung oder eines derartigen Salzes zur Verwendung als Arzneimittel.

14. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Behandlung von Wundheilungsstörungen.

15. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Wundheilung.

16. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Wundheilung bei Diabetikern.

17. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Behandlung des Diabetischen Fußsyndroms.

## Claims

1. A compound of the formula I, in any of its stereoisomeric forms, or a mixture of stereoisomeric forms in any ratio, or a physiologically acceptable salt thereof, or a physiologically acceptable solvate of any of them, 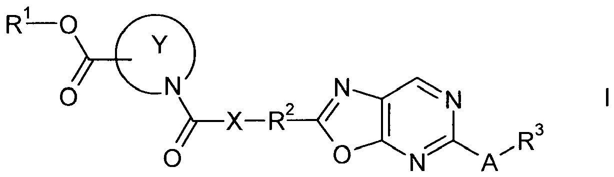 wherein
A is chosen from NH, O and S;
X is chosen from (C₁-C₆) -alkanediyl, (C₂-C₆)-alkenediyl, (C₂-C₆) -alkynediyl, (C₃-C₇) -cycloalkanediyl and (C₁-C₆) - alkanediyl-oxy, which all are optionally substituted by one or more identical or different substituents chosen from fluorine and hydroxy, wherein the oxygen atom of the (C₁-C₆)-alkanediyl-oxy group is bonded to the group R²;
Y is 4-membered to 7-membered, saturated or partially unsaturated, monocyclic or bicyclic heterocycle which, in addition to the ring nitrogen atom depicted in formula I, comprises 0, 1, 2 or 3 identical or different ring heteroatoms chosen from N, O and S, wherein one or two of the additional ring nitrogen atoms can carry a hydrogen atom or a (C₁-C₄)-alkyl substituent and one of the ring sulfur atoms can carry one or two oxo groups, and wherein the heterocycle is optionally substituted on one or more ring carbon atoms by identical or different (C₁-C₄)-alkyl substituents;
R¹ is chosen from hydrogen, (C₁-C₄)-alkyl and (C₃-C₇)-cycloalkyl-C_{z}H_{2z}-, wherein z is chosen from 0, 1 and 2;
R² is chosen from phenylene and a divalent residue of an aromatic, 5-membered to 6-membered monocyclic heterocycle which comprises 1, 2 or 3 identical or different ring heteroatoms chosen from N, O and S, wherein one of the ring nitrogen atoms can carry a hydrogen atom or a substituent R²¹, and wherein the phenylene and divalent residue of an aromatic heterocycle are optionally substituted on one or more ring carbon atoms by identical or different substituents R²²;
R³ is chosen from (C₁-C₆)-alkyl, (C₂-C₆)-alkenyl, (C₂-C₆)-alkynyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹;
R²¹ is chosen from (C₁-C₄)-alkyl, (C₃-C-₇)-cycloalkyl-C_{w}H_{2w}- and oxy, wherein w is chosen from 0, 1 and 2;
R²² is chosen from halogen, hydroxy, (C₁-C₄)-alkyl-, (C₁-C₄)-alkyloxy, (C₁-C₄)-alkyl-S(O)ₘ-, amino, nitro, cyano, hydroxycarbonyl, (C₁-C₉)-alkyloxycarbonyl, aminocarbonyl and aminosulfonyl;
R³¹ is chosen from halogen, (C₁-C₄)-alkyl, (C₃-C₇) - cycloalkyl, hydroxy, (C₁-C₄)-alkyloxy, oxo, (C₁-C₄)-alkyl-S (O)ₘ-, amino, (C₁-C₄) -alkylamino, di ((C₁-C₄)-alkyl) amino, (C₁-C₄)-alkylcarbonylamino, (C₁-C₄)-alkylsulfonylamino, nitro, cyano, (C₁-C₄)-alkylcarbonyl, aminosulfonyl, (C₁-C₄)-alkylaminosulfonyl and di((C₁-C₄)-alkyl)aminosulfonyl;
Het is a residue of a saturated, 4-membered to 7-membered, monocyclic heterocycle which comprises 1 or 2 identical or different ring heteroatoms chosen from N, O and S and which is bonded via a ring carbon atom, wherein the residue of a heterocycle is optionally substituted by one or more identical or different substituents chosen from fluorine and (C₁-C₄)-alkyl;
m is chosen from 0, 1 and 2, wherein all numbers m are independent of each other;
wherein all cycloalkyl and cycloalkanediyl groups, independently of each other and independently of any other substituents, are optionally substituted by one or more identical or different substituents chosen from fluorine and (C₁-C₄)-alkyl;
wherein all alkyl, alkanediyl, CᵤH₂ᵤ, CᵥH₂ᵥ, C_{w}H_{2w}, C_{z}H_{2z}, alkenyl, alkenediyl, alkynyl and alkynediyl groups, independently of each other and independently of any other substituents, are optionally substituted by one or more fluorine substituents.

2. A compound of the formula I, in any of its stereoisomeric forms, or a mixture of stereoisomeric forms in any ratio, or a physiologically acceptable salt thereof, or a physiologically acceptable solvate of any of them, as claimed in claim 1, wherein A is chosen from O and S.

3. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 and 2, wherein X is chosen from (C₁-C₆) -alkanediyl, (C₂-C₆)-alkenediyl and (C₁-C₆)-alkanediyl-oxy.

4. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 3, wherein Y is a 4-membered to 7-membered, saturated, monocyclic or bicyclic heterocycle which, in addition to the ring nitrogen atom depicted in formula I, comprises 0 or 1 ring heteroatoms chosen from N, O and S, wherein an additional ring nitrogen atom can carry a hydrogen atom or a (C₁-C₄)-alkyl substituent and a ring sulfur atom can carry one or two oxo groups, and wherein the heterocycle is optionally substituted on one or more ring carbon atoms by identical or different (C₁-C₄)-alkyl substituents.

5. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 4, wherein
R² is chosen from phenylene and pyridinediyl, wherein the phenylene and the pyridinediyl are optionally substituted on one or more ring carbon atoms by identical or different substituents R²².

6. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 5, wherein
R³ is chosen from (C₁-C₆)-alkyl, (C₃-C₇)-cycloalkyl-CᵤH₂ᵤ-and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹;
Het is a residue of a saturated, 4-membered to 6-membered, monocyclic heterocycle which comprises 1 ring heteroatom chosen from N, O and S and which is bonded via a ring carbon atom, wherein the residue of a heterocycle is optionally substituted by one or more identical or different substituents chosen from fluorine and (C₁-C₄)-alkyl.

7. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 6, wherein
A is chosen from O and S;
X is chosen from (C₁-C₆) -alkanediyl, (C₂-C₆)-alkenediyl and (C₁-C₆)-alkanediyl-oxy;
Y is a 4-membered to 7-membered, saturated, monocyclic or bicyclic heterocycle which, in addition to the ring nitrogen atom depicted in formula I, comprises 0 or 1 ring heteroatoms chosen from N, O and S, wherein an additional ring nitrogen atom can carry a hydrogen atom or a (C₁-C₄)-alkyl substituent and a ring sulfur atom can carry one or two oxo groups, and wherein the heterocycle is optionally substituted on one or more ring carbon atoms by identical or different (C₁-C₄)-alkyl substituents;
R² is chosen from phenylene and pyridinediyl, wherein the phenylene and the pyridinediyl are optionally substituted on one or more ring carbon atoms by identical or different substituents R²²;
R³ is chosen from (C₁-C₆)-alkyl, (C₃-C₇)-cycloalkyl-CᵤH₂ᵤ-and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹;
Het is a residue of a saturated, 4-membered to 6-membered, monocyclic heterocycle which comprises 1 ring heteroatom chosen from N, O and S and which is bonded via a ring carbon atom, wherein the residue of a heterocycle is optionally substituted by one or more identical or different substituents chosen from fluorine and (C₁-C₄)-alkyl.

8. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 7, wherein
A is O;
X is chosen from (C₁-C₆)-alkanediyl and (C₁-C₆)-alkanediyl-oxy;
Y is a 4-membered to 6-membered, saturated, monocyclic heterocycle which does not comprise further ring heteroatoms in addition to the ring nitrogen atom depicted in formula I, wherein the ring is optionally substituted on one or more ring carbon atoms by identical or different (C₁-C₄)-alkyl substituents;
R¹ is chosen from hydrogen and (C₁-C₄)-alkyl;
R² is phenylene which is optionally substituted on one or more ring carbon atoms by identical or different substituents R²²;
R³ is chosen from (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 7-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹; R²² is chosen from halogen, hydroxy, (C₁-C₄)-alkyl- and (C₁-C₄)-alkyloxy;
R³¹ is chosen from halogen, (C₁-C₄)-alkyl, (C₃-C₇)-cycloalkyl, hydroxy and (C₁-C₄)-alkyloxy;
Het is a residue of a saturated, 4-membered to 6-membered, monocyclic heterocycle which comprises 1 ring heteroatom chosen from O and S and which is bonded via a ring carbon atom, wherein the residue of a heterocycle is optionally substituted by one or more identical or different substituents chosen from fluorine and (C₁-C₄)-alkyl;
wherein all cycloalkyl groups, independently of each other and independently of any other substituents, are optionally substituted by one or more identical or different substituents chosen from fluorine and (C₁-C₄)-alkyl;
wherein all alkyl, alkanediyl, CᵤH₂ᵤ and CᵥH₂ᵥ groups, independently of each other and independently of any other substituents, are optionally substituted by one or more fluorine substituents.

9. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 8, wherein
A is O;
X is chosen from (C₁-C₆)-alkanediyl and (C₁-C₆)-alkanediyl-oxy;
Y is a 4-membered to 6-membered, saturated, monocyclic heterocycle which does not comprise further ring heteroatoms in addition to the ring nitrogen atom depicted in formula I, wherein the ring is optionally substituted on one or more ring carbon atoms by identical or different (C₁-C₄)-alkyl substituents;
R¹ is chosen from hydrogen and (C₁-C₄)-alkyl;
R² is phenylene which is optionally substituted on one or more ring carbon atoms by identical or different substituents R²²;
R³ is a residue of a saturated or unsaturated, 3-membered to 7-membered, monocyclic ring which comprises O or 1 ring heteroatom chosen from N, O and S, wherein a ring nitrogen atom can carry a hydrogen atom or a (C₁-C₄)-alkyl substituent and a ring sulfur atom can carry one or two oxo groups, and wherein the residue of a ring is optionally substituted on one or more ring carbon atoms by identical or different substituents R³¹; R²² is chosen from halogen, hydroxy, (C₁-C₄)-alkyl- and (C₁-C₄)-alkyloxy;
R³¹ is chosen from halogen, (C₁-C₄) -alkyl, (C₃-C₇)-cycloalkyl, hydroxy and (C₁-C₄)-alkyloxy;
wherein all cycloalkyl groups, independently of each other and independently of any other substituents, are optionally substituted by one or more identical or different substituents chosen from fluorine and (C₁-C₄)-alkyl;
wherein all alkyl, alkanediyl, CᵤH₂ᵤ and CᵥH₂ᵥ groups, independently of each other and independently of any other substituents, are optionally substituted by one or more fluorine substituents.

10. A compound of the formula I, or a physiologically acceptable salt thereof, or a physiologically acceptable solvate of any of them, as claimed in one or more of claims 1 to 9, chosen from
(S)-1-(2-{4-[5-(2-fluoro-phenoxy)-oxazolo[5,4-d]pyrimidin-2-yl]-2,6-dimethyl-phenoxy}-acetyl)-pyrrolidine-2-carboxylic acid,
(S)-1-(2-{4-[5-(2-fluoro-phenoxy)-oxazolo[5,4-d]pyrimidin-2-yl]-2,6-dimethyl-phenoxy}-propionyl)-pyrrolidine-2-carboxylic acid,
(S)-1-((E)-3-{4-[5-(2-fluoro-phenoxy)-oxazolo[5,4-d]pyrimidin-2-yl]-phenyl}-acryloyl)-pyrrolidine-2-carboxylic acid,
(S)-1-(3-{4-[5-(2-fluoro-phenoxy)-oxazolo[5,4-d]pyrimidin-2-yl]-phenyl}-propionyl)-pyrrolidine-2-carboxylic acid,
(S)-1-(2-{4-[5-(2-fluoro-phenoxy)-oxazolo[5,4-d]pyrimidin-2-yl]-2-methyl-phenoxy}-acetyl)-pyrrolidine-2-carboxylic acid,
(S)-1-(2-{4-[5-(2-fluoro-phenoxy)-oxazolo[5,4-d]pyrimidin-2-yl]-phenoxy}-acetyl)-pyrrolidine-2-carboxylic acid,
1-(2-{4-[5-(2-fluoro-phenoxy)-oxazolo[5,4-d]pyrimidin-2-yl]-phenoxy}-acetyl)-piperidine-4-carboxylic acid, and
(S)-1-(2-{4-[5-(2-fluoro-phenoxy)-oxazolo[5,4-d]pyrimidin-2-yl]-phenoxy}-propionyl)-pyrrolidine-2-carboxylic acid.

11. A process for the preparation of a compound of the formula I as claimed in any of claims 1 to 10, comprising reacting a compound of the formula II with a compound of the formula III, 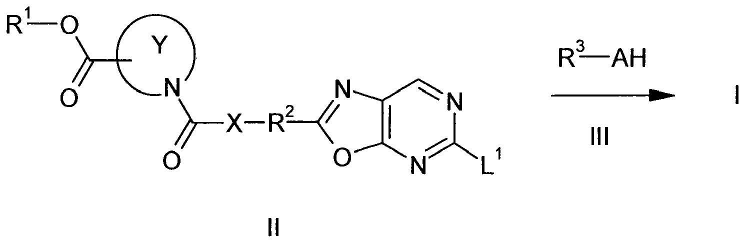 wherein the groups A, X, Y, R¹, R² and R³ in the compounds of the formulae II and III are defined as in the compounds of the formula I and additionally functional groups can be present in protected form or in the form of a precursor group, and L¹ is a halogen atom or a group of the formula -S(O)-Alk or -S(O)₂-Alk wherein Alk is a (C₁-C₄)-alkyl.

12. A pharmaceutical composition, comprising at least one compound of the formula I as claimed in any of claims 1 to 10 or a physiologically acceptable salt thereof, or a physiologically acceptable solvate of any of them, and a pharmaceutically acceptable carrier.

13. A compound of the formula I as claimed in any of claims 1 to 10 or a physiologically acceptable salt thereof, or a physiologically acceptable solvate of any of them, for use as a pharmaceutical.

14. A compound of the formula I as claimed in any of claims 1 to 10 or a physiologically acceptable salt thereof, or a physiologically acceptable solvate of any of them, for the treatment of wound healing disorders.

15. A compound of the formula I as claimed in any of claims 1 to 10 or a physiologically acceptable salt thereof, or a physiologically acceptable solvate of any of them, for wound healing.

16. A compound of the formula I as claimed in any of claims 1 to 10 or a physiologically acceptable salt thereof, or a physiologically acceptable solvate of any of them, for wound healing in diabetics.

17. A compound of the formula I as claimed in any of claims 1 to 10 or a physiologically acceptable salt thereof, or a physiologically acceptable solvate of any of them, for the treatment of diabetic foot syndrome.

## Revendications

1. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel 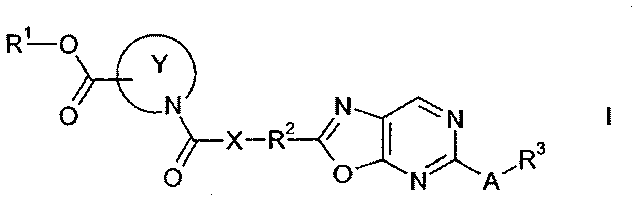 dans laquelle
A est choisi parmi NH, O et S ;
X est choisi parmi alcanediyle en (C₁-C₆), alcènediyle en (C₂-C₆), alcynediyle en (C₂-C₆), cycloalcanediyle en (C₃-C₇) et alcanediyloxy en (C₁-C₆), qui sont tous éventuellement substitués par un ou plusieurs substituants identiques ou différents, choisis parmi fluor et hydroxy, l'atome d'oxygène du groupe alcanediyloxy en (C₁-C₆) étant relié au groupe R² ;
Y représente un hétérocycle de 4 éléments à 7 éléments, saturé ou partiellement insaturé, monocyclique ou bicyclique, qui contient en plus de l'atome d'azote de cycle représenté sur la formule I 0, 1, 2 ou 3 hétéroatomes de cycle identiques ou différents choisis parmi N, O et S, un ou deux des atomes d'azote de cycle supplémentaires pouvant porter un atome d'hydrogène ou un substituant alkyle en (C₁-C₄) et un des atomes de soufre de cycle pouvant porter un ou deux groupes oxo et l'hétérocycle étant éventuellement substitué sur un ou plusieurs atomes de carbone de cycle par des substituants alkyle en (C₁-C₄) identiques ou différents ;
R¹ est choisi parmi hydrogène, alkyle en (C₁-C₄) et cycloalkyle en (C₃-C₇)-C_{z}H_{2z}-, z étant choisi parmi 0, 1 et 2 ;
R² est choisi parmi phénylène et un radical bivalent d'un hétérocycle aromatique, de 5 éléments à 6 éléments, monocyclique, qui contient 1, 2 ou 3 hétéroatomes de cycle identiques ou différents choisis parmi N, O et S, un des atomes d'azote de cycle pouvant porter un atome d'hydrogène ou un substituant R²¹ et le phénylène et le radical bivalent d'un hétérocycle aromatique étant éventuellement substitués sur un ou plusieurs atomes de carbone de cycle par des substituants R²² identiques ou différents ;
R³ est choisi parmi alkyle en (C₁-C₆), alcényle en (C₂-C₆), alcynyle en (C₂-C₆), cycloalkyle en (C₃-C₇)-CᵤH₂ᵤ- et Het-CᵥH₂ᵥ-, u et v étant choisis parmi 1 et 2, ou R³ représente un radical d'un cycle saturé ou insaturé, de 3 éléments à 10 éléments, monocyclique ou bicyclique, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄) et un ou deux des atomes de soufre de cycle pouvant porter un ou deux groupes oxo et le radical d'un cycle étant éventuellement substitué sur un ou plusieurs atomes de carbone de cycle par des substituants R³¹ identiques ou différents ;
R²¹ est choisi parmi alkyle en (C₁-C₄), cycloalkyle en (C₃-C₇)-C_{w}H_{2w}- et oxy, w étant choisi parmi 0, 1 et 2 ;
R²² est choisi parmi halogène, hydroxy, alkyle en (C₁-C₄), alkyloxy en (C₁-C₄), alkyl-S(O)ₘ en (C₁-C₄), amino, nitro, cyano, hydroxycarbonyle, alkyloxycarbonyle en (C₁-C₄), aminocarbonyle et aminosulfonyle ;
R³¹ est choisi parmi halogène, alkyle en (C₁-C₄), cycloalkyle en (C₃-C₇), hydroxy, alkyloxy en (C₁-C₄), oxo, alkyl-S(O)ₘ en (C₁-C₄), amino, alkylamino en (C₁-C₄), di (alkyle en (C₁-C₄))-amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) et di (alkyle en (C₁-C₄))aminosulfonyle ;
Het représente un radical d'un hétérocycle saturé, de 4 éléments à 7 éléments, monocyclique, qui contient 1 ou 2 hétéroatomes de cycle identiques ou différents choisis parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
m est choisi parmi 0, 1 et 2, tous les nombres m étant indépendants les uns des autres ;
tous les groupes cycloalkyle et cycloalcanediyle étant éventuellement substitués indépendamment les uns des autres et indépendamment des autres substituants par un ou plusieurs substituants identiques ou différents choisis parmi fluor et alkyle en (C₁-C₄) ;
tous les groupes alkyle, alcanediyle, CᵤH₂ᵤ, CᵥH₂ᵥ, C_{w}H_{2w}, C_{z}H_{2z}, alcényle, alcènediyle, alcynyle et alcynediyle étant éventuellement substitués indépendamment les uns des autres et indépendamment des autres substituants par un ou plusieurs substituants fluor.

2. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon la revendication 1, dans lequel A est choisi parmi O et S.

3. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 et 2, dans lequel X est choisi parmi alcanediyle en (C₁-C₆), alcènediyle en (C₂-C₆) et alcanediyloxy en (C₁-C₆).

4. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 3, dans lequel Y représente un hétérocycle de 4 éléments à 7 éléments, saturé, monocyclique ou bicyclique, qui contient en plus de l'atome d'azote de cycle représenté sur la formule I 0 ou 1 hétéroatome de cycle choisi parmi N, O et S, un atome d'azote de cycle supplémentaire pouvant porter un atome d'hydrogène ou un substituant alkyle en (C₁-C₄) et un atome de soufre de cycle pouvant porter un ou deux groupes oxo et l'hétérocycle étant éventuellement substitué sur un ou plusieurs atomes de carbone de cycle par des substituants alkyle en (C₁-C₄) identiques ou différents.

5. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 4, dans lequel
R² est choisi parmi phénylène et pyridinediyle, le phénylène et le pyridinediyle étant éventuellement substitués sur un ou plusieurs atomes de carbone de cycle par des substituants R²² identiques ou différents.

6. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 5, dans lequel
R³ est choisi parmi alkyle en (C₁-C₆), cycloalkyle en (C₃-C₇)-CᵤH₂ᵤ- et Het-CᵥH₂ᵥ-, u et v étant choisis parmi 1 et 2, ou R³ représente un radical d'un cycle saturé ou insaturé, de 3 éléments à 10 éléments, monocyclique ou bicyclique, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄) et un atome de soufre de cycle pouvant porter un ou deux groupes oxo et le radical d'un cycle étant éventuellement substitué sur un ou plusieurs atomes de carbone de cycle par des substituants R³¹ identiques ou différents ;
Het représente un radical d'un hétérocycle saturé, de 4 éléments à 6 éléments, monocyclique, qui contient un hétéroatome de cycle choisi parmi N, O et S, et est relié par un atome de carbone de cycle, le radical d'un hétérocycle étant éventuellement substitué par un ou plusieurs substituants identiques ou différents choisis parmi fluor et alkyle en (C₁-C₄).

7. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 6, dans lequel
A est choisi parmi O et S ;
X est choisi parmi alcanediyle en (C₁-C₆), alcènediyle en (C₂-C₆) et alcanediyloxy en (C₁-C₆) ;
Y représente un hétérocycle de 4 éléments à 7 éléments, saturé, monocyclique ou bicyclique, qui contient en plus de l'atome d'azote de cycle représenté sur la formule I 0 ou 1 hétéroatome de cycle choisi parmi N, O et S, un atome d'azote de cycle supplémentaire pouvant porter un atome d'hydrogène ou un substituant alkyle en (C₁-C₄) et un atome de soufre de cycle pouvant porter un ou deux groupes oxo et l'hétérocycle étant éventuellement substitué sur un ou plusieurs atomes de carbone de cycle par des substituants alkyle en (C₁-C₄) identiques ou différents ;
R² est choisi parmi phénylène et pyridinediyle, le phénylène et pyridinediyle étant éventuellement substitués sur un ou plusieurs atomes de carbone de cycle par des substituants R²² identiques ou différents ;
R³ est choisi parmi alkyle en (C₁-C₆), cycloalkyle en (C₃-C₇)-CᵤH₂ᵤ- et Het-CᵥH₂ᵥ-, u et v étant choisis parmi 1 et 2, ou R³ représente un radical d'un cycle saturé ou insaturé, de 3 éléments à 10 éléments, monocyclique ou bicyclique, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄) et un des atomes de soufre de cycle pouvant porter un ou deux groupes oxo et le radical d'un cycle étant éventuellement substitué sur un ou plusieurs atomes de carbone de cycle par des substituants R³¹ identiques ou différents ;
Het représente un radical d'un hétérocycle saturé, de 4 éléments à 6 éléments, monocyclique, qui contient 1 hétéroatome de cycle choisi parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄).

8. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 7, dans lequel
A représente O ;
X est choisi parmi alcanediyle en (C₁-C₆) et alcanediyloxy en (C₁-C₆) ; 1
Y représente un hétérocycle de 4 éléments à 6 éléments, saturé, monocyclique, qui ne contient pas d'hétéroatomes de cycle supplémentaires en plus de l'atome d'azote de cycle représenté sur la formule I, le cycle étant éventuellement substitué sur un ou plusieurs atomes de carbone de cycle par des substituants alkyle en (C₁-C₄) identiques ou différents ;
R¹ est choisi parmi hydrogène et alkyle en (C₁-C₄) ;
R² représente phénylène, qui est éventuellement substitué sur un ou plusieurs atomes de carbone de cycle par des substituants R²² identiques ou différents ;
R³ est choisi parmi cycloalkyle en (C₃-C₇)-CᵤH₂ᵤ- et Het-CᵥH₂ᵥ-, u et v étant choisis parmi 1 et 2, ou R³ représente un radical d'un cycle saturé ou insaturé, de 3 éléments à 7 éléments, monocyclique ou bicyclique, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄) et un des atomes de soufre de cycle pouvant porter un ou deux groupes oxo et le radical d'un cycle étant éventuellement substitué sur un ou plusieurs atomes de carbone de cycle par des substituants R³¹ identiques ou différents ;
R²² est choisi parmi halogène, hydroxy, alkyle en (C₁-C₄) et alkyloxy en (C₁-C₄)
R³¹ est choisi parmi halogène, alkyle en (C₁-C₄) , cycloalkyle en (C₃-C₇), hydroxy et alkyloxy en (C₁-C₄) Het représente un radical d'un hétérocycle saturé, de 4 éléments à 6 éléments, monocyclique, qui contient 1 hétéroatome de cycle choisi parmi O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
tous les groupes cycloalkyle étant éventuellement substitués indépendamment les uns des autres et indépendamment des autres substituants par un ou plusieurs substituants identiques ou différents choisis parmi fluor et alkyle en (C₁-C₄) ;
tous les groupes alkyle, alcanediyle, CᵤH₂ᵤ et CᵥH₂ᵥ étant éventuellement substitués indépendamment les uns des autres et indépendamment des autres substituants par un ou plusieurs substituants fluor.

9. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 8, dans lequel
A représente O ;
X est choisi parmi alcanediyle en (C₁-C₆) et alcanediyloxy en (C₁-C₆) ;
Y représente un hétérocycle de 4 éléments à 6 éléments, saturé, monocyclique, qui ne contient pas d'hétéroatomes de cycle supplémentaires en plus de l'atome d'azote de cycle représenté sur la formule I, le cycle étant éventuellement substitué sur un ou plusieurs atomes de carbone de cycle par des substituants alkyle en (C₁-C₄) identiques ou différents ;
R¹ est choisi parmi hydrogène et alkyle en (C₁-C₄) ;
R² représente phénylène, qui est éventuellement substitué sur un ou plusieurs atomes de carbone de cycle par des substituants R²² identiques ou différents ;
R³ représente un radical d'un cycle saturé ou insaturé, de 3 éléments à 7 éléments, monocyclique, qui contient 0 ou 1 hétéroatome de cycle choisi parmi N, O et S, un atome d'azote de cycle pouvant porter un atome d'hydrogène ou un substituant alkyle en (C₁-C₄) et un atome de soufre de cycle pouvant porter un ou deux groupes oxo et le radical d'un cycle étant éventuellement substitué sur un ou plusieurs atomes de carbone de cycle par des substituants R³¹ identiques ou différents ;
R²² est choisi parmi halogène, hydroxy, alkyle en (C₁-C₄) et alkyloxy en (C₁-C₄)
R³¹ est choisi parmi halogène, alkyle en (C₁-C₄), cycloalkyle en (C₃-C₇), hydroxy et alkyloxy en (C₁-C₄) ; tous les groupes cycloalkyle étant éventuellement substitués indépendamment les uns des autres et indépendamment des autres substituants par un ou plusieurs substituants identiques ou différents choisis parmi fluor et alkyle en (C₁-C₄)
tous les groupes alkyle, alcanediyle, CᵤH₂ᵤ et CᵥH₂ᵥ étant éventuellement substitués indépendamment les uns des autres et indépendamment des autres substituants par un ou plusieurs substituants fluor.

10. Composé de formule I ou sel physiologiquement acceptable de celui-ci ou solvate physiologiquement acceptable d'un tel composé ou d'un tel sel selon une ou plusieurs des revendications 1 à 9, choisi parmi :
l'acide (S)-1-(2-{4-[5-(2-fluorophénoxy)oxazolo[5,4-d]pyrimidin-2-yl]-2,6-diméthylphénoxy}-acétyl)pyrrolidine-2-carboxylique,
l'acide (S)-1-(2-{4-[5-(2-fluorophénoxy)oxazolo[5,4-d]pyrimidin-2-yl]-2,6-diméthylphénoxy}-propionyl)pyrrolidine-2-carboxylique,
l'acide (S)-1-((E)-3-{4-[5-(2-fluorophénoxy)oxazolo[5,4-d]pyrimidin-2-yl]phényl}acryloyl)-pyrrolidine-2-carboxylique,
l'acide (S)-1-(3-{4-[5-(2-fluorophénoxy)oxazolo[5,4-d]pyrimidin-2-yl]phényl}propionyl)pyrrolidine-2-carboxylique,
l'acide (S)-1-(2-{4-[5-(2-fluorophénoxy)oxazolo[5,4-d]pyrimidin-2-yl]-2-méthylphénoxy}-acétyl)pyrrolidine-2-carboxylique,
l'acide (S)-1-(2-{4-[5-(2-fluorophénoxy)oxazolo[5,4-d]pyrimidin-2-yl]phénoxy}acétyl)pyrrolidine-2-carboxylique,
l'acide 1-(2-{4-[5-(2-fluorophénoxy)oxazolo[5,4-d]pyrimidin-2-yl]phénoxy}acétyl)pipéridine-4-carboxylique et
l'acide (S)-1-(2-{4-[5-(2-fluorophénoxy)oxazolo[5,4-d]pyrimidin-2-yl]phénoxy}propionyl)-pyrrolidine-2-carboxylique.

11. Procédé de fabrication d'un composé de formule I selon l'une quelconque des revendications 1 à 10, selon lequel un composé de formule II est mis en réaction avec un composé de formule III 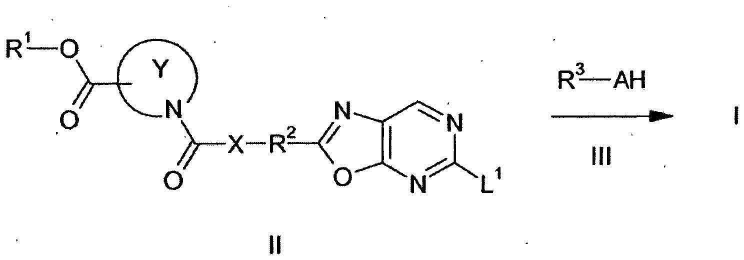 les groupes A, X, Y, R¹, R² et R³ dans les composés de formules II et III étant définis comme dans les composés de formule I et des groupes fonctionnels pouvant également être présents sous forme protégée ou sous la forme d'un groupe précurseur, et L¹ représentant un atome d'halogène ou un groupe de formule -S(O)-Alk ou -S(O)₂-Alk, Alk représentant alkyle en (C₁-C₄) .

12. Composition pharmaceutique contenant au moins un composé de formule I selon l'une quelconque des revendications 1 à 10 ou un sel physiologiquement acceptable de celui-ci ou un solvate physiologiquement acceptable d'un tel composé ou d'un tel sel et un véhicule pharmaceutiquement acceptable.

13. Composé de formule I selon l'une quelconque des revendications 1 à 10 ou sel physiologiquement acceptable de celui-ci ou solvate physiologiquement acceptable d'un tel composé ou d'un tel sel destiné à être utilisé en tant que médicament.

14. Composé de formule I selon l'une quelconque des revendications 1 à 10 ou sel physiologiquement acceptable de celui-ci ou solvate physiologiquement acceptable d'un tel composé ou d'un tel sel pour le traitement de troubles de la cicatrisation.

15. Composé de formule I selon l'une quelconque des revendications 1 à 10 ou sel physiologiquement acceptable de celui-ci ou solvate physiologiquement acceptable d'un tel composé ou d'un tel sel pour la cicatrisation.

16. Composé de formule I selon l'une quelconque des revendications 1 à 10 ou sel physiologiquement acceptable de celui-ci ou solvate physiologiquement acceptable d'un tel composé ou d'un tel sel pour la cicatrisation chez les diabétiques.

17. Composé de formule I selon l'une quelconque des revendications 1 à 10 ou sel physiologiquement acceptable de celui-ci ou solvate physiologiquement acceptable d'un tel composé ou d'un tel sel pour le traitement du syndrome du pied diabétique.
